# NEUE EUROPÄISCHE PATENTSCHRIFT

(11) **EP 2 619 284 B2**
(45) Veröffentlichungstag und Bekanntmachung des Hinweises auf die Entscheidung über den Einspruch: **28.12.2022**
(45) Hinweis auf die Patenterteilung: 12.04.2017
(21) Anmeldenummer: 11764088.8
(22) Anmeldetag: 29.08.2011
(51) Int. Cl.: C09K 19/54, C09K 19/42, C09K 19/56, C09K 19/04, G02F 1/1333, G02F 1/1337

(54) **FLÜSSIGKRISTALLANZEIGEN UND FLÜSSIGKRISTALLINE MEDIEN MIT HOMÖOTROPER AUSRICHTUNG**
LIQUID CRYSTAL DISPLAYS AND LIQUID CRYSTALLINE MEDIA WITH HOMEOTROPIC ALIGNMENT
ÉCRANS À BASE DE CRISTAUX LIQUIDES ET FLUIDES CRISTALLINS LIQUIDES À ORIENTATION HOMÉOTROPE

(30) Priorität: 25.09.2010 DE 102010046593
(43) Veröffentlichungstag der Anmeldung: 31.07.2013
(60) Teilanmeldung: 17165814.9
(73) Patentinhaber: Merck Patent GmbH, 64293 Darmstadt (DE)
(72) Erfinder: ARCHETTI, Graziano, 64289 Darmstadt (DE); TAUGERBECK, Andreas, 64285 Darmstadt (DE)
(74) Vertreter: Lederer & Keller Patentanwälte Partnerschaft mbB
(86) Internationale Anmeldenummer: PCT/EP2011/004340
(87) Internationale Veröffentlichungsnummer: WO 2012/038026

(56) Entgegenhaltungen:
- WO-A1-2007/100136
- WO-A1-2010/089092
- WO-A1-2010/089092
- DE-A1- 10 117 224
- DE-A1- 19 539 141
- DE-A1- 19 903 746
- FR-A1- 2 204 002
- JP-A- 5 841 827
- JP-A- S5 035 076
- JP-A- 57 140 737
- JP-A- H02 145 531
- JP-A- 2003 003 170
- JP-A- 2003 253 265
- JP-A- 2010 170 090
- US-A- 4 482 472
- US-A- 4 564 694
- US-A- 5 484 552
- US-A- 5 698 134
- US-A- 6 106 908
- US-A- 6 139 925
- US-A1- 2002 004 108
- US-A1- 2003 025 865
- US-A1- 2003 048 401
- US-A1- 2004 011 996
- US-A1- 2004 113 119
- US-A1- 2005 092 965
- US-A1- 2006 182 897
- US-A1- 2006 198 967
- US-A1- 2008 090 026
- US-A1- 2008 180 608
- US-A1- 2009 103 011
- US-A1- 2009 103 011
- US-A1- 2012 032 112
- KAYARAD et al.: Radiation Curable Products, pages 1-18,

## Beschreibung

Die vorliegende Erfindung betrifft flüssigkristalline Medien (FK-Medien) mit negativer dielektrischer Anisotropie enthaltend selbstausrichtende Mesogene (SAM, engl. 'self-aligning mesogenes'), die eine homöotrope (vertikale) Ausrichtung der FK-Medien an einer Oberfläche oder den Zellwänden einer Flüssigkristallanzeige (FK-Anzeige) bewirken. Die Erfindung umfasst daher auch FK-Anzeigen mit homöotroper Ausrichtung des flüssigkristallinen Mediums (FK-Medium) ohne konventionelle Imid-Orientierungsschichten. Die FK-Medien können durch eine polymerisierbare oder polymerisierte Komponente ergänzt sein, die zur Stabilisierung der Ausrichtung, zur Justierung des Tiltwinkels und/oder als Passivierungsschicht dient.

Das Prinzip der elektrisch kontrollierten Doppelbrechung, der ECB-Effekt (electrically controlled birefringence) oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten K₃/K₁, hohe Werte für die optische Anisotropie Δn und Werte für die dielektrische Anisotropie von Δε ≤ -0,5 aufweisen müssen, um für hochinformative Anzeigeelemente basierend auf dem ECB-Effekt eingesetzt werden zu können. Auf dem ECB-Effekt basierende elektrooptische Anzeigeelemente weisen eine homöotrope Randorientierung auf (VA-Technologie = Vertical Aligned).

Anzeigen, die den ECB-Effekt verwenden, haben sich als sogenannte VAN-(Vertically Aligned Nematic) Anzeigen beispielsweise in den Bauformen MVA- (Multi-Domain Vertical Alignment, z.B.: Yoshide, H. et al., Vortrag 3.1: "MVA LCD for Notebook or Mobile PCs ...", SID 2004 International Symposium, Digest of Technical Papers, XXXV, Buch I, S. 6 bis 9 und Liu, C.T. et al., Vortrag 15.1: "A 46-inch TFT-LCD HDTV Technnology ...", SID 2004 International Symposium, Digest of Technical Papers, XXXV, Buch II, S. 750 bis 753), PVA- (Patterned Vertical Alignment, z.B.: Kim, Sang Soo, Vortrag 15.4: "Super PVA Sets New State-of-the-Art for LCD-TV", SID 2004 International Symposium, Digest of Technical Papers, XXXV, Buch II, S. 760 bis 763), ASV- (Advanced Super View, z.B.: Shigeta, Mitzuhiro und Fukuoka, Hirofumi, Vortrag 15.2: "Development of High Quality LCDTV", SID 2004 International Symposium, Digest of Technical Papers, XXXV, Buch II, S. 754 bis 757) Anzeigen, neben IPS-(In Plane Switching) (z.B.: Yeo, S.D., Vortrag 15.3: "A LC Display for the TV Application", SID 2004 International Symposium, Digest of Technical Papers, XXXV, Buch II, S. 758 & 759) und den lange bekannten TN- (Twisted Nematic) Anzeigen, als eine der drei zur Zeit wichtigsten neueren Typen von Flüssigkristallanzeigen, insbesondere für Fernsehanwendungen, etabliert. In allgemeiner Form werden die Technologien z.B. in Souk, Jun, SIDSeminar 2004, Seminar M-6: "Recent Advances in LCD Technology", Seminar Lecture Notes, M-6/1 bis M-6/26 und Miller, lan, SIDSeminar 2004, Seminar M-7: "LCD-Television", Seminar Lecture Notes, M-7/1 bis M-7/32, verglichen. Obwohl die Schaltzeiten moderner ECB-Anzeigen durch Ansteuerungsmethoden mit Übersteuerung (overdrive) bereits deutlich verbessert wurden, z.B.: Kim, Hyeon Kyeong et al., Vortrag 9.1: "A 57-in. Wide UXGA TFT-LCD for HDTV Application", SID 2004 International Symposium, Digest of Technical Papers, XXXV, Buch I, S. 106 bis 109, ist die Erzielung von videotauglichen Schaltzeiten, insbesondere beim Schalten von Graustufen, immer noch ein noch nicht zufriedenstellend gelöstes Problem.

Mit der Erzeugung der VA-Displays mit zwei oder mehr Domänen unterschiedlicher Vorzugsrichtung ist ein beträchtlicher Aufwand verbunden. Ein Ziel dieser Erfindung ist es, die Herstellprozesse und die Anzeigevorrichtungen selbst zu vereinfachen, ohne die Vorteile der VA-Technik, wie relativ schnelle Schaltzeiten und gute Blickwinkelabhängigkeit, aufzugeben.

VA-Anzeigen die FK-Medien mit positiver dielektrischer Anisotropie enthalten, werden in S.H. Lee et al. Appl. Phys. Lett. (1997), 71, 2851-2853 beschrieben. Diese Anzeigen verwenden auf eine Substratoberfläche angeordnete Interdigitalelektroden (In-plane Ansteuerelektroden-Konfiguration kammförmiger Struktur), wie sie unter anderem bei den kommerziell erhältlichen IPS-(in-plane switching) Anzeigen zum Einsatz kommen (wie z.B. in DE 40 00 451 und EP 0 588 568 offenbart), und weisen eine homöotrope Anordnung des Flüssigkristallmediums auf, die zu einer planaren Anordnung beim Anlegen einer elektrischen Feldes wechselt.

Weiterentwicklungen der oben genannten Anzeige sind zum Beispiel in K.S. Hun et al. J. Appl. Phys. (2008), 104, 084515 (DSIPS: 'double-side in-plane switching' für Verbesserungen von Treiberspannung und Trasmission), M. Jiao et al. App. Phys. Lett (2008), 92, 111101 (DFFS: 'dual fringe field switching' für verbesserte Schaltzeiten) und Y.T. Kim et al. Jap. J. App. Phys. (2009), 48, 110205 (VAS: 'viewing angle switchable' LCD) zu finden. Darüber hinaus sind VA-IPS-Anzeigen auch unter dem Namen Positiv-VA und HT-VA bekannt.

Bei allen solche Anzeigen (hier nachfolgend allgemein als VA-IPS-Anzeigen bezeichnet) ist auf beiden Substratoberflächen eine Orientierungsschicht zur homöotropen Ausrichtung des FK-Mediums aufgebracht, deren Erzeugung bisher mit einem beträchtlichen Aufwand verbunden ist.

Ein Ziel dieser Erfindung ist es, die Herstellprozesse selbst zu vereinfachen, ohne die Vorteile der VA-IPS-Technik, wie relativ schnelle Schaltzeiten, gute Blickwinkelabhängigkeit und hohen Kontrast aufzugeben.

Für die technische Anwendung dieser Effekte in elektrooptischen Anzeigeelementen werden FK-Phasen benötigt, die einer Vielzahl von Anforderungen genügen müssen. Besonders wichtig sind hier die chemische Beständigkeit gegenüber Feuchtigkeit, Luft, den Materialien in den Substratoberflächen und physikalischen Einflüssen wie Wärme, Strahlung im infraroten, sichtbaren und ultravioletten Bereich und elektrische Gleich-und Wechselfelder.

Ferner wird von technisch verwendbaren FK-Phasen eine flüssigkristalline Mesophase in einem geeigneten Temperaturbereich und eine niedrige Viskosität gefordert.

VA- und VA-IPS-Anzeigen sollen im Allgemeinen einen sehr hohen spezifischen Widerstand bei gleichzeitig großem Arbeitstemperaturbereich, kurze Schaltzeiten und niedriger Schwellenspannung, mit deren Hilfe verschiedene Graustufen erzeugt werden können, besitzen.

In den herkömmlichen VA- und VA-IPS-Displays sorgt eine Polyimidschicht auf den Substratoberflächen für die homöotrope Orientierung des Flüssigkristalls. Die Herstellung einer geeigneten Orientierungsschicht im Display erfordert einen erheblichen Aufwand. Außerdem können Wechselwirkungen der Orientierungsschicht mit dem FK-Medium den elektrischen Widerstand der Anzeige verschlechtern. Wegen solcher möglichen Wechselwirkungen reduziert sich die Zahl der geeigneten Flüssigkristallkomponenten erheblich. Daher wäre es erstrebenswert die homöotrope Ausrichtung des FK-Mediums ohne Polyimid zu erreichen.

Der Nachteil der häufig verwendeten Aktivmatrix-TN-Anzeigen beruht in ihrem vergleichsweise niedrigen Kontrast, der relativ hohen Blickwinkelabhängigkeit und der Schwierigkeit in diesen Anzeigen Graustufen zu erzeugen.

Wesentlich bessere Blickwinkelabhängigkeiten weisen VA-Displays auf und werden daher hauptsächlich für Fernseher und Monitore verwendet.

Eine Weiterentwicklung stellen die sogenannten PS-bzw. PSA-Anzeigen ("Polymer Sustained" bzw. "Polymer Sustained Alignment") dar, für die auch gelegentlich der Begriff "Polymer Stabilized" verwendet wird. Ohne nennenswerte Einbußen sonstiger Parameter, wie insbesondere der günstigen Blickwinkelabhängigkeit des Kontrastes, zeichnen sich die PSA-Anzeigen durch die Verkürzung der Schaltzeiten aus.

In diesen Anzeigen wird dem FK-Medium eine geringe Menge (zum Beispiel 0,3 Gew.%, typischerweise <1 Gew.%) einer oder mehrerer polymerisierbarer Verbindung(en) zugesetzt, welche nach Einfüllen in die FK-Zelle mit oder ohne angelegte elektrische Spannung zwischen den Elektroden *in situ* polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als reaktive Mesogene oder "RM"s bezeichnet, zur FK-Mischung erwiesen. Die PSA-Technik wird bisher hauptsächlich für FK-Medien mit negativer dielektrischer Anisotropie eingesetzt.

Nachfolgend wir der Begriff "PSA", falls nicht anders angegeben, stellvertretend für PS-Anzeigen und PSA-Anzeigen verwendet.

Mittlerweile wird das PSA-Prinzip in diversen klassischen FK-Anzeigen angewendet. So sind beispielsweise PSA-VA-, PSA-OCB-, PSA-IPS-, PSA-FFS- und PSA-TN-Anzeigen bekannt. Die Polymerisation der polymerisierbaren Verbindung(en) erfolgt bei PSA-VA- und PSA-OCB-Anzeigen vorzugsweise bei angelegter elektrischer Spannung, bei PSA-IPS-Anzeigen mit oder ohne angelegte elektrische Spannung. Wie man in Testzellen nachweisen kann, führt das PS(A)-Verfahren zu einem 'pretilt' in der Zelle. Bei PSA-OCB-Anzeigen beispielsweise kann man erreichen, dass die Bend-Struktur stabilisiert wird, so dass man ohne Offset-Spannung auskommt oder diese reduzieren kann. Im. Falle von PSA-VA-Anzeigen wirkt sich der 'pretilt' positiv auf die Schaltzeiten aus. Für PSA-VA-Anzeigen kann ein Standard-MVA- bzw. -PVA Pixel- und Elektroden-Layout verwendet werden. Darüber hinaus kann man aber beispielsweise auch mit nur einer strukturierten Elektrodenseite und ohne 'Protrusions' auskommen, was die Herstellung wesentlich vereinfacht und gleichzeitig zu einem sehr guten Kontrast bei sehr guter Lichtdurchlässigkeit führt.

PSA-VA-Anzeigen sind beispielsweise in JP 10-036847 A, EP 1 170 626 A2, US 6,861,107, US 7,169,449, US 2004/0191428 A1, US 2006/0066793 A1 und US 2006/0103804 A1 beschrieben. PSA-OCB-Anzeigen sind beispielsweise in T.-J- Chen et al., Jpn. J. Appl. Phys. 45, 2006, 2702-2704 und S. H. Kim, L.-C- Chien, Jpn. J. Appl. Phys. 43, 2004, 7643-7647 beschrieben. PSA-IPS-Anzeigen sind zum Beispiel in US 6,177,972 und Appl. Phys. Lett. 1999, 75(21), 3264 beschrieben. PSA-TN-Anzeigen sind zum Beispiel in Optics Express 2004, 12(7), 1221 beschrieben. PSA-VA-IPS Anzeigen sind zum Beispiel in WO 2010/089092 A1 offenbart.

PSA-Anzeigen können ebenso wie die oben beschriebenen konventionellen FK-Anzeigen als Aktivmatrix- oder Passivmatrix-Anzeigen betrieben werden. Bei Aktivmatrix-Anzeigen erfolgt die Ansteuerung einzelner Bildpunkte üblicherweise durch integrierte, nicht-lineare aktive Elemente wie beispielsweise Transistoren (z.B. Dünnfilmtransistoren, engl. 'thin film transistor' bzw. "TFT"), bei Passivmatrix-Anzeigen üblicherweise nach dem Multiplex-Verfahren, wobei beide Verfahren aus dem Stand der Technik bekannt sind.

Insbesondere für Monitor- und vor allem TV-Anwendungen ist nach wie vor die Optimierung der Schaltzeiten, wie aber auch des Kontrastes und der Luminanz (also auch Transmission) der FK-Anzeige gefragt. Hier kann das PSA-Verfahren entscheidende Vorteile bringen. Insbesondere bei PSA-VA-Anzeigen kann man ohne nennenswerte Einbußen sonstiger Parameter eine Verkürzung der Schaltzeiten erreichen, die mit einem in Testzellen messbaren 'pretilt' korrelieren.

Im Stand der Technik werden für PSA-VA beispielsweise polymerisierbare Verbindungen der folgenden Formel verwendet 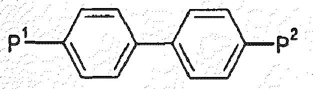 worin P eine polymerisierbare Gruppe, üblicherweise eine Acrylat- oder Methacrylatgruppe bedeutet, wie beispielsweise in US 7,169,449 beschrieben.

Der Aufwand für das Erzeugen einer Polyimidschicht, Behandlung der Schicht und Verbesserung mit Erhebungen oder Polymerschichten, ist relativ groß. Eine vereinfachende Technologie wäre daher wünschenswert, die einerseits die Produktionskosten verringert und andererseits die Bildqualität (Blickwinkelabhängigkeit, Kontrast, Schaltzeiten) zu optimieren hilft.

Über eine spontane horizontale bis vertikale Ausrichtung einer Flüssigkristallschicht mit Hilfe von Nanopartikeln basierend auf polyhedralen oligomeren Silsesquioxanen (nachfolgend einfach Silsesquioxane, PSS) berichtet die Druckschrift Shie-Chang Jeng et al. Optics Letters (2009), 34, 455-457. Ab einer Konzentration von ca. 1 Gew.-% wird eine nahezu homöotrope Ausrichtung beobachtet. Der 'pretilt' (engl.) ist nur durch die Konzentration beeinflussbar.

In der Druckschrift US 2008/0198301 A1 wird ebenfalls PSS als Orientierungsmaterial vorgeschlagen. Man erkennt, dass die Selbstorientierung auf ITO und auf planar orientierendem Polyimid funktioniert.

Auf das Problem der Temperaturabhängigkeit des Schaltvorgangs und der fehlenden Passivierungsschicht wird in beiden Druckschriften nicht hingewiesen. In der Tat hat es sich gezeigt, dass der Grad der von PSS induzierten homöotropen Orientierung mit steigender Temperatur rasch abnimmt. Eine Passivierungsschicht ist darüber hinaus besonders wichtig, da die Polyimidschicht nicht nur für die Orientierung des FK-Mediums, sondern auch für eine elektrische Isolierung sorgt. Ohne Passivierungsschicht können Probleme mit der Zuverlässigkeit der Anzeige ('Reliability') wie R-DC ('Residual-DC') erscheinen.

Auf einem Konferenzposter zur SID 2010 (H.Y. Gim et al., P-128) wird beschrieben, dass ein phenethylsubstituiertes polyhedrales oligomeres Silsesquioxan in einer Konzentration von 10 Gew.-% in einer Anzeige ohne konventionelle Orientierungschicht vom PSA-VA-Typ verwendet wird. Das FK-Medium mit negativer dielektrischer Anisotropie wird durch das PSS homöotrop orientiert. Die große Menge an Dotierstoff beeinflusst aber erheblich die Eigenschaften des FK-Mediums, daher ist die Anzahl an einsetzbaren Flüssigkristallkomponenten für eine derartige FK-Anzeige sehr begrenzt.

Die Druckschrift JP 2010170090 A offenbart ein Dendrimer als Zusatz zu Flüssigkristallmischungen, das eine vertikale Ausrichtung gegenüber Substraten bewirkt.

Flüssigkristalle mit Aminogruppen sind bisher selten beschrieben worden, weil sie kaum wünschenswerte Eigenschaften erzeugt haben. Über die homöotrope Ausrichtung einer flüssigkristallinen Phase aus N-alkyliertem p-(4-Alkylcyclohexyl)anilin berichtet J.H. MacMillan et al. Mol. Cryst. Liq. Cryst., 1979, 55, 61-70. Ebenso wird eine überwiegend homöotrope Ausrichtung der Flüssigkristallmatrix an einem Bis-p-aminobenzoesäureester des Hydrochinons beobachtet (D.C. Schroeder et al. J. Am. Chem. Soc., 1974, 96(13), 4347-4348). Flüssigkristalle mit Aminogruppen kommen bisher nicht in FK-Anzeigen zum Einsatz.

In den Druckschriften US 2006/182897A1, US 4,482,472,US 4,564,694 und FR 2204002A1 werden Verbindungen mit Alkoxygruppen offenbart, aber keine Verbindungen mit z.B. einer Hydroxygruppe als Teil einer Ankergruppe. In den Druckschriften US 2005/0092965A1 und US 2008/0180608A1 werden 4-N-Morpholinderivate als Photoinitiator für dielektrisch neutrale Flüssigkristallmischungen offenbart. In den Druckschriften US 2006/0198967A1, DE 19539141A1 und DE 10117224A1 werden 2,6-Di-tert-butyl-phenolderivate als Antioxidantien für eine Flüssigkristallmischung offenbart. In der oben genannten Druckschrift US 4,564,694 werden zusätzlich Verbindungen mit einer Endgruppe der Formel -CH₂OH als synthetische Zwischenstufen offenbart. In der Druckschrift US 2011/0113119A1 wird ein Anilinderivat als Zusatz für cholesterische Flüssigkristallmischungen mit positiver dielektrischer Anisotropie offenbart.

Die Druckschrift US 5,698,134 A offenbart Photoinitiatoren und ein Monomer, die eine OH Gruppe und gleichzeitig einen Ring aufweisen. Das FK-Medium hat eine positive dielektrische Anisotropie.

Die Druckschrift US 6,106,908 A offenbart Synthesen flüssigkristalliner Verbindungen, bei denen als Vorstufen Verbindungen mit einer OH-Gruppe auftreten.

Die Druckschrift US 2012/032112 A1 offenbart FK-Medien mit positiver dielektrischer Anisotropie und einen optionalen Stabilisator, der eine OH Gruppe und gleichzeitig einen Ring aufweist.

US 6,339,925 A offenbart mehrere FK-Medien mit positiver dielektrischer Anisotropie und weiterhin Additive zur Steuerung der elektrischen Leitfähigkeit in sehr geringen Konzentrationen, die eine OH Gruppe und gleichzeitig einen Ring aufweisen.

JP 58-41827 A offenbart als Intermediat der Synthese eine organische Verbindung mit einer Hydroxyverbindung und ein oder zwei Ringgruppen.

JP 57-1400737 A offenbart als Intermediat der Synthese organische Verbindungen mit einer Hydroxyverbindung. Da es sich um nicht um eine Endverbindung handelt, offenbart die Druckschrift auch kein FK-Medium mit solchen Verbindungen.

US 2008/0090026 A1 offenbart ein FK-Medium mit negativer dielektrischer Anisotropie, das eine polymerunterstützte Ausrichtung (PSA) aufweist.

US 2009/103011 A1 offenbart ein FK-Medium mit einer polymerisierbaren Komponente. Die polymerisierbare Komponente umfasst in der allgemeinen Form (Meth-)Acrylate und OH-Gruppen.

Die bestehenden Ansätze um zu Displayanwendungen ohne Polyimidschicht zu gelangen sind daher noch nicht vollständig zufrieden stellend.

Ein erster Gegenstand der vorliegenden Erfindung ist ein FK-Medium, wie in den Ansprüchen definiert, mit negativer dielektrischer Anisotropie enthaltend eine niedermolekulare flüssigkristalline Komponente und eine oder mehrere organische Verbindungen, die mindestens eine polare Ankergruppe und mindestens eine Ringgruppe aufweisen (Selbstorientierungsadditive), wobei die polare Ankergruppe mindestens eine OH-Struktur oder ein N-Atom in einer primären oder sekundären Amingruppe umfasst, und wobei die Ankergruppe nicht aus einer OH-Gruppe besteht, die wie die OH-Gruppe in 2,6-Di-tert-butylphenol abgeschirmt ist. Das erfindungsgemäße FK-Medium ist vorzugsweise nematisch.

Das FK-Medium enthält außerdem eine polymerisierte oder polymerisierbare Komponente, wobei die polymerisierte Komponente erhältlich ist durch Polymerisation einer polymerisierbaren Komponente. Mit dieser Komponente lässt sich das FK-Medium und insbesondere seine Orientierung stabilisieren und gegebenenfalls ein gewünschter'pre-tilt' einstellen.

Ein weiterer Gegenstand der vorliegenden Erfindung ist ein Verfahren zur Herstellung eines FK-Mediums wie vorangehend beschrieben und im Anspruch 1 umfasst, indem man eine niedermolekulare flüssigkristalline Komponente mit einer oder mehreren organischen Verbindungen, die mindestens eine polare Ankergruppe und mindestens eine Ringgruppe aufweisen, mischt und optional eine oder mehrere polymerisierbare Verbindungen und/oder Hilfsstoffe zugibt.

Ein weiterer Gegenstand der vorliegenden Erfindung ist eine Flüssigkristallanzeige (FK-Anzeige) enthaltend eine Flüssigkristallzelle (FK-Zelle) mit zwei Substraten und mindestens zwei Elektroden, wobei mindestens ein Substrat lichtdurchlässig ist und mindestens ein Substrat eine oder zwei Elektroden aufweist, sowie einer zwischen den Substraten befindlichen Schicht eines Flüssigkristallmediums (FK-Mediums) enthaltend eine niedermolekulare flüssigkristalline Komponente und eine oder mehrere organische Verbindungen, wobei die organische Verbindung dadurch gekennzeichnet ist, dass sie mindestens eine polare Ankergruppe aufweist und mindestens eine Ringgruppe aufweist, und die geeignet ist, eine homöotrope (vertikale) Ausrichtung des FK-Mediums gegenüber den Substratoberflächen herbeizuführen. Die Ankergruppe ist wie vor- und nachstehend näher gekennzeichnet.

Das FK-Medium der FK-Anzeige enthält außerdem eine polymerisierte oder polymerisierbare Komponente, wobei die polymerisierte Komponente erhältlich ist durch Polymerisation einer oder mehrerer polymerisierbarer Verbindungen zwischen den Substraten der FK-Zelle im FK-Medium, optional unter Anlegen einer elektrischen Spannung an die Elektroden der Zelle oder unter der Wirkung eines anderen elektrischen Feldes.

Ein weiterer Gegenstand der Erfindung ist ein Verfahren zu Herstellung einer FK-Anzeige, vorzugsweise des PSA-VA-Typs, enthaltend eine FK-Zelle mit zwei Substraten und mindestens zwei Elektroden, wobei mindestens ein Substrat lichtdurchlässig ist und mindestens ein Substrat ein oder zwei Elektroden aufweist, umfassend die Verfahrensschritte:
- Befüllen der Zelle mit einem FK-Medium wie vor und nachstehend oder in den Ansprüchen beschrieben, umfassend eine organische Verbindung die geeignet ist, eine homöotrope (vertikale) Ausrichtung des FK-Mediums gegenüber den Substratoberflächen herbeizuführen, und optional:
- Polymerisieren einer gegebenenfalls enthaltenen polymerisierbaren Komponente, optional unter Anlegen einer Spannung an die Elektroden der Zelle oder unter der Wirkung eines elektrischen Feldes.

Die organische Verbindung, die mindestens eine polare Ankergruppe und mindestens eine Ringgruppe aufweist (das Selbstorientierungsadditiv) wird im Flüssigkristall gelöst oder dispergiert. Sie bewirkt eine homöotrope Ausrichtung des Flüssigkristalls gegenüber den Substratoberflächen (wie z.B. eine Oberfläche aus Glas oder mit ITO oder mit Polyimid beschichtet). Es scheint in Anbetracht der Untersuchungen zu dieser Erfindung so, dass die polare Ankergruppe in Wechselwirkung mit der Substratoberfläche tritt. Dadurch richten sich die organischen Verbindungen auf der Substratoberfläche aus und induzieren eine homöotrope Orientierung des Flüssigkristalls. Die Ankergruppe sollte nach dieser Auffassung sterisch zugänglich sein, also beispielsweise nicht aus einer abgeschirmten OH-Gruppe wie in 2,6-Di-tert-butylphenol bestehen.

Das Selbstorientierungsadditiv wird vorzugsweise in einer Konzentration von weniger als 10 Gew.-%, besonders bevorzugt ≤ 8 Gew.-% und ganz besonders ≤ 5 Gew.-% eingesetzt. Es wird bevorzugt in einer Konzentration von mindestens 0,1 Gew.-% eingesetzt, bevorzugt mindestens 0,2 Gew.-%. Der Einsatz von 0,1 bis 2,5 Gew.-% des Selbstorientierungsadditivs führt in der Regel schon zu vollständig homöotroper Orientierung der FK-Schicht bei den üblichen Zelldicken (3 bis 4 µm).

Die polare Ankergruppe umfasst bevorzugt keine polymerisierbare Gruppe wie z.B. Acrylatgruppen.

Die polare Ankergruppe des Selbstorientierungsadditivs besteht bevorzugt aus einer Gruppe, die eine nicht-kovalente Wechselwirkung mit der Substratoberfläche aus Glas oder Metalloxiden eingeht. Geeignete Gruppen sind polare Gruppen umfassend polare Strukturelemente mit Atomen ausgewählt aus N, O, S, und P. Die Gruppen sollten gleichzeitig ausreichend stabil für den Einsatz als LC-Medium sein. Sie sollen außerdem die VHR-Werte ('voltage holding ratio') des FK-Mediums nur gering beeinflussen. Bevorzugt sind ein oder mehrere, bevorzugt zwei oder mehr, dieser Heteroatome in der Ankergruppe enthalten.

Die polare Ankergruppe umfasst mindestens eine OH-Struktur oder ein N-Atom in einer primären oder sekundären Amingruppe. Die polare Ankergruppe besteht bevorzugt aus ein bis zwei Strukturelementen enthaltend Heteroatome ausgewählt aus N und O und kovalenten, verknüpfenden Strukturen zwischen den Heteroatomen und zwischen einem oder mehreren der Heteroatome und dem Rest des Moleküls der Formel I (ohne die Ankergruppe).

Unter den Begriff Ringgruppe fallen alle herstellbaren Ringsysteme, d.h. auch alle aromatischen, heteroaromatischen, alicyclischen oder heterocyclischen Ringsysteme, auch polycyclische Ringsysteme, wobei bevorzugt wenigstens ein Teilring mindestens 4 Ringatome umfassen sollte. Das Ringsystem erzeugt einen sterischen Effekt, dient also als raumerfüllende Gruppe, und macht die Verbindung mehr oder weniger mesogen, wodurch sich die Löslichkeit und Kompatibilität im FK-Medium verbessert. Die Art der Ringgruppe kann daher stark variieren.

Das Selbstorientierungsadditiv ist bevorzugt eine organische Verbindung mit einer relativen Molmasse größer 100 g/mol, damit die Substanzen weniger flüchtig sind. Besonders bevorzugt weist es eine relative Molmasse größer 126 g/mol auf, um einen noch stabileren Effekt der Selbstausrichtung zu erzielen. Als Obergrenze hat es bevorzugt eine relativen Molmasse kleiner 700 g/mol.
Die Selbstorientierungsadditive haben eine Struktur der allgemeinen Formel I:

R¹-A¹-(Z²-A²)ₘ₁-R² (I)

In Formel I bedeutet der Rest R² eine polare Ankergruppe wie vor- und nachstehend definiert, der Rest "R¹-A¹-(Z²-A²)ₘ₁-" bedeutet eine Ausführungsform für eine mesogene Gruppe MES. Insbesondere bedeuten in Formel (I):
- A¹ und A²: jeweils unabhängig voneinander eine aromatische, heteroaromatische, alicyclische oder heterocyclische Gruppe, vorzugsweise mit 4 bis 25 C-Atomen, welche auch anellierte Ringe enthalten kann, und welche auch durch L ein- oder mehrfach substituiert sein kann, besonders bevorzugt jeweils unabhängig voneinander 1,4-Phenylen, Naphthalin-1,4-diyl oder Naphthalin-2,6-diyl, wobei in diesen Gruppen auch eine oder mehrere CH-Gruppen durch N ersetzt sein können, Cyclohexan-1,4-diyl, worin auch eine oder mehrere nicht-benachbarte CH₂-Gruppen durch O und/oder S ersetzt sein können, 3,3'-Bicyclobutyliden, 1,4-Cyclohexenylen, Bicyclo[1.1.1]pentan-1,3-diyl, Bicyclo[2.2.2]octan-1,4-diyl, Spiro[3.3]heptan-2,6-diyl, Piperidin-1,4-diyl, Decahydronaphthalin-2,6-diyl, 1,2,3,4-Tetrahydronaphthalin-2,6-diyl, Indan-2,5-diyl oder Octahydro-4,7-methano-indan-2,5-diyl, Perhydro-cyclopenta[a]phenanthren-3,17-diyl (insbesondere Gonan-3,17-diyl), wobei alle diese Gruppen unsubstituiert oder durch eine Gruppe L ein- oder mehrfach substituiert sein können,
- L: jeweils unabhängig voneinander OH, -(CH₂)ₙ₁-OH, F, Cl, Br, I, -CN, -NO₂, -NCO, -NCS, -OCN, -SCN, -C(=O)N(R⁰)₂, -C(=O)R⁰, -N(R⁰)₂, -(CH₂)ₙ₁-N(R⁰)₂, optional substituiertes Silyl, optional substituiertes Aryl oder Cycloalkyl mit 6 bis 20 C-Atomen, oder geradkettiges oder verzweigtes Alkyl, Alkoxy, Alkylcarbonyl, Alkoxycarbonyl, Alkylcarbonyloxy oder Alkoxycarbonyloxy mit 1 bis 25 C-Atomen, worin auch ein oder mehrere H-Atome durch F oder Cl ersetzt sein können,
- Z²: jeweils unabhängig voneinander eine Einfachbindung, -O-, -S-, -CO-, -CO-O-, -OCO-, -O-CO-O-, -OCH₂-, -CH₂O-, -SCH₂-, -CH₂S-, -CF₂O-, -OCF₂-, -CF₂S-, -SCF₂-, -(CH₂)ₙ₁-, -CF₂CH₂-, -CH₂CF₂-, -(CF₂)ₙ₁-, -CH=CH-, -CF=CF-, -C≡C-, -CH=CH-COO-, -OCO-CH=CH- oder CR⁰R⁰⁰, bevorzugt eine Einfachbindung,
- R⁰ und R⁰⁰: jeweils unabhängig voneinander H oder Alkyl mit 1 bis 12 C-Atomen,
- R¹: H, Halogen, geradkettiges, verzweigtes oder cyclisches Alkyl mit 1 bis 25 C-Atomen, worin auch eine oder mehrere nicht benachbarte CH₂-Gruppen durch -NR⁰-, -O-, -S-, -CO-, -CO-O-, -O-CO-, -O-CO-O- so ersetzt sein können, dass N-, O- und/oder S-Atome nicht direkt miteinander verknüpft sind, und worin auch ein oder mehrere tertiäre Kohlenstoffatome (CH-Gruppen) durch N ersetzt sein können, und worin auch ein oder mehrere H-Atome durch F oder Cl, ersetzt sein können, mit der Maßgabe, dass mindestens der Rest R² eine vorangehend beschriebene polare Ankergruppe umfasst, bevorzugt dass mindestens der Rest R² eine oder mehrere Gruppen NH, OH oder SH umfasst,
- m1: 0, 1, 2, 3, 4 oder 5, und
- n1: 1, 2, 3 oder 4.

Die Gruppe R² umfasst in den vorangehenden Formeln eine polare Ankergruppe, insbesondere eine Gruppe der Formel (A1)

-Sp-[X²-Z³-]ₖX¹ (A1)

worin
- Sp: eine Abstandsgruppe definiert wie Sp^{a} wie nachstehend für Formel M definiert, bevorzugt eine Abstandsgruppe Sp"-X" wie für Formel M unten definiert, wobei Sp" ein Alkylen mit 1 bis 12 C-Atomen bedeutet,
- X¹: eine Gruppe -NH₂, -NHR¹¹ -NR¹¹₂, -OR¹¹, -OH, -(CO)OH oder eine Gruppe der Formeln 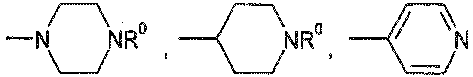 oder 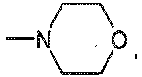
- R⁰: H oder Alkyl mit 1 bis 12 C-Atomen,
- X²: jeweils unabhängig -NH-, -NR¹¹-, -O- oder eine Einfachbindung, Z³ jeweils unabhängig eine Alkylengruppe mit 1-15 C-Atomen, carbocyclische Ringe mit 5 oder 6 C-Atomen (z.B. optional substituiertes Benzol, Cyclohexan), oder Kombinationen aus einem oder mehreren Ringen und Alkylengruppen, worin jeweils optional ein oder mehrere Wasserstoffatome durch -OH, OR¹¹, -(CO)OH, -NH₂, -NHR¹¹, -NR¹¹₂, oder Halogen (bevorzugt F, Cl) sind,
- R¹¹: jeweils unabhängig einen halogenierten oder unsubstituierten Alkylrest mit 1 bis 15 C-Atomen, wobei in diesem Rest auch eine oder mehrere CH₂-Gruppen jeweils unabhängig voneinander durch -C≡C-, -CH=CH-, -(CO)O-, -O(CO)-, -(CO)- oder -O- so ersetzt sein können, dass O-Atome nicht direkt miteinander verknüpft sind, und wobei zwei Reste R¹¹ optional miteinander zu einem Ring verknüpft sind, und
- k: 0 bis 3
bedeutet.

Besonders bevorzugt umfasst die Gruppe R² in den vorangehenden Formeln eine (N/O)-heteroatomhaltige Gruppe der Teilformel (A2) 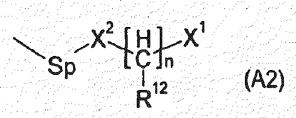 worin Sp, X¹ und X² wie oben für Formel A1 definiert sind, und
- R¹²: H, F, Cl, CN, -OH, -NH₂, oder einen halogenierten oder unsubstituierten Alkylrest mit 1 bis 15 C-Atomen, wobei in diesem Rest auch eine oder mehrere CH₂-Gruppen jeweils unabhängig voneinander durch -C≡C-, -CH=CH-, -(CO)O-, -O(CO)-, -(CO)-, -O-, -NH-, oder -NR¹- so ersetzt sein können, dass O- und N-Atome nicht direkt miteinander verknüpft sind, und
- N: 1, 2 oder 3,
bedeuten.

Es bedeutet die Gruppe R² genau eine Gruppe der Formeln (A1) bzw. (A2).

Besonders bevorzugte stickstoffhaltige Gruppen R² sind ausgewählt aus --(CH₂)ₙ-NH₂, -(CH₂)ₙ-NH-(CH₂)ₙ₃H, -NH-(CH₂)ₙ-NH₂, -NH-(CH₂)ₙ-NH-(CH₂)ₙ₃H, - (CH₂)ₙ₁-NH-(CH₂)ₙ₂-NH₂, -(CH₂)ₙ₁-NH-(CH₂)ₙ₂-NH-(CH₂)ₙ₃H, -O-(CH₂)ₙ-NH₂, -(CH₂)ₙ₁-O-(CH₂)ₙ-NH₂, -(CH₂)ₙ₁-NH-(CH₂)ₙ₂-OH, -O(CH₂)ₙ₁-NH-(CH₂)ₙ₂-NH₂, -O-(CH₂)ₙ₁-NH-(CH₂)ₙ₂-OH, -(CH₂)ₙ₁-NH-(CH₂)ₙ₂-NH-(CH₂)ₙ₃H, worin n, n1, n2 und n3 unabhängig 1, 2, 3, 4, 5, 6, 7, 8, 9, 10, 11 oder 12, insbesondere 1,2,3 oder 4 bedeuten.

Besonders bevorzugte stickstofffreie Gruppen R² sind ausgewählt aus -(CH₂)ₙ-OH, -O-(CH₂)ₙ-OH, -[O-(CH₂)ₙ₁-]ₙ₂-OH, -(CH₂)ₙ-(CO)OH, -O-(CH₂)ₙ-(CO)OH oder -[O-(CH₂)ₘ-]ₙ₂-(CO)OH,
worin n, n1 und n2 unabhängig 1, 2, 3, 4, 5, 6, 7, 8, 9, 10, 11 oder 12, insbesondere 1, 2, 3 oder 4 bedeuten.

Der Begriff "Aryl" bedeutet eine aromatische Kohlenstoffgruppe oder eine davon abgeleitete Gruppe. Der Begriff "Heteroaryl" bedeutet "Aryl" gemäß vorstehender Definition, enthaltend ein oder mehrere Heteroatome.

Aryl- und Heteroarylgruppen können einkernig oder mehrkernig sein, d.h. sie können einen Ring (wie z.B. Phenyl) oder zwei oder mehr Ringe aufweisen, welche auch anelliert (wie z.B. Naphthyl) oder kovalent verknüpft sein können (wie z.B. Biphenyl), oder eine Kombination von anellierten und verknüpften Ringen beinhalten. Heteroarylgruppen enthalten ein oder mehrere Heteroatome, vorzugsweise ausgewählt aus O, N, S und Se.

Besonders bevorzugt sind ein-, zwei- oder dreikernige Arylgruppen mit 6 bis 25 C-Atomen sowie ein-, zwei- oder dreikernige Heteroarylgruppen mit 2 bis 25 C-Atomen, welche optional anellierte Ringe enthalten und optional substituiert sind. Ferner bevorzugt sind 5-, 6- oder 7-gliedrige Aryl- und Heteroarylgruppen, worin auch eine oder mehrere CH-Gruppen durch N, S oder O so ersetzt sein können, dass O-Atome und/oder S-Atome nicht direkt miteinander verknüpft sind.

Bevorzugte Arylgruppen sind beispielsweise Phenyl, Biphenyl, Terphenyl, [1,1':3',1"]Terphenyl-2'-yl, Naphthyl, Anthracen, Binaphthyl, Phenanthren, Pyren, Dihydropyren, Chrysen, Perylen, Tetracen, Pentacen, Benzpyren, Fluoren, Inden, Indenofluoren, Spirobifluoren, etc.

Bevorzugte Heteroarylgruppen sind beispielsweise 5-gliedrige Ringe wie Pyrrol, Pyrazol, Imidazol, 1,2,3-Triazol, 1,2,4-Triazol, Tetrazol, Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 6-gliedrige Ringe wie Pyridin, Pyridazin, Pyrimidin, Pyrazin, 1,3,5-Triazin, 1,2,4-Triazin, 1,2,3-Triazin, 1,2,4,5-Tetrazin, 1,2,3,4-Tetrazin, 1,2,3,5-Tetrazin, oder kondensierte Gruppen wie Indol, Isoindol, Indolizin, Indazol, Benzimidazol, Benzotriazol, Purin, Naphthimidazol, Phenanthrimidazol, Pyridimidazol, Pyrazinimidazol, Chinoxalinimidazol, Benzoxazol, Naphthoxazol, Anthroxazol, Phenanthroxazol, Isoxazol, Benzothiazol, Benzofuran, Isobenzofuran, Dibenzofuran, Chinolin, Isochinolin, Pteridin, Benzo-5,6-chinolin, Benzo-6,7-chinolin, Benzo-7,8-chinolin, Benzoisochinolin, Acridin, Phenothiazin, Phenoxazin, Benzopyridazin, Benzopyrimidin, Chinoxalin, Phenazin, Naphthyridin, Azacarbazol, Benzocarbolin, Phenanthridin, Phenanthrolin, Thieno[2,3b]thiophen, Thieno[3,2b]thiophen, Dithienothiophen, Isobenzothiophen, Dibenzothiophen, Benzothiadiazothiophen, oder Kombinationen dieser Gruppen. Die Heteroarylgruppen können auch mit Alkyl, Alkoxy, Thioalkyl, Fluor, Fluoralkyl oder weiteren Aryl- oder Heteroarylgruppen substituiert sein.

Die (nicht-aromatischen) alicyclischen und heterocyclischen Gruppen umfassen sowohl gesättigte Ringe, d.h. solche die ausschließlich Einfachbindungen enthalten, als auch teilweise ungesättigte Ringe, d.h. solche die auch Mehrfachbindungen enthalten können. Heterocyclische Ringe enthalten ein oder mehrere Heteroatome, vorzugsweise ausgewählt aus Si, O, N, S und Se.

Die (nicht-aromatischen) alicyclischen und heterocyclischen Gruppen können einkernig sein, d.h. nur einen Ring enthalten (wie z.B. Cyclohexan), oder mehrkernig sein, d.h. mehrere Ringe enthalten (wie z.B. Decahydronaphthalin oder Bicyclooctan). Besonders bevorzugt sind gesättigte Gruppen. Ferner bevorzugt sind ein-, zwei- oder dreikernige Gruppen mit 3 bis 25 C-Atomen, welche optional anellierte Ringe enthalten und optional substituiert sind. Ferner bevorzugt sind 5-, 6-, 7- oder 8-gliedrige carbocyclische Gruppen worin auch ein oder mehrere C-Atome durch Si ersetzt sein können und/oder eine oder mehrere CH-Gruppen durch N ersetzt sein können und/oder eine oder mehrere nicht-benachbarte CH₂-Gruppen durch -O- und/oder -S- ersetzt sein können.

Bevorzugte alicyclische und heterocyclische Gruppen sind beispielsweise 5-gliedrige Gruppen wie Cyclopentan, Tetrahydrofuran, Tetrahydrothiofuran, Pyrrolidin, 6-gliedrige Gruppen wie Cyclohexan, Cyclohexen, Tetrahydropyran, Tetrahydrothiopyran, 1,3-Dioxan, 1,3-Dithian, Piperidin, 7- gliedrige Gruppen wie Cycloheptan, und anellierte Gruppen wie Tetrahydronaphthalin, Decahydronaphthalin, Indan, Bicyclo[1.1.1]pentan-1,3-diyl, Bicyclo[2.2.2]octan-1,4-diyl, Spiro[3.3]heptan-2,6-diyl, Octahydro-4,7-methano-indan-2,5-diyl.

Im Zusammenhang mit der vorliegenden Erfindung bedeutet der Ausdruck "Alkyl" einen geradkettigen oder verzweigten, gesättigten oder ungesättigten, bevorzugt gesättigten, aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen.

Der Begriff "cyclisches Alkyl" umfasst Alkylgruppen, die wenigstens einen carbocyclischen Teil aufweisen, also beispielsweise auch Cycloalkylalkyl, Alkylcycloalky und Alkylcycloalkylalkyl. Die carbocyclischen Gruppen umfassen z. B. Cyclopropyl, Cyclobutyl, Cyclopentyl, Cyclohexyl, Cycloheptyl, Cyclooctyl, usw.

"Halogen" steht im Zusammenhang der vorliegenden Erfindung für Fluor, Chlor, Brom beziehungsweise Iod, bevorzugt für Fluor oder Chlor.

Besonders bevorzugte Verbindungen der Formel I sind ausgewählt aus den folgenden beispielhaften Verbindungen, welche gleichzeitig besonders bevorzugte Gruppen R² der Selbstorientierungsadditive wiedergeben:

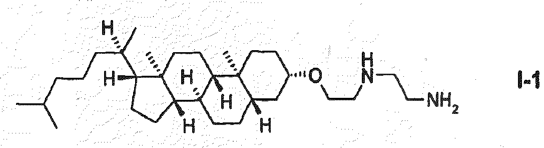

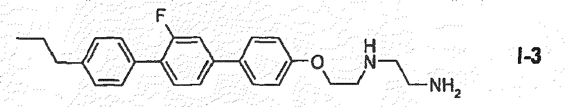

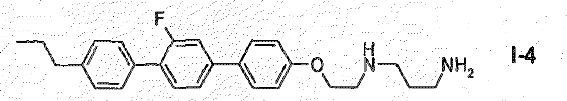

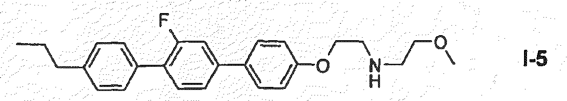

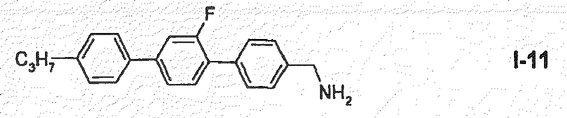

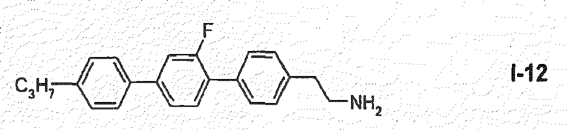

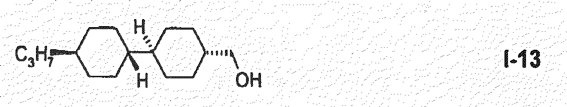

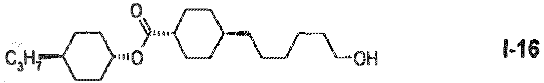

In einer weiteren bevorzugten Ausführungsform der Erfindung werden organische Verbindungen mit der polaren Ankergruppe bzw. Verbindungen der Formel I verwendet, die als weitere Funktionalisierung neben dem polaren Anker eine oder mehrere polymerisierbare Gruppen aufweisen (vergleiche Gruppe P^{a} oder P^{b} unten). Bevorzugte polymerisierbare Gruppen sind Gruppen wie Acrylat-, Methacrylat-, Fluoracrylat-, Oxetan-, Vinyloxy- oder Epoxygruppe, besonders bevorzugt Acrylat und Methacrylat. Durch den Einschluss der Verbindungen der Formel I in die Polymerisation werden die Verbindungen nachhaltig immobilisiert, wodurch sie ihre Funktion beibehalten.

Ein Vorteil der erfindungsgemäßen FK-Anzeigen ist, dass die Anzeige ohne die übliche Polyimid-Orientierungsschicht die gewünschte homöotrope Orientierung erreicht. Diese Orientierung bleibt in der Regel auch bei höherer Temperatur erhalten. Durch die Polymerstabilisierung wird die homöotrope Orientierung zusätzlich stabilisiert; dadurch wird eine verbesserte Temperaturstabilität des elektrooptischen Schaltens erreicht. Die erfindungsgemäßen polymerstabilisierten Anzeigen zeichnen sich durch verbesserte Schaltzeiten und besseres Kontrastverhältnis (Pretilt-Winkel und Temperaturabhängigkeit des Kontrasts) aus. Die optional enthaltene polymerisierte Komponente kann gleichzeitig als eine Passivierungsschicht dienen, die die Zuverlässigkeit (die sog. 'reliability') des Displays erhöht.

Die organischen Verbindungen mit der polaren Ankergruppe bzw. die Verbindungen der Formel I destabilisieren dank ihrer Struktur die nematische Phase des FK-Medium nicht, sondern tragen zu der Stabilität bei. Darüber hinaus beeinflusst die relativ kleine Menge an Verbindungen der Formel I die Eigenschaften der FK-Medien praktisch unerheblich. Daher kann eine breite Vielfalt an FlüssigkristallKomponenten in der FK-Anzeige verwendet werden.

Die erfindungsgemäßen FK-Anzeigen besitzen daher bevorzugt keine Orientierungsschicht ('alignment layer') für homöotrope Ausrichtung auf den Oberflächen der FK-Zelle, d.h. sie sind polyimidfrei. Für den Fall, dass die FK-Anzeigen dennoch ein- oder beidseitig Orientierungsschichten aufweisen, so bestehen diese vorzugsweise aus Polyimid. Die Orientierungsschichten sind vorzugsweise nicht gerieben. Damit entfällt das bisher notwendige Reiben der Orientierungsschicht, ein besonders aufwändiger Schritt in der Herstellung. Die ungeriebene Polyimidschicht kann als Passivierungsschicht dienen.

Die erfindungsgemäßen FK-Anzeigen verwenden ein FK-Medium mit negativer dielektrischer Anisotropie (Δε ≤ -1,5). In der Regel handelt es sich dabei um eine VA-Anzeige mit auf gegenüberliegenden Seiten der FK-Zelle angeordneten Elektroden, bevorzugt mit Elektroden, die so angeordnet sind, dass sie ein überwiegend senkrecht zur Substratoberfläche orientiertes elektrisches Feld erzeugen können. Typische verwendete Substrate sind die, die aus dem VAN-mode und PSA-VA verwendet werden (Strukturierung der Elektroden ist daher möglich).

Die FK-Anzeigen sind in der üblichen Art und Weise mit Polarisator(en) versehen, die den Schaltvorgang des FK-Mediums sichtbar machen.

Die polymerisierte Komponente der FK-Zelle (Polymer) ist erhältlich durch Polymerisieren einer polymerisierbaren Komponente (Monomere). In der Regel sind die Monomere zunächst in dem FK-Medium gelöst und werden in der FK-Zelle polymerisiert nachdem sich eine homöotrope Ausrichtung oder ein hoher Tiltwinkel des FK-Medium eingestellt hat. Zur Unterstützung der gewünschten Ausrichtung kann eine Spannung an die FK-Zelle angelegt werden. Im einfachsten Fall erübrigt sich eine solche Spannung und die gewünschte Ausrichtung stellt sich allein durch die Beschaffenheit des FK-Mediums und der Zellgeometrie ein.

Geeignete Monomere (polymerisierbare Komponente) für das FK-Medium sind solche aus dem Stand der Technik, die für PSA-VA- Anzeigen verwendet werden, insbesondere polymerisierbare Verbindungen der unten genannten Formel M und/oder der Formeln M1 bis M29. Die erfindungsgemäßen FK-Medien zur Verwendung in PSA-Anzeigen enthalten vorzugsweise < 5 Gew.-%, besonders bevorzugt < 1 Gew.-% und ganz besonders bevorzugt < 0.5 Gew.-% an polymerisierbaren Verbindungen, insbesondere polymerisierbare Verbindungen der unten genannten Formeln. Um einen ausreichenden Effekt zu erreichen werden bevorzugt 0,2 Gew.-% oder mehr eingesetzt. Die optimale Menge ist abhängig von der Schichtdicke.

Geeignete Monomere der polymerisierbaren Komponente des FK-Mediums werden durch die folgende Formel M beschrieben:

P^{a}-(Sp^{a})ₛ₁-A²-(Z¹-A¹)ₙ-(Sp^{b})ₛ₂-P^{b} M

worin die einzelnen Reste folgende Bedeutung besitzen:
- P^{a}, P^{b}: jeweils unabhängig voneinander eine polymerisierbare Gruppe,
- Sp^{a}, Sp^{b}: bei jedem Auftreten gleich oder verschieden eine Abstandsgruppe,
- s1, s2: jeweils unabhängig voneinander 0 oder 1,
- A¹, A²,: jeweils unabhängig voneinander einen Rest ausgewählt aus folgenden Gruppen
a) der Gruppe bestehend aus trans-1,4-Cyclohexylen, 1,4-Cyclohexenylen und 4,4'-Bicyclohexylen, worin auch eine oder mehrere nicht benachbarte CH₂-Gruppen durch -O-und/oder -S- ersetzt sein können und worin auch ein oder mehrere H-Atome durch F ersetzt sein können,
b) der Gruppe bestehend aus 1,4-Phenylen und 1,3-Phenylen, worin auch eine oder zwei CH-Gruppen durch N ersetzt sein können und worin auch ein oder mehrere H-Atome durch L ersetzt sein können,
c) der Gruppe bestehend aus Tetrahydropyran-2,5-diyl, 1,3-Dioxan-2,5-diyl, Tetrahydrofuran-2,5-diyl, Cyclobut-1,3-diyl, Piperidin-1,4-diyl, Thiophen-2,5-diyl und Selenophen-2,5-diyl, welche auch ein oder mehrfach durch L substituiert sein können,
d) der Gruppe bestehend aus gesättigten, teilweise ungesättigten oder vollständig ungesättigten, und optional substituierten, polycyclischen Resten mit 5 bis 20 cyclischen C-Atomen, von denen auch eines oder mehrere durch Heteroatome ersetzt sein können, vorzugsweise ausgewählt aus der Gruppe bestehend aus Bicyclo[1.1.1]pentan-1,3-diyl, Bicyclo[2.2.2]octan-1,4-diyl, Spiro[3.3]heptan-2,6-diyl, 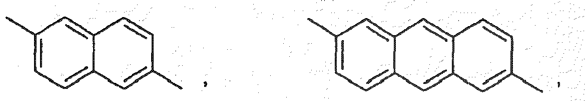 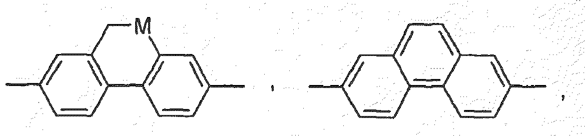 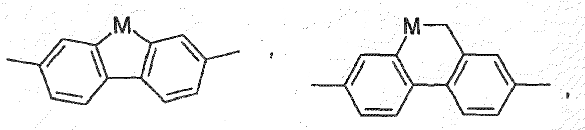 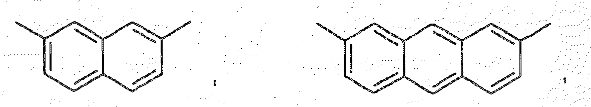 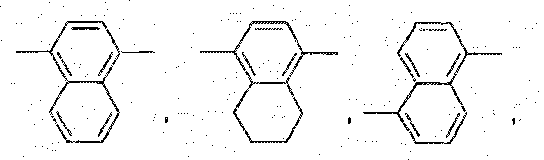 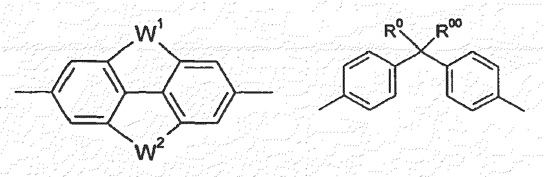 wobei in diesen Resten auch ein oder mehrere H- Atome durch L ersetzt sein können, und/oder eine oder mehrere Doppelbindungen durch Einfachbindungen ersetzt sein können, und/oder ein oder mehrere CH-Gruppen durch N ersetzt sein können,
- n: 0, 1, 2 oder 3
- Z¹: jeweils unabhängig voneinander -CO-O-, -O-CO-, -CH₂O-, -OCH₂-,-CF₂O-, -OCF₂-, oder -(CH₂)ₙ-, wobei n 2, 3 oder 4 ist, -O-, -CO-, -C(R^{c}R^{d})-, -CH₂CF₂-, -CF₂CF₂-, oder eine Einfachbindung,
- L: bei jedem Auftreten gleich oder verschieden F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
- R⁰, R⁰⁰: jeweils unabhängig voneinander H, F oder geradkettiges oder verzweigtes Alkyl mit 1 bis 12 C-Atomen, worin auch ein oder mehrere H-Atome durch F ersetzt sein können,
- M: -O-, -S-, -CH₂-, -CHY¹- oder -CY¹Y²-, und
- Y¹, und Y²: jeweils unabhängig voneinander eine der oben für R⁰ angegebenen Bedeutungen, Cl oder CN, und vorzugsweise H, F, Cl, CN, OCF₃ oder CF₃.
- W¹, W²: jeweils unabhängig voneinander -CH₂CH₂-, -CH=CH-, -CH₂-O-, -O-CH₂-, -C(R^{c}R^{d})- oder -O- bedeuten,
- R^{c} und R^{d}: jeweils unabhängig voneinander H oder Alkyl mit 1 bis 6 C-Atomen, vorzugsweise H, Methyl oder Ethyl, bedeuten,

Die polymerisierbare Gruppe P^{a,b} ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C=C-Doppelbindung oder -C=C-Dreifachbindung, sowie zur Polymerisation unter Ringöffnung geeignete Gruppen wie beispielsweise Oxetan- oder Epoxygruppen Bevorzugte Gruppen P^{a,b} sind ausgewählt aus der Gruppe bestehend aus CH₂=CW¹-CO-O-, CH₂=CW¹-CO-, 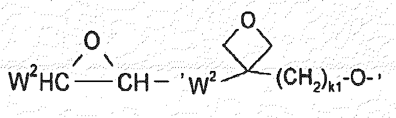 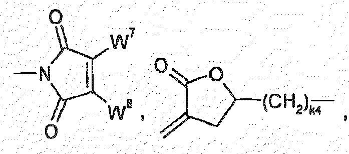 CH₂=CW²-(O)ₖ₃-, CW¹=CH-CO-(O)ₖ₃-,
CW¹=CH-CO-NH-, CH₂=CW¹-CO-NH-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit einem oder mehreren, von P-Sp- verschiedenen Resten L wie oben definiert substituiert ist, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Besonders bevorzugte Gruppen P^{a,b} sind ausgewählt aus der Gruppe bestehend aus CH₂=CW¹-CO-O-, CH₂=CW¹-CO-, 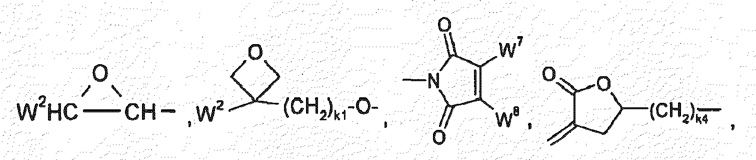 CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH- und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Ganz besonders bevorzugte Gruppen P^{a},^{b} sind sind ausgewählt aus der Gruppe bestehend aus CH₂=CW¹-CO-O-, insbesondere CH₂=CH-CO-O-, CH₂=C(CH₃)-CO-O- und CH₂=CF-CO-O-, ferner CH₂=CH-O-, 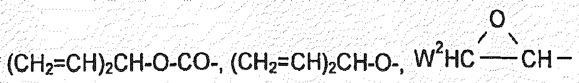 und 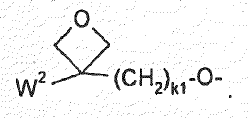 Ganz besonders bevorzugte Gruppen P^{a,b} sind daher ausgewählt aus der Gruppe bestehend aus Acrylat-, Methacrylat-, Fluoracrylat-, ferner Vinyloxy-, Chloracrylat-, Oxetan- und Epoxygruppen, und unter diesen bevorzugt eine Acrylat- oder Methacrylatgruppe.

Bevorzugte Abstandsgruppen Sp^{a,b} sind ausgewählt aus der Formel Sp"-X", so dass der Rest P^{a/b}-Sp^{a/b}- der Formel P^{a/b}-Sp"-X"- entspricht, wobei
- Sp": Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)- -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C=C- ersetzt sein können, dass O- und/oder S-Atome nicht direkt miteinander verknüpft sind,
- X": -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C=C-, -CH=CH-CO-O-, -O-CO-CH=CH- oder eine Einfachbindung bedeutet,
- R⁰⁰ und R⁰⁰⁰: jeweils unabhängig voneinander H oder Alkyl mit 1 bis 12 C-Atomen bedeuten, und
- Y² und Y³: jeweils unabhängig voneinander H, F, Cl oder CN bedeuten.
X" ist vorzugsweise -O-, -S -CO-, -COO-, -OCO-, -O-COO-, -CO-NR⁰-, -NR⁰-CO-, -NR⁰-CO-NR⁰- oder eine Einfachbindung.

Typische Abstandsgruppen Sp" sind beispielsweise -(CH₂)p₁-, -(CH₂CH₂O)_{q1}-CH₂CH₂-, -CH₂CH₂-S-CH₂CH₂-, -CH₂CH₂-NH-CH₂CH₂- oder -(SiR⁰⁰R⁰⁰⁰-O)ₚ₁-, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -Sp"-X"- sind -(CH₂)ₚ₁-, -(CH₂)ₚ₁-O-, -(CH₂)ₚ₁-O-CO-, -(CH₂)ₚ₁-O-CO-O-, worin p1 und q1 die oben angegebene Bedeutung haben.

Besonders bevorzugte Gruppen Sp" sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

Besonders bevorzugte Monomere sind die folgenden: 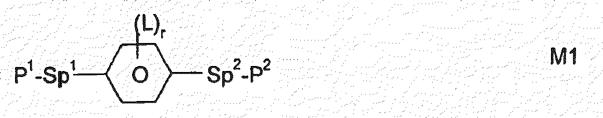 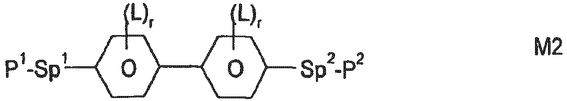 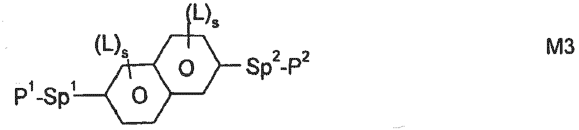 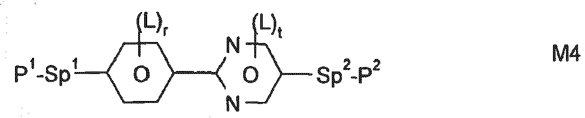 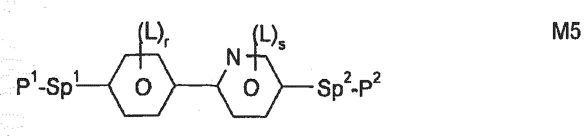 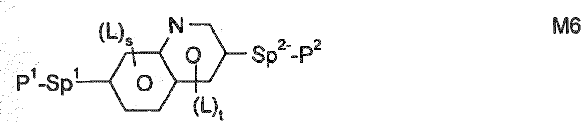 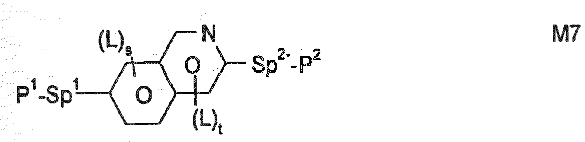 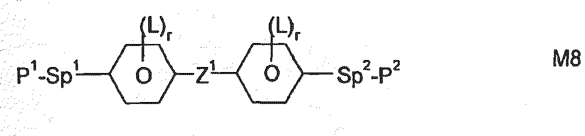 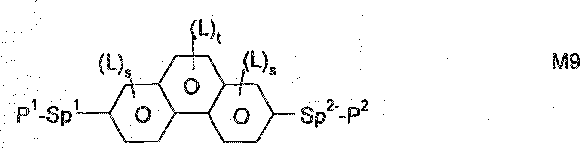 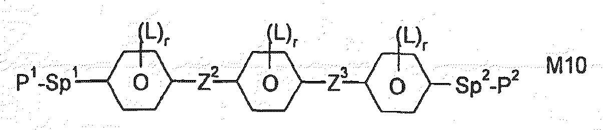 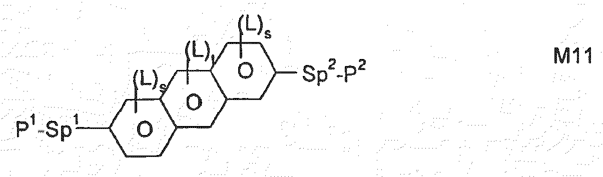 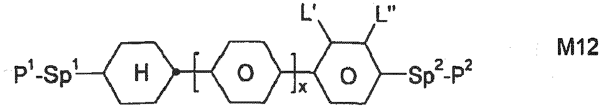 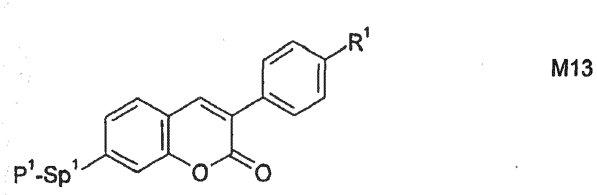 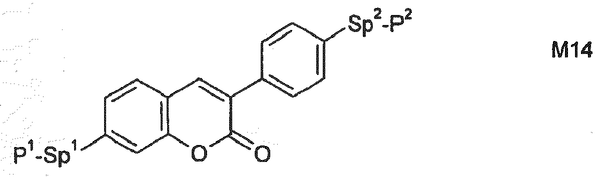 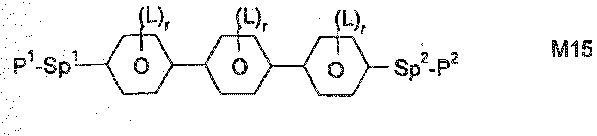 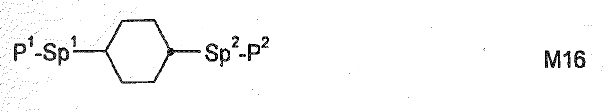 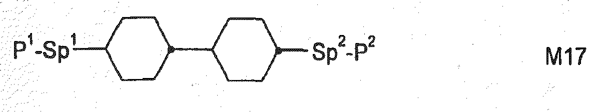 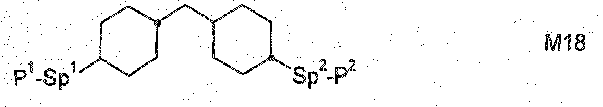 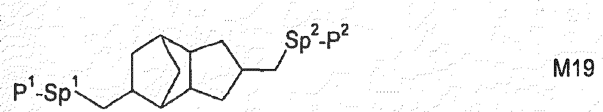 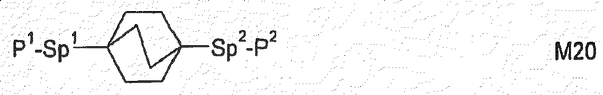 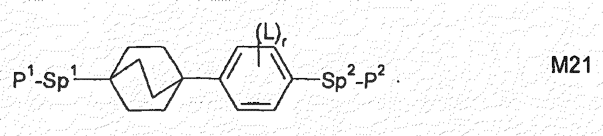 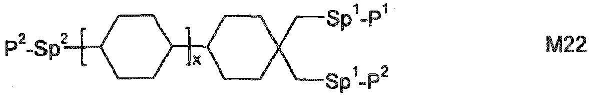 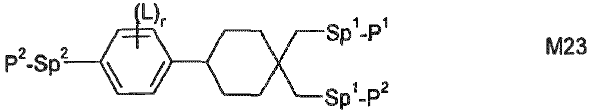 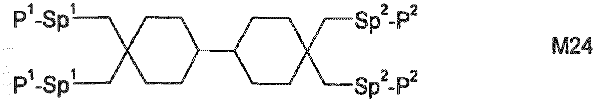 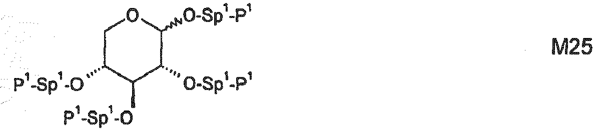 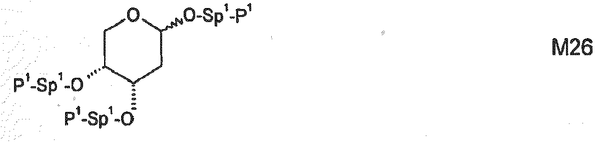 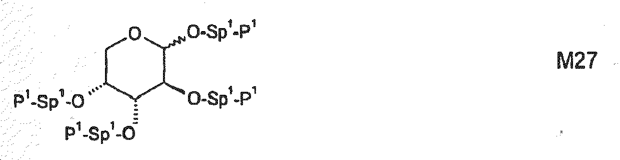 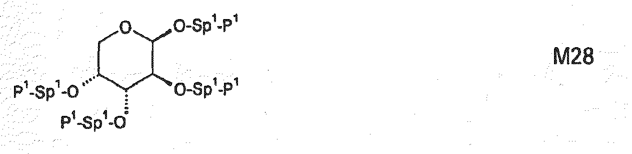 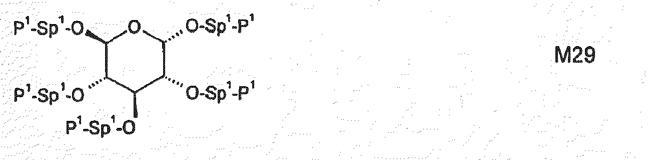 worin die einzelnen Reste folgende Bedeutung besitzen:
- P¹ und P²: jeweils unabhängig voneinander eine polymerisierbare Gruppe wie für Formel I definiert, bevorzugt eine Acrylat-, Methacrylat-, Fluoracrylat-, Oxetan-, Vinyloxy- oder Epoxygruppe,
- Sp¹ und Sp²: jeweils unabhängig voneinander eine Einfachbindung oder eine Abstandsgruppe, vorzugsweise mit einer der vor- und nachstehend für Sp^{a} angegebenen Bedeutungen, und besonders bevorzugt -(CH₂)ₚ₁-, -(CH₂)ₚ₁-O-, -(CH₂)ₚ₁-CO-O- oder -(CH₂)ₚ₁-O-CO-O-, worin p1 eine ganze Zahl von 1 bis 12 ist, und wobei in den letztgenannten Gruppen die Verknüpfung zur benachbarten Ring über das O-Atom erfolgt, wobei auch einer oder mehrere der Reste P¹-Sp¹- und P²-Sp²-einen Rest R^{aa} bedeuten können, mit der Maßgabe dass mindestens einer der vorhandenen Reste P¹-Sp¹- und P²-Sp²-nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹-ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander H oder Alkyl mit 1 bis 12 C-Atomen,
- R^{y} und R^{z}: jeweils unabhängig voneinander H, F, CH₃ oder CF₃,
- Z¹: -O-, -CO-, -C(R^{y}R^{z})-, oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, SCN, SF₅ oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen, vorzugsweise F,
- L' und L": jeweils unabhängig voneinander H, F oder Cl,
- R: 0, 1, 2, 3 oder 4,
- s: 0, 1, 2 oder 3,
- t: 0, 1 oder 2, und
- x: 0 oder 1.

Vorzugsweise umfasst das FK-Medium oder die polymerisierbare Komponente eine oder mehrere Verbindungen ausgewählt aus der Gruppe der Formeln M1-M21, besonders bevorzugt der Formeln M2-M15, ganz besonders bevorzugt der Formeln M2, M3, M9, M14 und M15.

Vorzugsweise umfasst das FK-Medium oder die polymerisierbare Komponente keine Verbindungen der Formel M10, worin Z² und Z³ -(CO)O-oder -O(CO)- bedeuten.

Zur Herstellung von PSA-Anzeigen werden die polymerisierbaren Verbindungen im FK-Medium zwischen den Substraten der FK-Anzeige, optional unter Anlegen einer Spannung, durch in-situ-Polymerisation polymerisiert oder vernetzt (falls eine polymeriserbare Verbindung zwei oder mehr polymerisierbare Gruppen enthält). Die Polymerisation kann in einem Schritt durchgeführt werden. Es ist auch möglich, zunächst in einem ersten Schritt die Polymerisation unter Anlegen einer Spannung durchzuführen, um einen pretilt-Winkel zu erzeugen, und anschließend in einem zweiten Polymerisationsschritt ohne anliegende Spannung die im ersten Schritt nicht abreagierten Verbindungen zu polymerisieren bzw. zu vernetzen ("end curing").

Geeignete und bevorzugte Polymerisationsmethoden sind beispielsweise die thermische oder Photopolymerisation, vorzugsweise Photopolymerisation, insbesondere UV-Photopolymerisation. Dabei können gegebenenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren Irgacure651^{®}, Irgacure184^{®}, Irgacure907^{®}, Irgacure369^{®}, oder Darocure1173^{®} (Ciba AG). Falls ein Initiator eingesetzt wird, beträgt dessen Anteil vorzugsweise 0,001 bis 5 Gew.-%, besonders bevorzugt 0,001 bis 1 Gew.-%.

Die erfindungsgemäßen polymerisierbaren Verbindungen eignen sich auch für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte. Die Polymerisation kann somit auch ohne Zusatz eines Initiators erfolgen. Somit enthält das FK-Medium in einer bevorzugten Ausführungsform keinen Polymerisationsinitiator.

Die polymerisierbare Komponente oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox^{®} (Ciba AG), wie beispielsweise Irganox^{®} 1076. Falls Stabilisatoren eingesetzt werden, beträgt deren Anteil, bezogen auf die Gesamtmenge der RMs beziehungsweise der polymerisierbaren Komponente, vorzugsweise 10 - 10000 ppm, besonders bevorzugt 50 - 500 ppm.

Die FK-Medien zur Verwendung in den erfindungsgemäßen FK-Anzeigen enthalten, neben den oben beschriebenen SAM und den optionalen polymerisierbaren Verbindungen (M) eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA- und VA-IPS-Anzeigen geeignete dielektrisch negative oder positive FK-Mischung.

Geeignete FK-Mischungen sind dem Fachmann bekannt und in der Literatur beschrieben. FK-Medien für VA-Anzeigen mit negativer dielektrischer Anisotropie sind in EP 1 378 557 A1 beschrieben.

Im Folgenden werden bevorzugte Ausführungsformen für das erfindungsgemäße flüssigkristalline Medium mit negativer dielektrischer Anisotropie angeführt:
a) FK-Medium, welches zusätzlich eine oder mehrere Verbindungen ausgewählt aus der Gruppe der Verbindungen der Formeln A, B und C enthält, 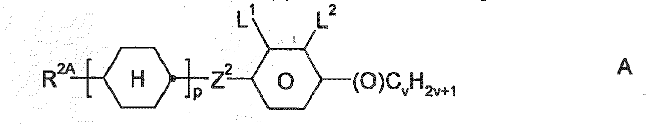 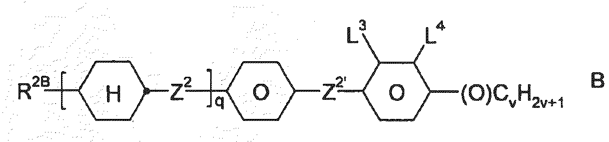 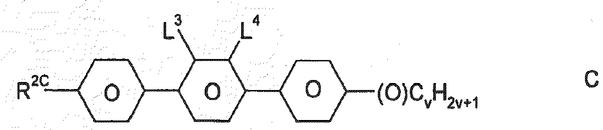 worin
   - R^{2A}, R^{2B} und R^{2C}: jeweils unabhängig voneinander H, einen unsubstituierten, einen einfach durch CN oder CF₃ oder mindestens einfach durch Halogen substituierten Alkylrest mit bis zu 15 C-Atomen, wobei in diesen Resten auch eine oder mehrere CH₂-Gruppen durch -O-, -S-, 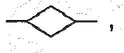 -C≡C-, CH₂-Gruppen durch -O-, -S-, 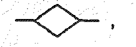 -C≡C-, -CF₂O-, -OCF₂-, -OC-O- oder -O-CO- so ersetzt sein können, dass O-Atome nicht direkt miteinander verknüpft sind,
   - L¹⁻⁴: jeweils unabhängig voneinander F, Cl, CF₃ oder CHF₂,
   - Z² und Z^{2'}: jeweils unabhängig voneinander Einfachbindung, -CH₂CH₂-, -CH=CH-, -CF₂O-, -OCF₂-, -CH₂O-, -OCH₂-, -COO-, -OCO-, -C₂F4-, -CF=CF-, -CH=CHCH₂O-,
   - p: 1 oder 2,
   - q: 0 oder 1, und
   - v: 1 bis 6
   bedeuten.
   In den Verbindungen der Formeln A und B können Z² gleiche oder unterschiedliche Bedeutungen haben. In den Verbindungen der Formel B können Z² und Z^{2'} gleiche oder verschiedene Bedeutungen aufweisen.
   In den Verbindungen der Formeln A, B und C bedeuten R^{2A}, R^{2B} und R^{2C} jeweils vorzugsweise Alkyl mit 1-6 C-Atomen, insbesondere CH₃, C₂H₅, n-C₃H₇, n-C₄H₉, n-C₅H₁₁.
   In den Verbindungen der Formeln A und B bedeuten L¹, L², L³ und L⁴ vorzugsweise L¹ = L² = F und L³ = L⁴ = F, ferner L¹ = F und L² = Cl, L¹ = Cl und L² = F, L³ = F und L⁴ = Cl, L³ = Cl und L⁴ = F. Z² und Z^{2'} bedeuten in den Formeln A und B vorzugsweise jeweils unabhängig voneinander eine Einfachbindung, ferner eine -C₂H₄-Brücke.
   Sofern in der Formel B Z² = -C₂H₄- ist, ist Z^{2'} vorzugsweise eine Einfachbindung bzw. falls Z^{2'} = -C₂H₄- bedeutet, ist Z² vorzugsweise eine Einfachbindung. In den Verbindungen der Formeln A und B bedeutet (O)CᵥH₂ᵥ₊₁ vorzugsweise OCᵥH₂ᵥ₊₁, ferner CᵥH₂ᵥ₊₁. In den Verbindungen der Formel C bedeutet (O)CᵥH₂ᵥ₊₁ vorzugsweise CᵥH₂ᵥ₊₁. In den Verbindungen der Formel C bedeuten L³ und L⁴ vorzugsweise jeweils F.
   Bevorzugte Verbindungen der Formeln A, B und C werden nachfolgend genannt: 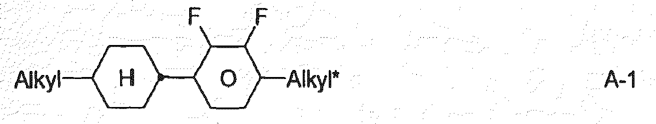 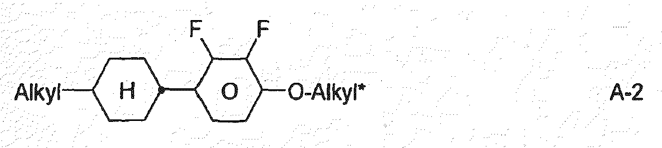 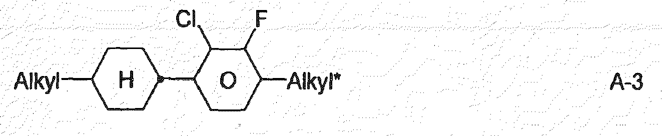 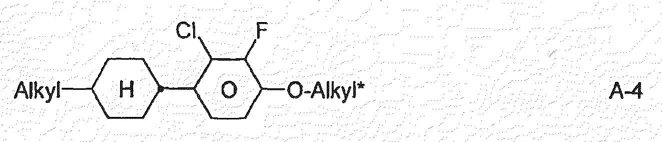 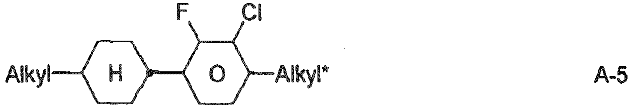 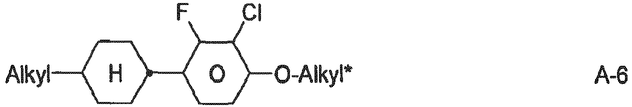 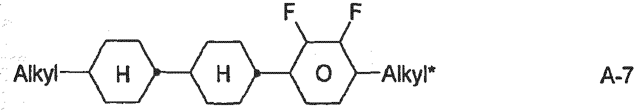 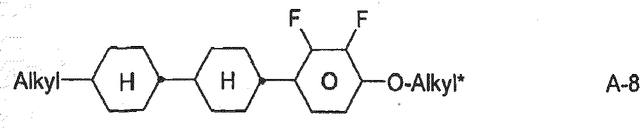 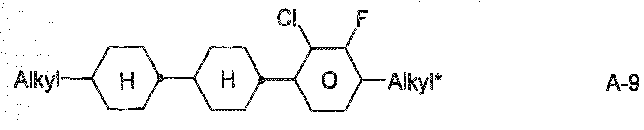 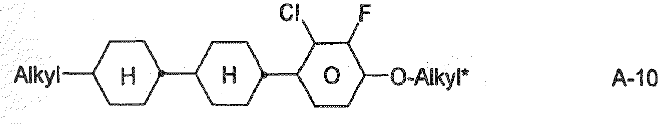 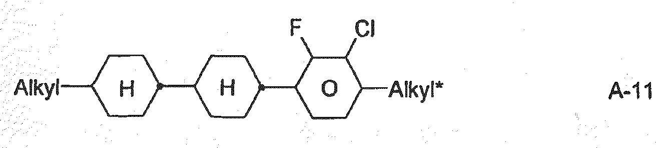 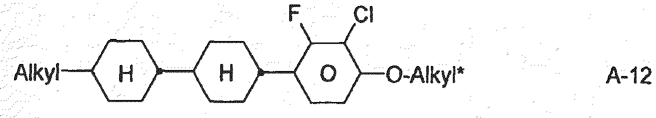 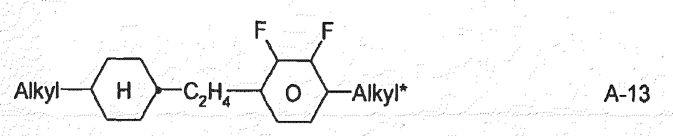 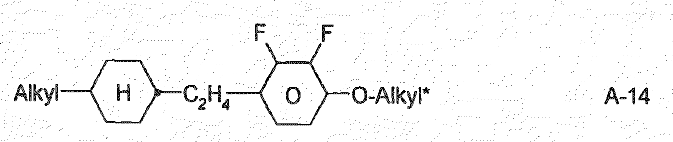 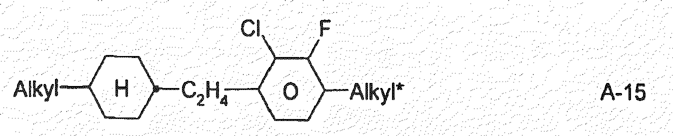 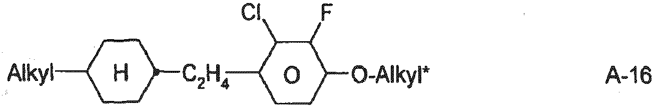 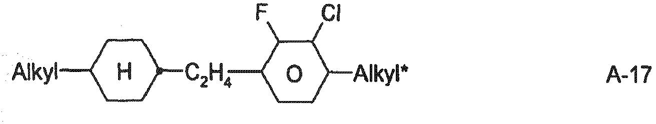 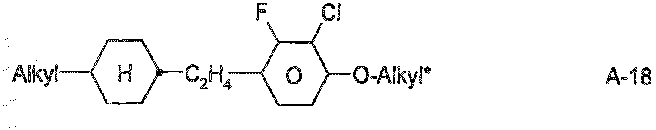 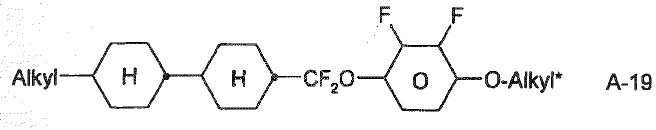 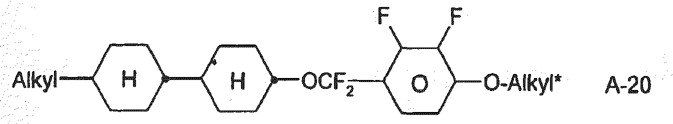 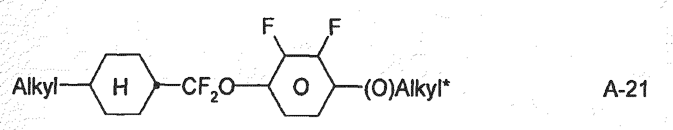 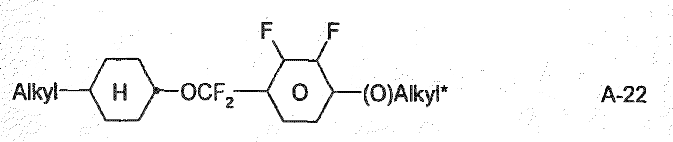 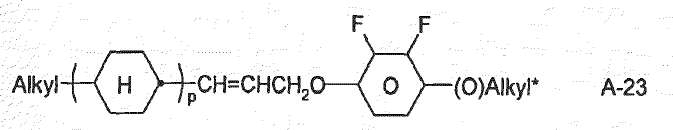 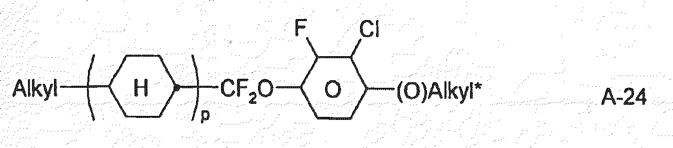 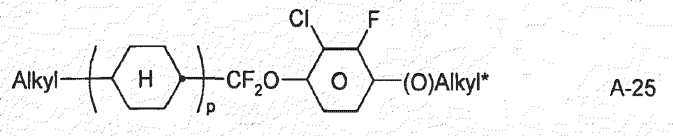 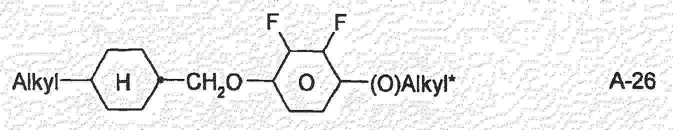 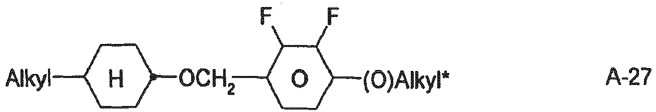 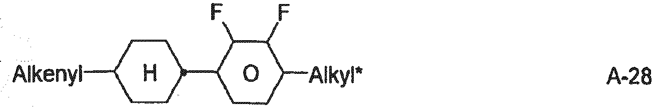 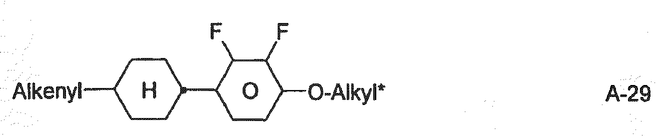 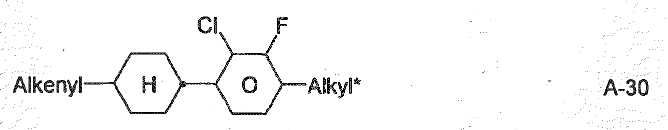 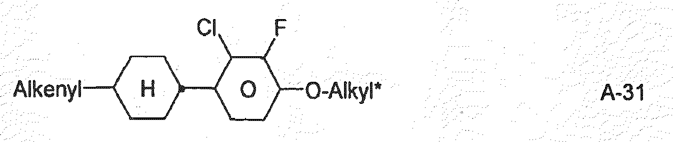 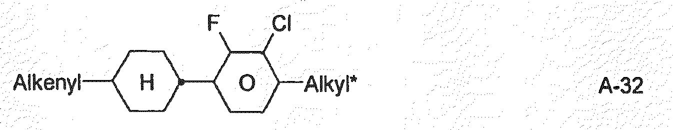 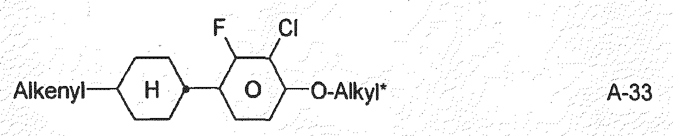 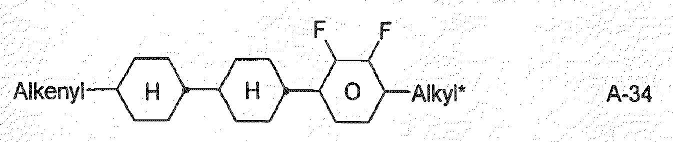 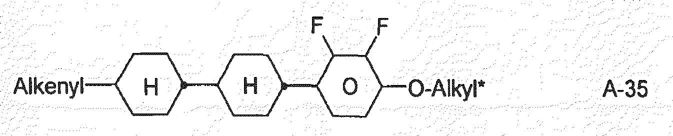 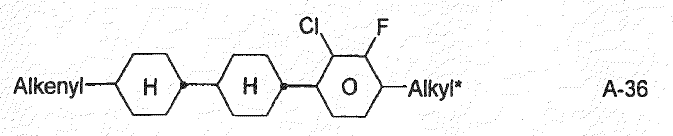 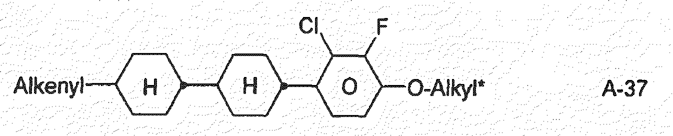 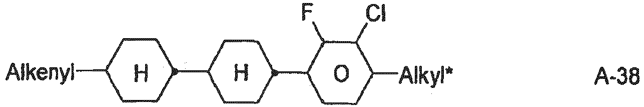 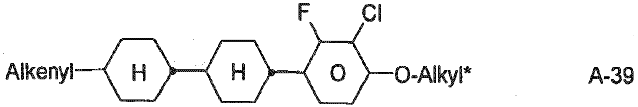 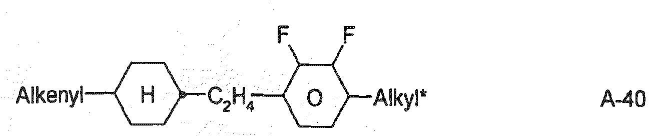 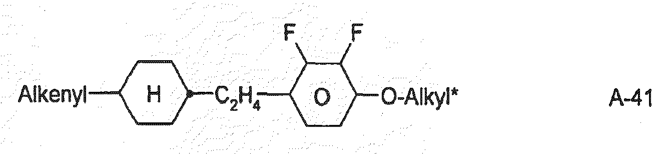 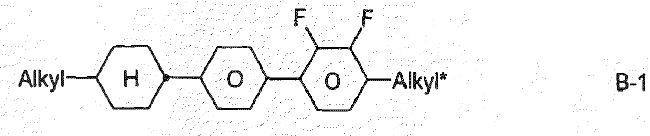 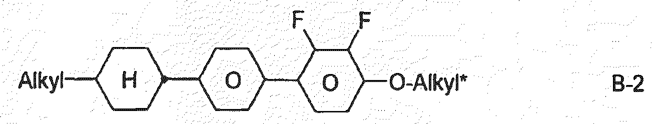 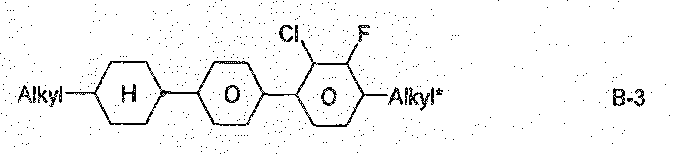 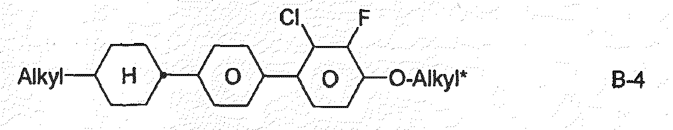 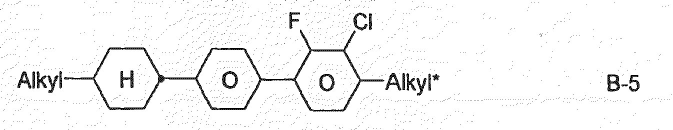 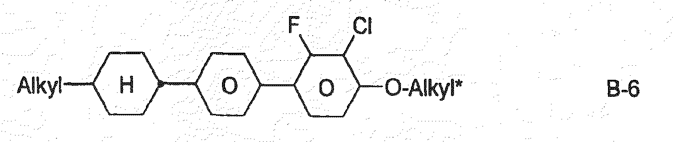 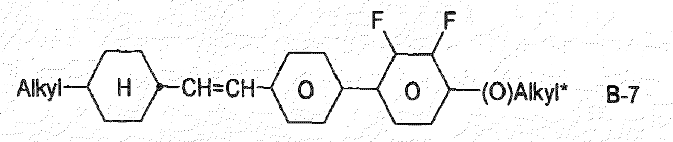 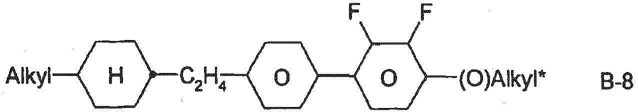 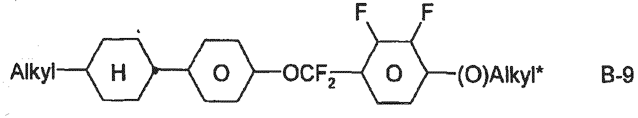 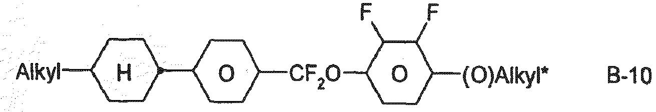 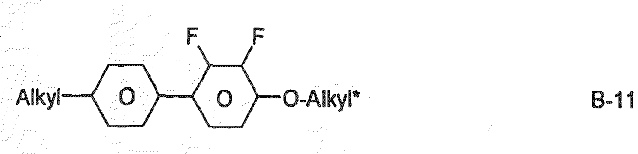 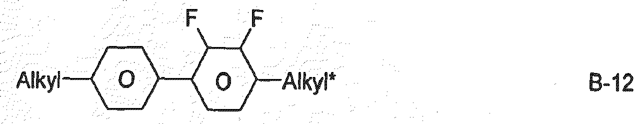 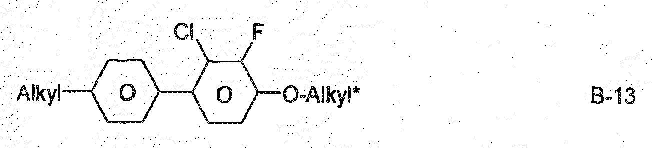 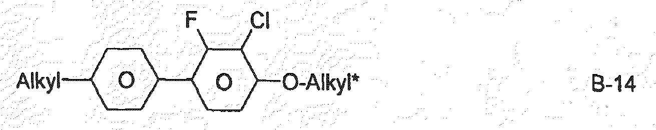 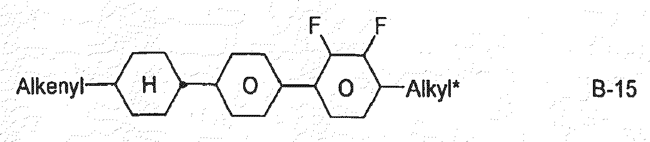 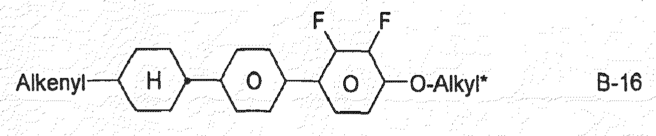 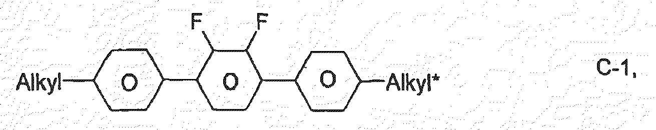 worin Alkyl und Alkyl* jeweils unabhängig voneinander einen geradkettigen Alkylrest mit 1-6 C-Atomen bedeuten.
   Besonders bevorzugte erfindungsgemäße Mischungen enthalten eine oder mehrere Verbindungen der Formeln A-2, A-8, A-14, A-29, A-35, B-2, B-11, B-16 und C-1. Vorzugsweise beträgt der Anteil an Verbindungen der Formeln A und/oder B im Gesamtgemisch mindestens 20 Gew.%. Besonders bevorzugte erfindungsgemäße Medien enthalten mindestens eine Verbindung der Formel C-1, 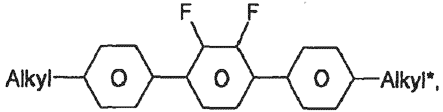 C-1 worin Alkyl und Alkyl*' die oben angegebenen Bedeutungen haben, vorzugsweise in Mengen von > 3 Gew.%, insbesondere > 5 Gew.% und besonders bevorzugt von 5-25 Gew.%.
b) FK-Medium, welches zusätzlich eine oder mehrere Verbindungen der folgenden Formel enthält: 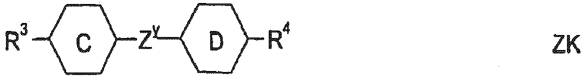 worin die einzelnen Reste folgende Bedeutung besitzen 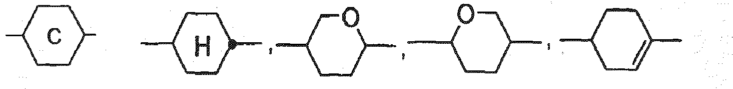 oder 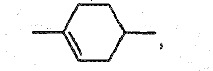 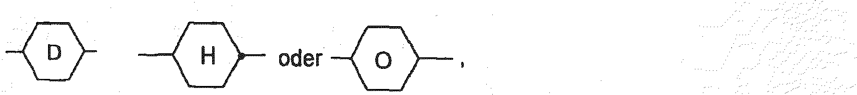
   - R³ und R⁴: jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
   - Z^{y}: -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung.

   Die Verbindungen der Formel ZK sind vorzugsweise ausgewählt aus der Gruppe bestehend aus folgenden Unterformeln: 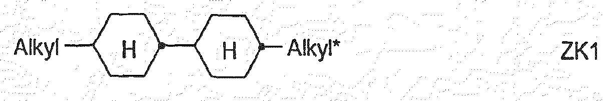 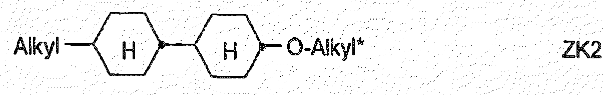 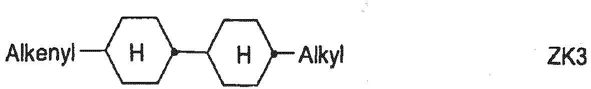 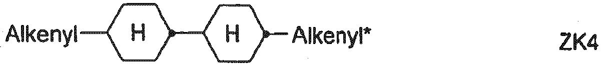 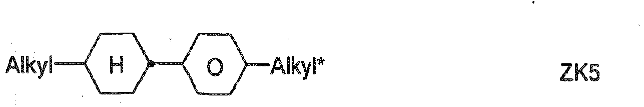 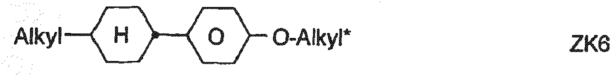 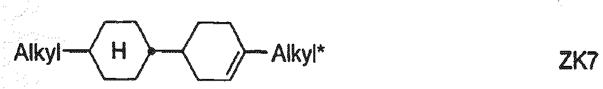 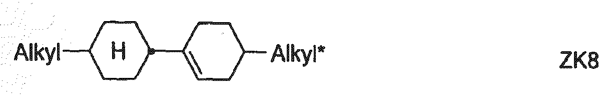 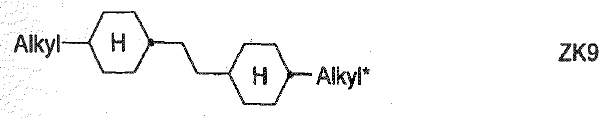 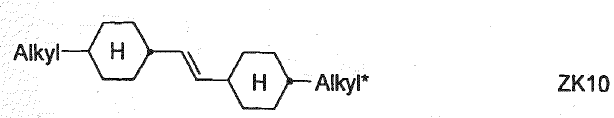 worin Alkyl und Alkyl* jeweils unabhängig voneinander einen geradkettigen Alkylrest mit 1-6 C-Atomen bedeuten, und Alkenyl und Alkenyl* einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
c) FK-Medium, welches zusätzlich eine oder mehrere Verbindungen der folgenden Formel enthält: 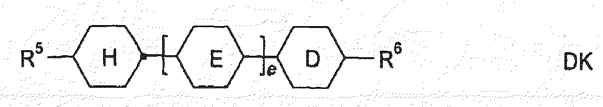 worin die einzelnen Reste bei jedem Auftreten gleich oder verschieden folgende Bedeutung haben:
   - R⁵ und R⁶: jeweils unabhängig voneinander eine der oben für R^{3/4} angegebenen Bedeutungen, 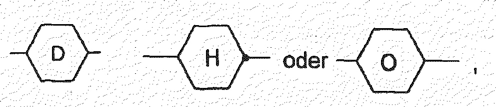 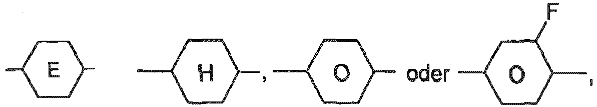 und
   e 1 oder 2.

   Die Verbindungen der Formel DK sind vorzugsweise ausgewählt aus der Gruppe bestehend aus folgenden Unterformeln: 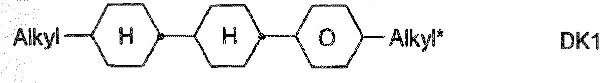 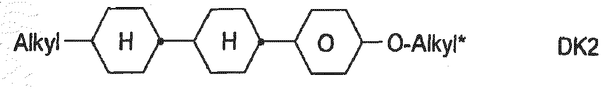 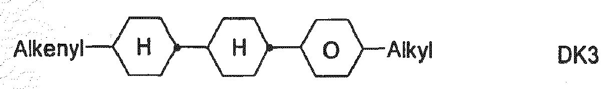 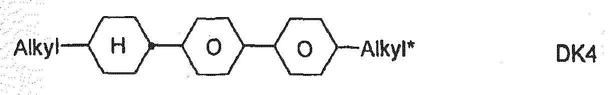 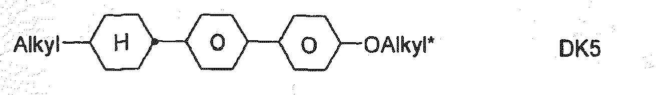 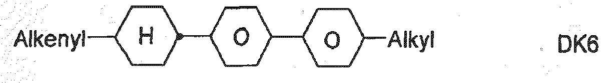 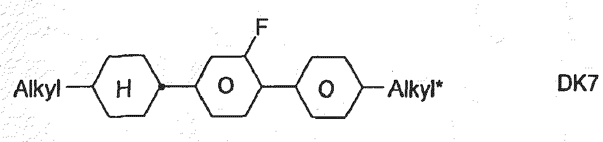 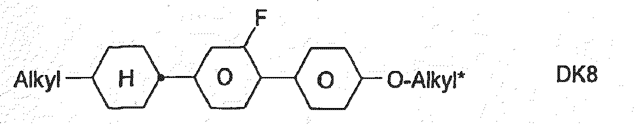 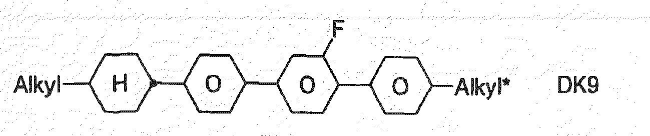 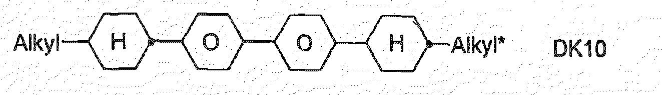 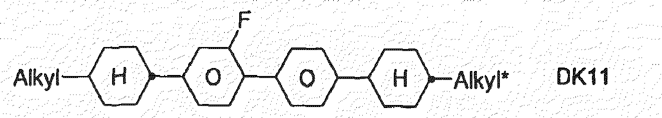 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
d) FK-Medium, welches zusätzlich eine oder mehrere Verbindungen der folgenden Formel enthält: 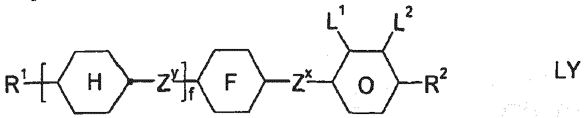 worin die einzelnen Reste folgende Bedeutung besitzen 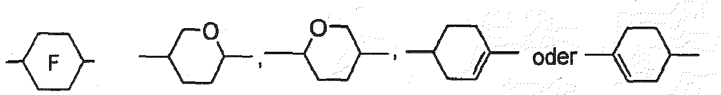
   - f: 0 oder 1,
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CF=CF-, -CO-, -O(CO)-oder -(CO)O- so ersetzt sein können, dass O-Atome nicht direkt miteinander verknüpft sind,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹ und L²: jeweils unabhängig voneinander F, Cl, OCF₃, CF₃, CH₃, CH₂F, CHF₂.

   Vorzugsweise bedeuten beide Reste L¹ und L² F oder einer der Reste L¹ und L² F und der andere Cl.
   Die Verbindungen der Formel LY sind vorzugsweise ausgewählt aus der Gruppe bestehend aus folgenden Unterformeln 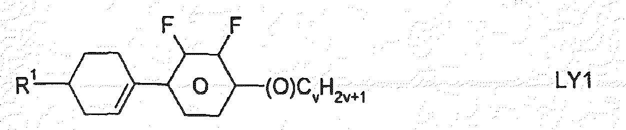 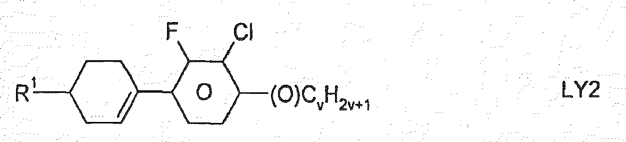 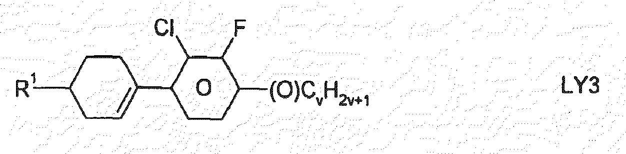 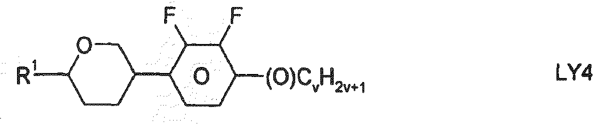 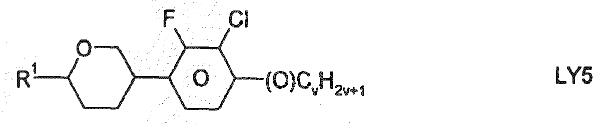 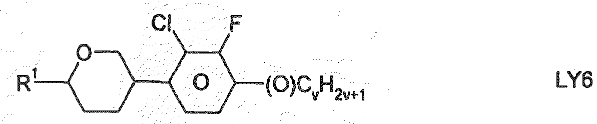 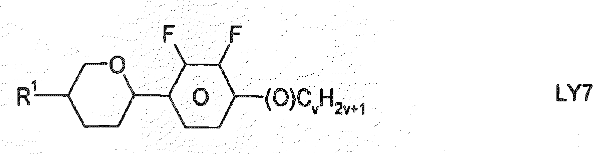 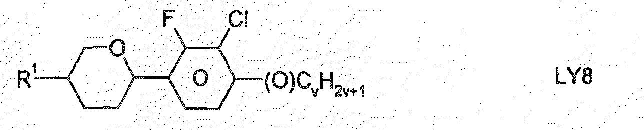 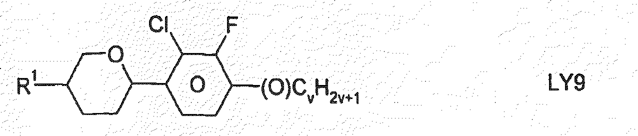 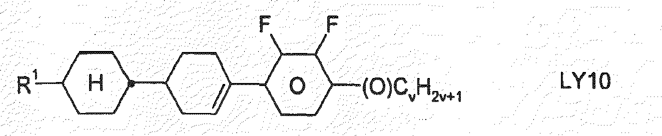 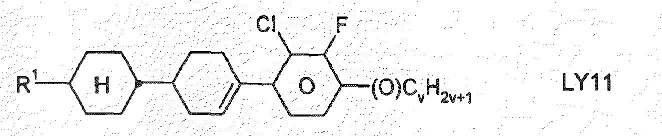 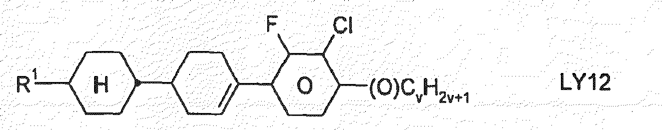 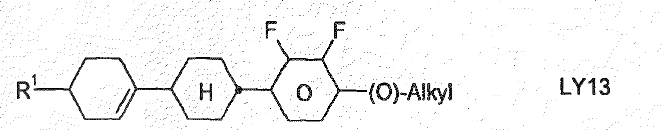 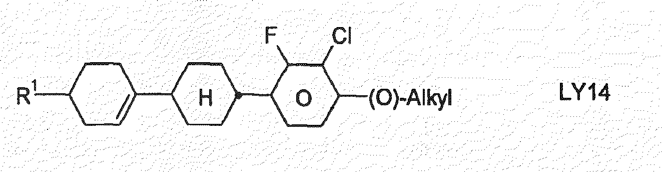 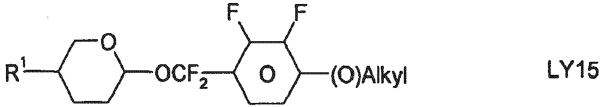 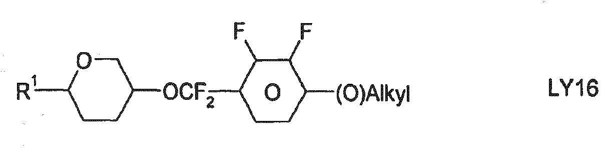 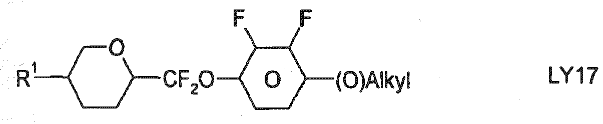 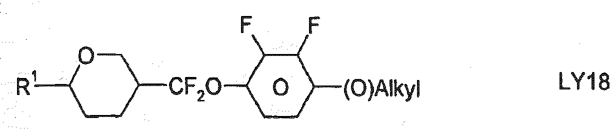 worin R¹ die oben angegebene Bedeutung hat, Alkyl einen geradkettigen Alkylrest mit 1-6 C-Atomen, (O) ein Sauerstoffatom oder eine Einfachbindung und v eine ganze Zahl von 1 bis 6 bedeuten. R¹ bedeutet vorzugsweise geradkettiges Alkyl mit 1 bis 6 C-Atomen oder geradkettiges Alkenyl mit 2 bis 6 C-Atomen, insbesondere CH₃, C₂H₅, n-C₃H₇, n-C₄H₉, n-C₅H₁₁, CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
e) FK-Medium, welches zusätzlich eine oder mehrere Verbindungen ausgewählt aus der Gruppe bestehend aus folgenden Formeln enthält: 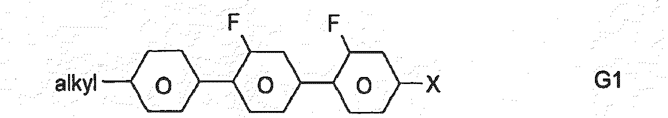 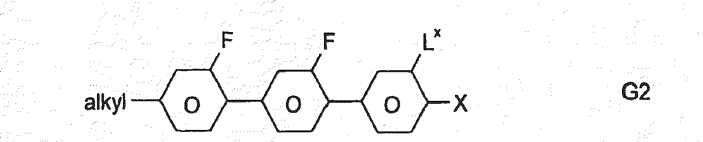 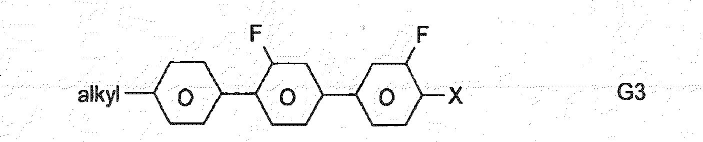 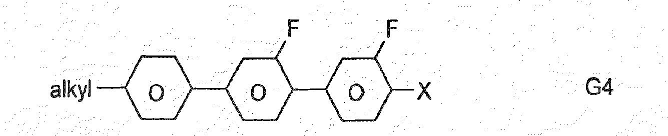 worin alkyl C₁-₆-alkyl, L^{x} H oder F und X F, Cl, OCF₃, OCHF₂ oder OCH=CF₂ bedeutet. Besonders bevorzugt sind Verbindungen der Formel G1, worin X F bedeutet.
f) FK-Medium, welches zusätzlich eine oder mehrere Verbindungen ausgewählt aus der Gruppe bestehend aus folgenden Formeln enthält: 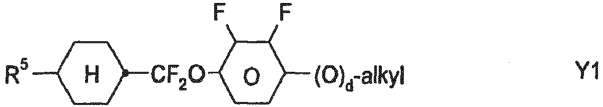 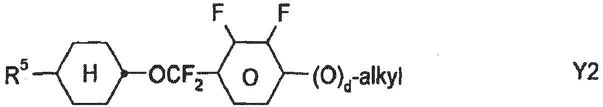 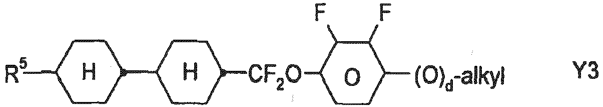 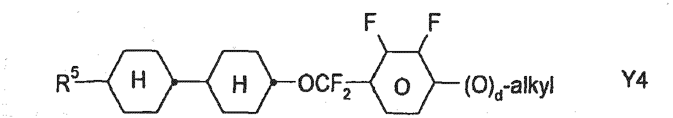 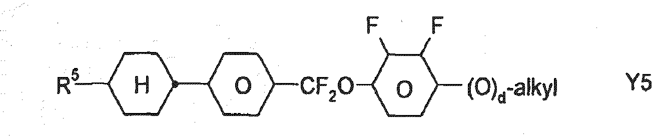 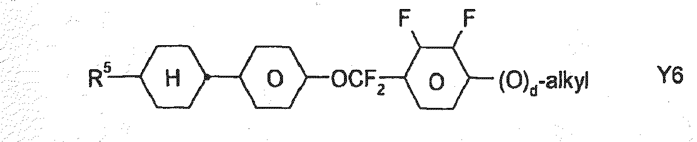 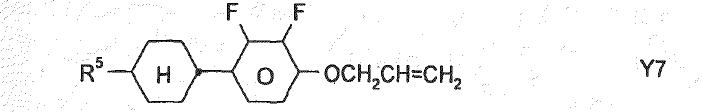 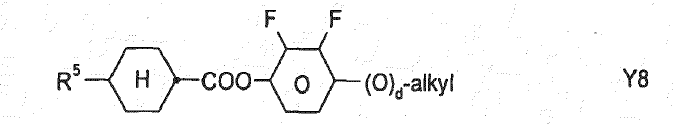 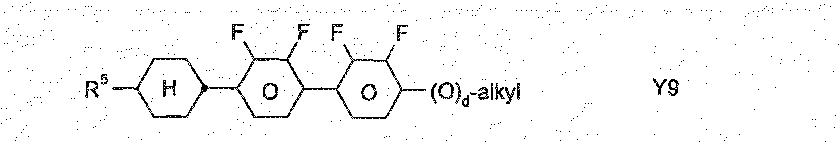 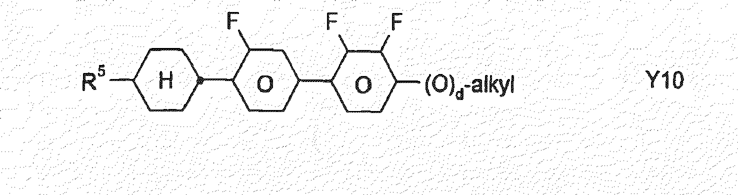 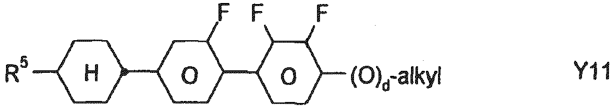 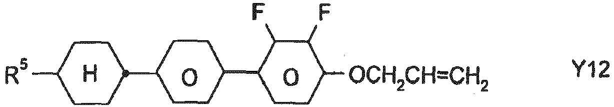 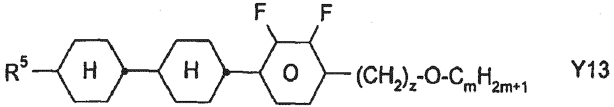 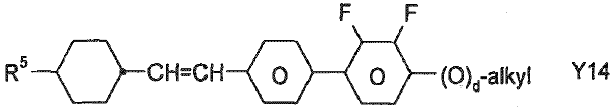 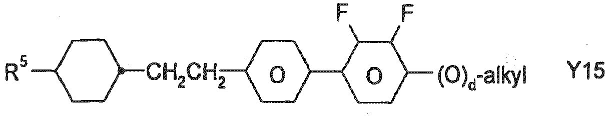 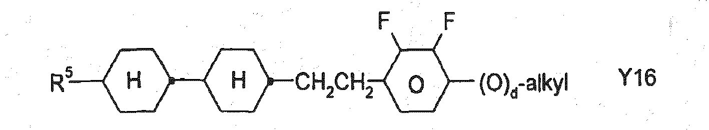 worin R⁵ eine der oben für R¹ angegebenen Bedeutungen besitzt, alkyl C₁₋₆-alkyl, d 0 oder 1, und z und m jeweils unabhängig voneinander eine ganze Zahl von 1 bis 6 bedeuten. R⁵ ist in diesen Verbindungen besonders bevorzugt C₁₋₆-alkyl oder -alkoxy oder C₂₋₆-alkenyl, d ist vorzugsweise 1. Vorzugsweise enthält das erfindungsgemäße FK-Medium eine oder mehrere Verbindungen der oben genannten Formeln in Mengen von ≥ 5 Gew.%.
g) FK-Medium, welches zusätzlich eine oder mehrere Biphenylverbindungen ausgewählt aus der Gruppe bestehend aus folgenden Formeln enthält: 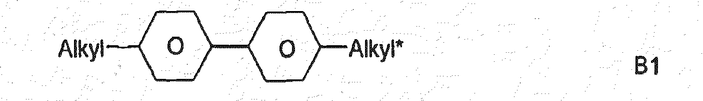 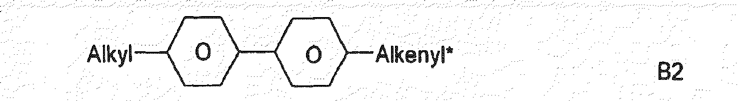 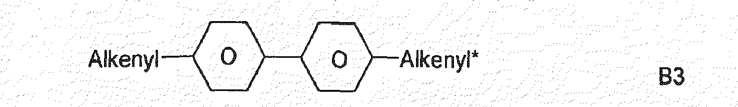 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Der Anteil der Biphenyle der Formeln B1 bis B3 in der FK-Mischung beträgt vorzugsweise mindestens 3 Gew.%, insbesondere ≥ 5 Gew.%. Die Verbindungen der Formel B2 sind besonders bevorzugt. Die Verbindungen der Formel B1 bis B3 sind vorzugsweise ausgewählt aus der Gruppe bestehend aus folgenden Unterformeln 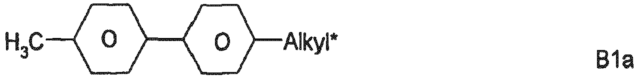 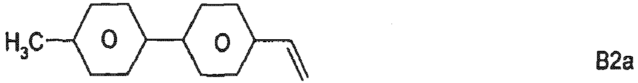 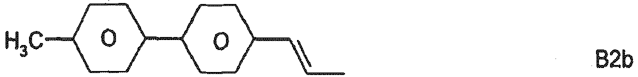 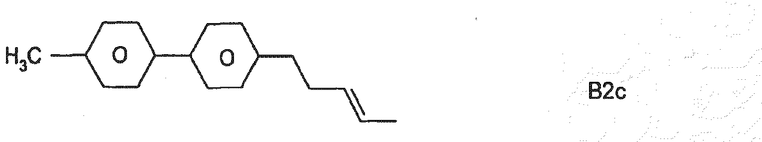 worin Alkyl* einen Alkylrest mit 1-6 C-Atomen bedeutet. Insbesondere bevorzugt enthält das erfindungsgemäße Medium eine oder mehrere Verbindungen der Formeln B1a und/oder B2c.
h) FK-Medium, welches zusätzlich eine oder mehrere Terphenylverbindungen der folgenden Formel enthält: 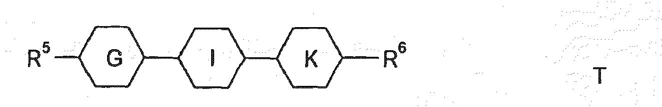 worin R⁵ und R⁶ jeweils unabhängig voneinander eine der oben für R¹ angegebenen Bedeutungen besitzen und 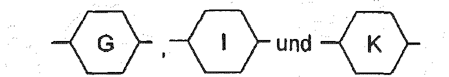 jeweils unabhängig voneinander 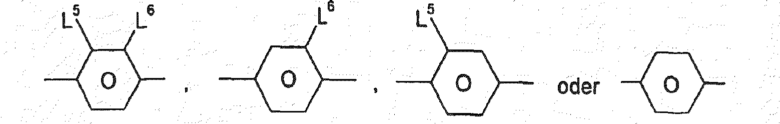 bedeuten, worin L⁵ F oder Cl, vorzugsweise F, und L⁶ F, Cl, OCF₃, CF₃, CH₃, CH₂F oder CHF₂, vorzugsweise F, bedeuten.
   Die Verbindungen der Formel T sind vorzugsweise ausgewählt aus der Gruppe bestehend aus folgenden Unterformeln 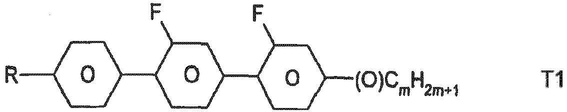 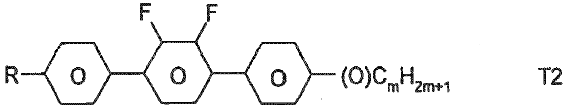 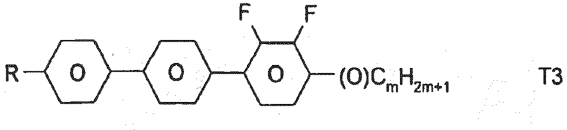 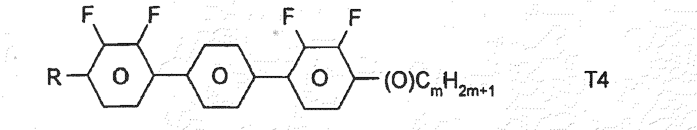 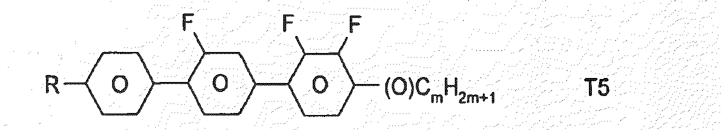 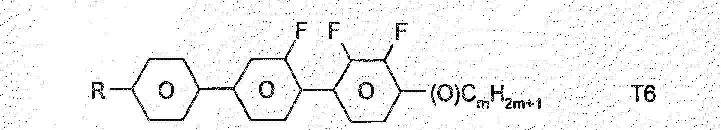 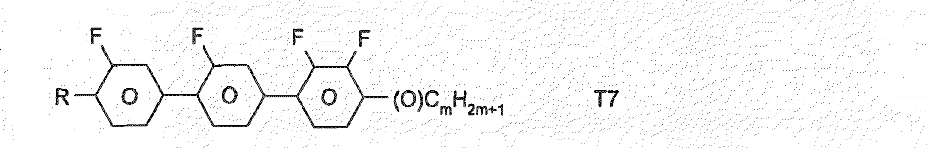 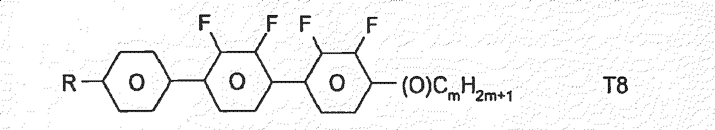 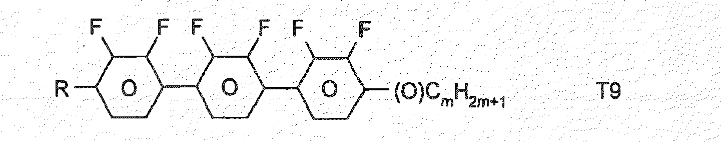 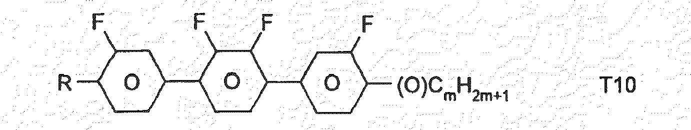 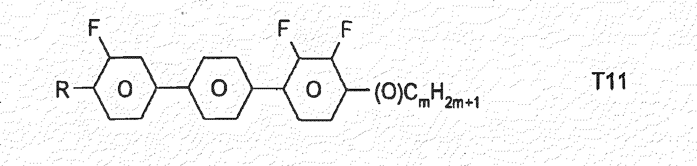 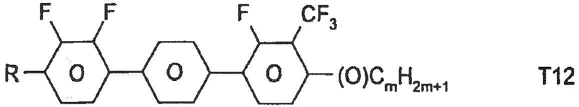 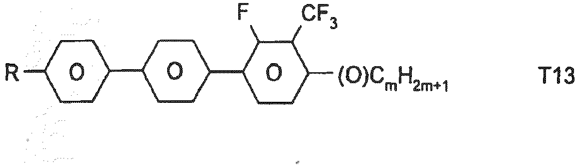 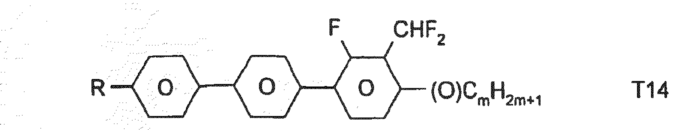 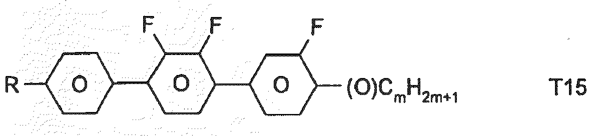 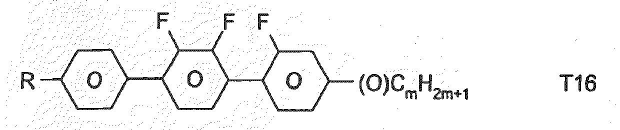 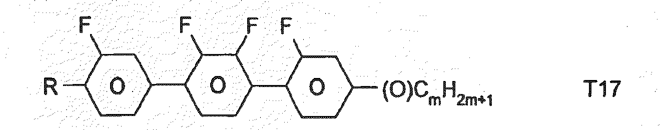 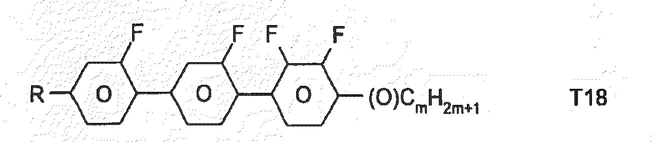 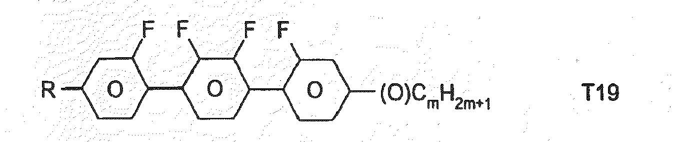 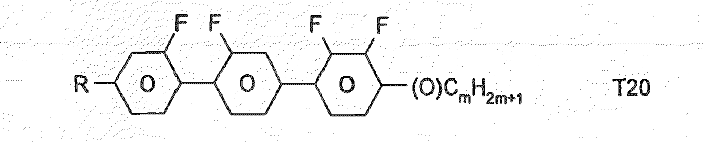 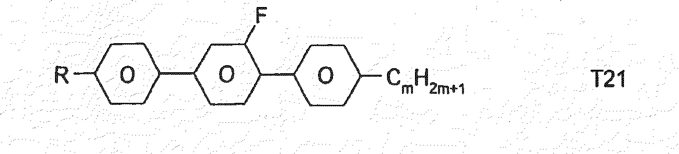 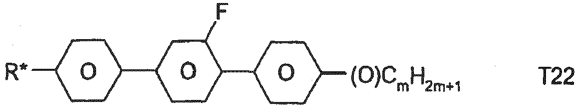 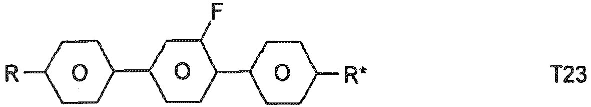 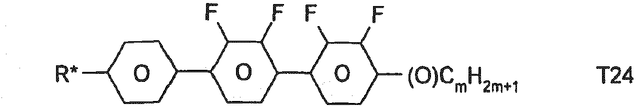 worin R einen geradkettigen Alkyl- oder Alkoxyrest mit 1-7 C-Atomen, R* einen geradkettigen Alkenylrest mit 2-7 C-Atomen, (O) ein Sauerstoffatom oder eine Einfachbindung, und m eine ganze Zahl von 1 bis 6 bedeutet. R* bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-. Vorzugsweise bedeutet R Methyl, Ethyl, Propyl, Butyl, Pentyl, Hexyl Methoxy, Ethoxy, Propoxy, Butoxy oder Pentoxy.
   Das erfindungsgemäße FK-Medium enthält die Terphenyle der Formeln T und deren bevorzugte Unterformeln vorzugsweise in einer Menge von 0,5-30 Gew.%, insbesondere von 1-20 Gew.%. Besonders bevorzugt sind Verbindungen der Formeln T1, T2, T3 und T21. In diesen Verbindungen bedeutet R vorzugsweise Alkyl, ferner Alkoxy jeweils mit 1-5 C-Atomen. Vorzugsweise werden die Terphenyle in erfindungsgemäßen Mischungen eingesetzt, wenn der Δn-Wert der Mischung ≥ 0,1 sein soll. Bevorzugte Mischungen enthalten 2-20 Gew.% einer oder mehrerer TerphenylVerbindungen der Formel T, vorzugsweise ausgewählt aus der Gruppe der Verbindungen T1 bis T22.
i) FK-Medium, welches zusätzlich eine oder mehrere Verbindungen ausgewählt aus der Gruppe bestehend aus folgenden Formeln enthält: 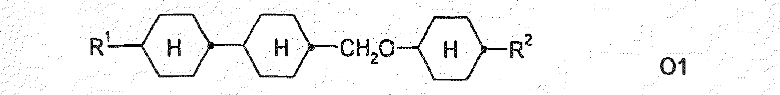 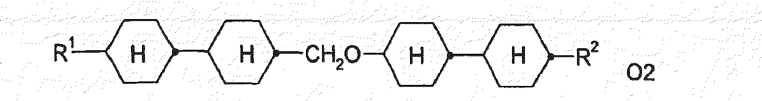 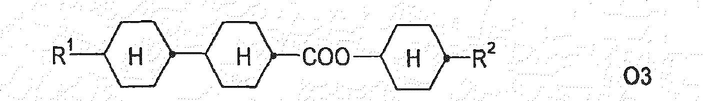 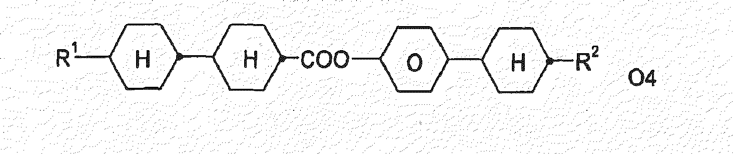 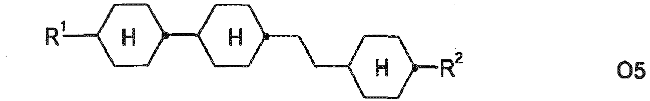 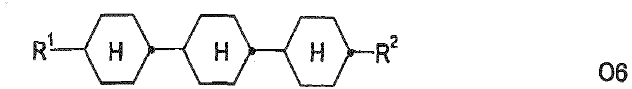 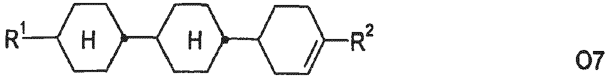 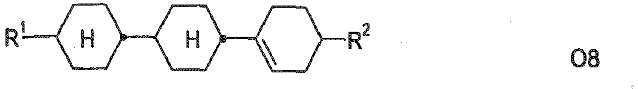 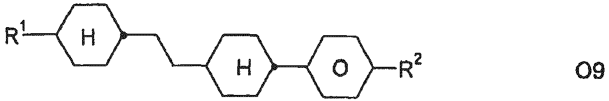 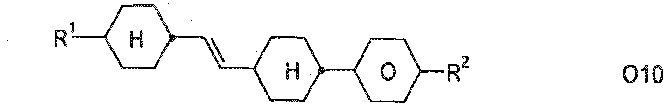 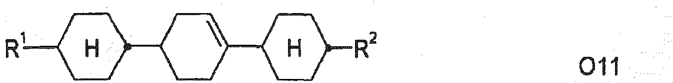 worin R¹ und R² die oben für Formel LY angegebenen Bedeutungen haben, und vorzugsweise jeweils unabhängig voneinander geradkettiges Alkyl mit 1 bis 6 C-Atomen oder geradkettiges Alkenyl mit 2 bis 6 C-Atomen bedeuten.
   Bevorzugte Medien enthalten eine oder mehrere Verbindungen ausgewählt aus den Formeln 01, 03 und 04.
k) FK-Medium, welches zusätzlich eine oder mehrere Verbindungen der folgenden Formel enthält: 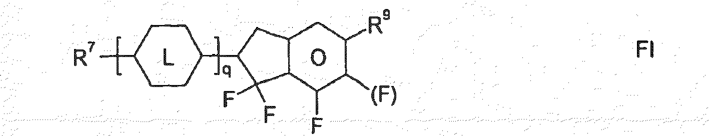 worin 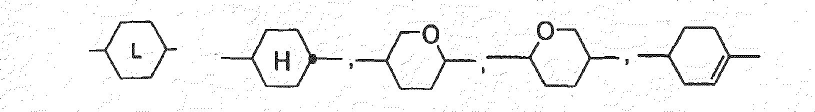 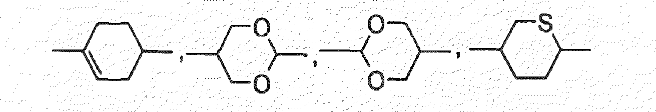 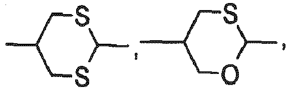 R⁹ H, CH₃, C₂H₅ oder n-C₃H₇, (F) einen optionalen Fluor-substituenten und q 1, 2 oder 3 bedeutet, und R⁷ eine der für R¹ angegebenen Bedeutungen hat, vorzugsweise in Mengen von > 3 Gew.%, insbesondere ≥ 5 Gew.%, und ganz besonders bevorzugt von 5-30 Gew.%.
   Besonders bevorzugte Verbindungen der Formel FI sind ausgewählt aus der Gruppe bestehend aus folgenden Unterformeln: 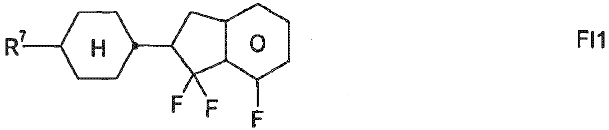 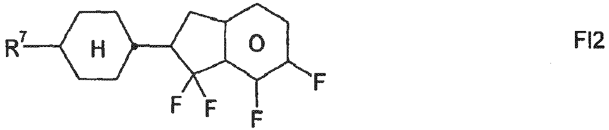 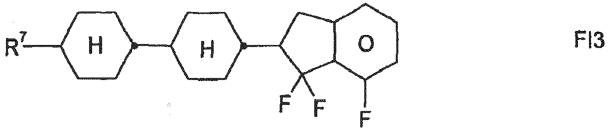 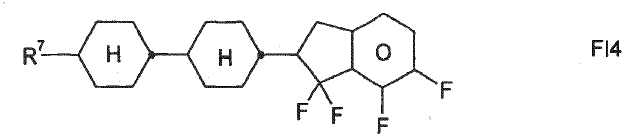 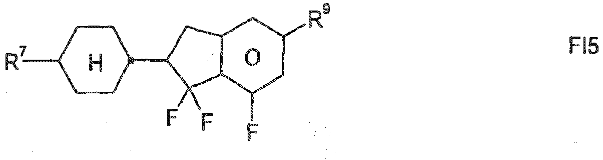 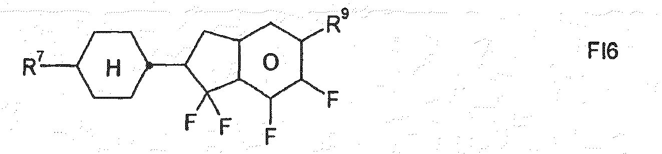 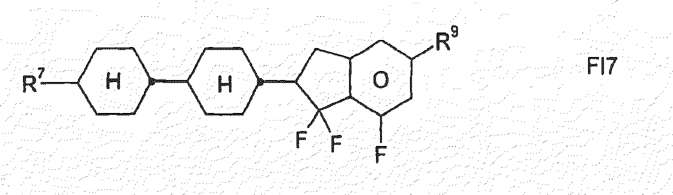 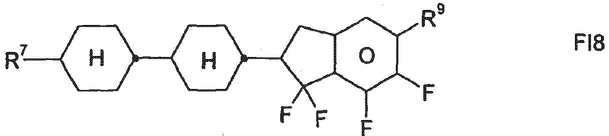 worin R⁷ vorzugsweise geradkettiges Alkyl bedeutet und R⁹ CH₃, C₂H₅ oder n-C₃H₇ bedeutet. Besonders bevorzugt sind die Verbindungen der Formel FI1, FI2 und FI3.
m) FK-Medium, welches zusätzlich eine oder mehrere Verbindungen ausgewählt aus der Gruppe bestehend aus folgenden Formeln enthält: 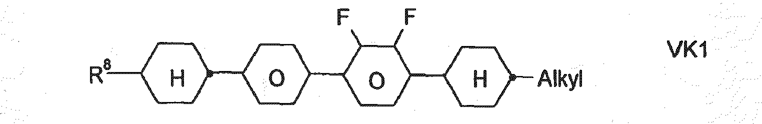 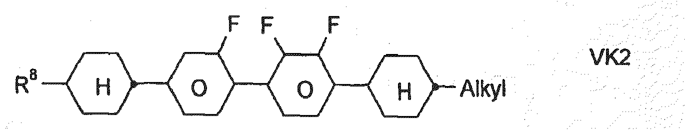 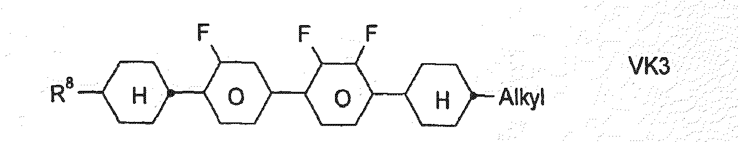 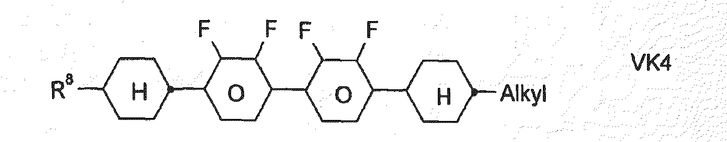 worin R⁸ die für R¹ für Formel LY angegebene Bedeutung hat und Alkyl einen geradkettigen Alkylrest mit 1-6 C-Atomen bedeutet.
n) FK-Medium, welches zusätzlich eine oder mehrere Verbindungen enthält, die eine Tetrahydronaphthyl- oder Naphthyl-Einheit aufweisen, wie z.B. die Verbindungen ausgewählt aus der Gruppe bestehend aus folgenden Formeln: 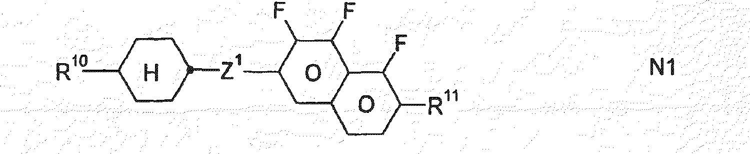 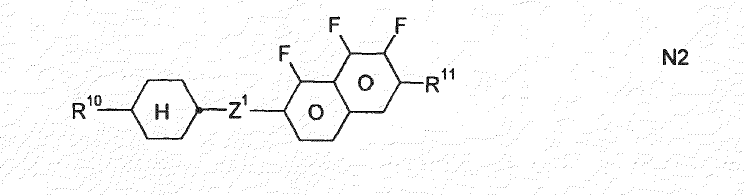 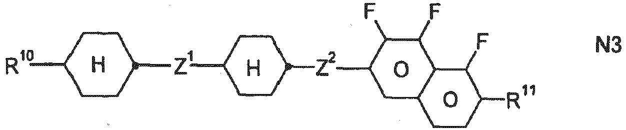 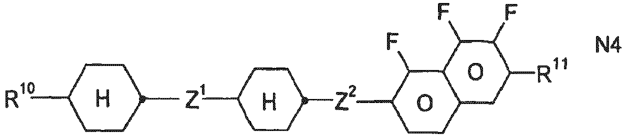 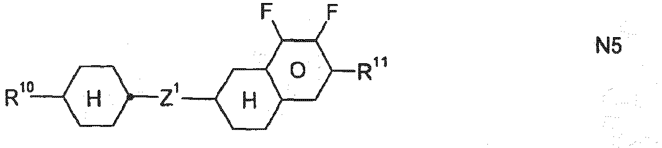 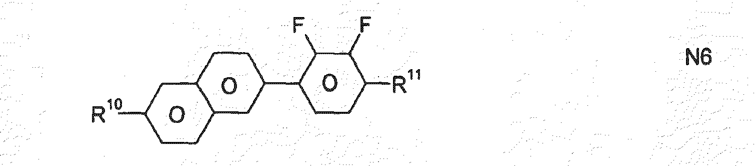 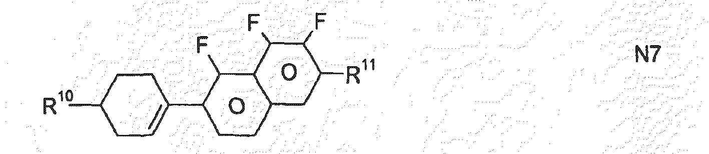 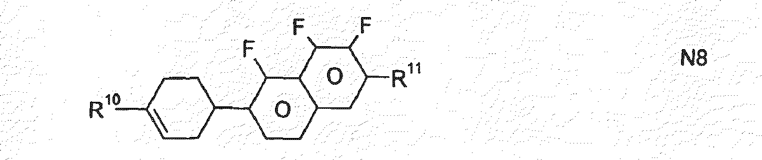 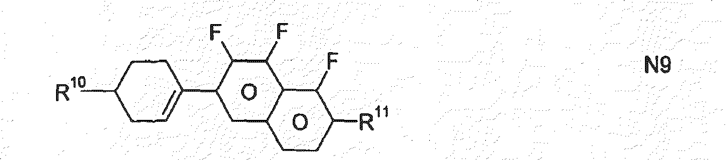 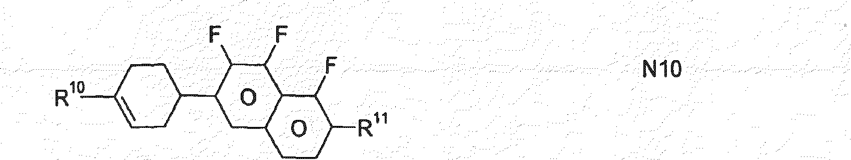 worin R¹⁰ und R¹¹ jeweils unabhängig voneinander eine der für R¹ für Formel LY angegebenen Bedeutungen haben, vorzugsweise geradkettiges Alkyl oder Alkoxy mit 1 bis 6 C-Atomen oder geradkettiges Alkenyl mit 2 bis 6 C-Atomen bedeuten, und Z¹ und Z² jeweils unabhängig voneinander -C₂H₄-, -CH=CH-, -(CH₂)₄-, -(CH₂)₃O- -O(CH₂)₃-, -CH=CH-CH₂CH₂-, -CH₂CH₂CH=CH-, -CH₂O-, -OCH₂-, -CO-O-, -O-CO-, -C₂F₄-, -CF=CF-, -CF=CH-, -CH=CF-, -CH₂- oder eine Einfachbindung bedeuten.
o) FK-Medium, welches zusätzlich eine oder mehrere eine oder mehrere Difluordibenzochromane und/oder Chromane der folgenden Formeln enthält: 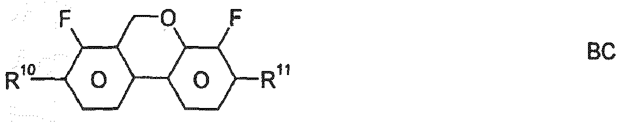 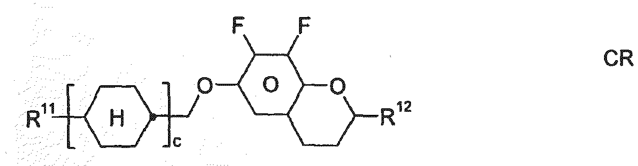 worin R¹⁰ und R¹¹ jeweils unabhängig voneinander die oben für R¹ in Formel LY angegebene Bedeutung aufweisen, und c 0 oder 1 bedeutet, vorzugsweise in Mengen von 3 bis 20 Gew.%, insbesondere in Mengen von 3 bis 15 Gew.%.
   Besonders bevorzugte Verbindungen der Formeln BC und CR sind ausgewählt aus der Gruppe bestehend aus folgenden Unterformeln: 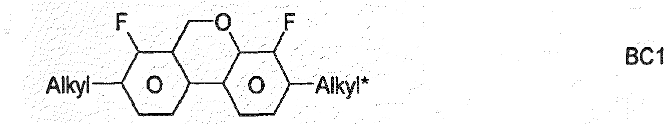 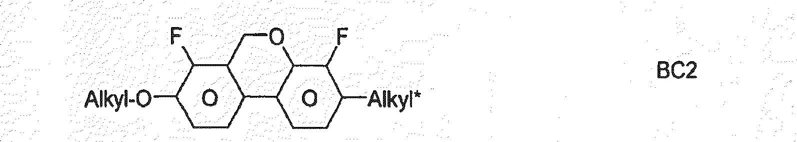 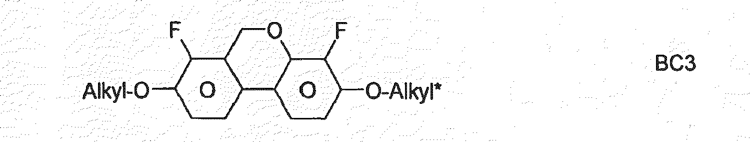 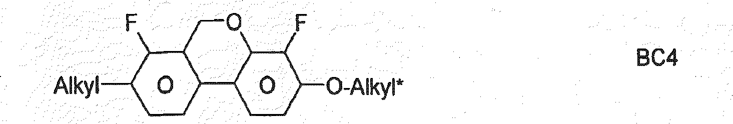 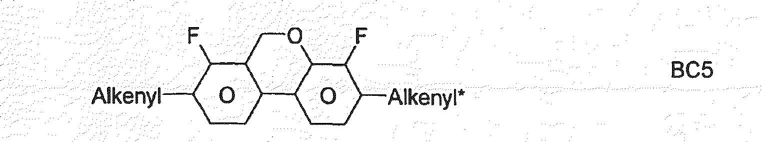 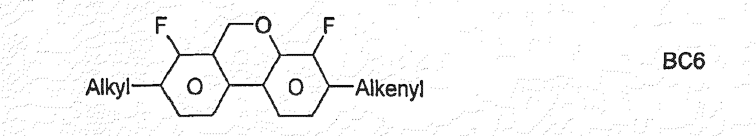 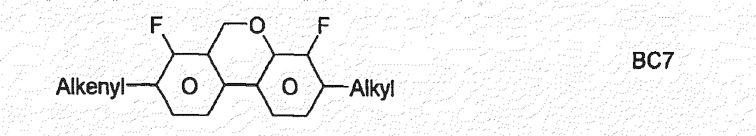 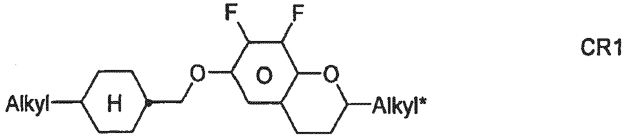 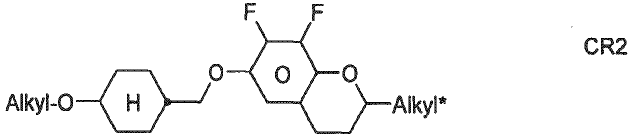 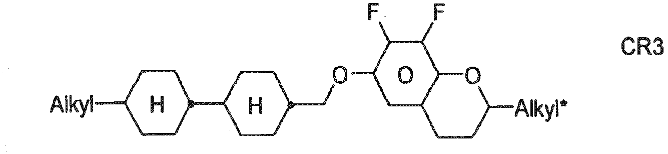 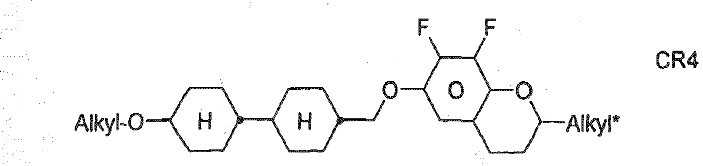 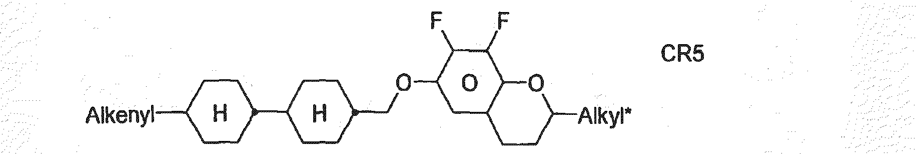 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH-oder CH₃-CH=CH-(CH₂)₂-. Ganz besonders bevorzugt sind Mischungen enthaltend eine, zwei oder drei Verbindungen der Formel BC-2.
p) FK-Medium, welches zusätzlich eine oder mehrere fluorierte Phenanthrene und/oder Dibenzofurane der folgenden Formeln enthält: 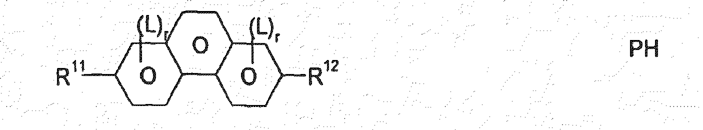 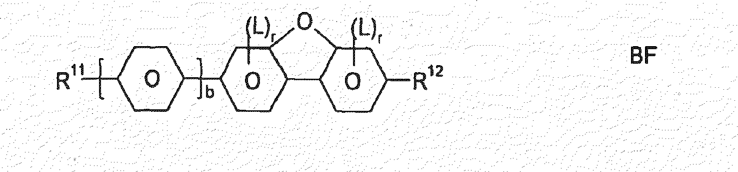 worin R¹¹ und R¹² jeweils unabhängig voneinander die oben für R¹ in Formel LY angegebenen Bedeutungen besitzen, b 0 oder 1, L F und r 1, 2 oder 3 bedeutet.
   Besonders bevorzugte Verbindungen der Formeln PH und BF sind ausgewählt aus der Gruppe bestehend aus folgenden Unterformeln: 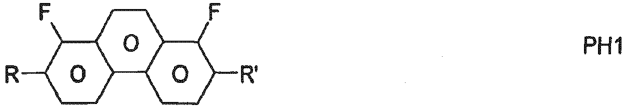 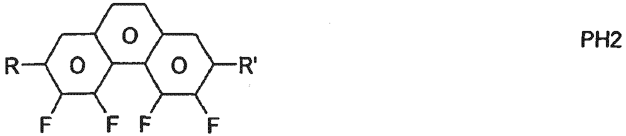 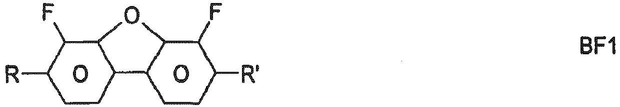 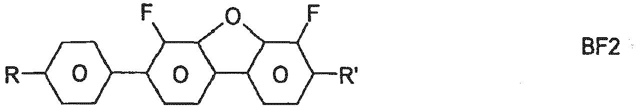 worin R und R¹ jeweils unabhängig voneinander einen geradkettigen Alkyl- oder Alkoxyrest mit 1-7 C-Atomen bedeuten.

Die erfindungsgemäße Flüssigkristallmischung ist mit einer dielektrischen Anisotropie (Δε) von ≤-1,5 dielektrisch negativ. Die Verbindungen der Formeln IIIA , IIIB, IIIC, LY1-LY18, Y1-Y16, T1-T24, FI, VK1-VK4, N1-N10, BC, CR, PH und BF eignen sich als dielektrisch negative Komponente. Bevorzugt sind die dielektrisch negativen Verbindungen der Formeln ausgewählt aus den Formeln IIIA , IIIB und IIIC. Das FK-Medium weist vorzugsweise ein Δε von -1,5 bis -8,0, insbesondere von -2,5 bis -6,0 auf.

Die Werte der Doppelbrechung Δn in der Flüssigkristallmischung liegen in der Regel zwischen 0,07 und 0,16, vorzugsweise zwischen 0,08 und 0,12. Die Rotationsviskosität γ₁ bei 20 °C vor der Polymerisation ist vorzugsweise ≤ 165 mPa·s, insbesondere ≤ 140 mPa·s.
- Das Medium enthält vorzugsweise 20-70 Gew.%, besonders bevorzugt 25-60 Gew.% an Verbindungen der Formel IIa;
- Das Medium enthält vorzugsweise 2-25 Gew.%, besonders bevorzugt 3-20 Gew.% ausgewählt aus der Gruppe der Verbindungen der VI-2;
- Das Medium enthält insgesamt 2-30 Gew.%, besonders bevorzugt 3-20 Gew.% an Verbindungen der Formeln XI und XXVII zusammen;
- Das Medium enthält vorzugsweise 1-20 Gew.%, besonders bevorzugt 2-15 Gew.% an Verbindungen der Formel XXIV;
- Das Medium enthält insgesamt 15-65 Gew.%, besonders bevorzugt 30-55 Gew.% ausgewählt aus den hochpolaren Verbindungen der Formeln VI-2, X, XI und XXV zusammen.

Die nematische Phase des dielektrisch negativen FK-Mediums gemäß der Erfindung hat bevorzugt eine nematische Phasen in einem Temperaturbereich von 10 °C oder weniger bis 60 °C oder mehr, besonders bevorzugt von 0 oder weniger bis 70 °C oder mehr.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n, m, z und k sind ganze Zahlen und bedeuten vorzugsweise 1, 2, 3, 4, 5, 6, 7, 8, 9, 10, 11 oder 12. Die Codierung gemäß Tabelle B versteht sich von selbst. In Tabelle A' ist nur das Akronym für den Grundkörper angegeben. Im Einzelfall folgt getrennt von Akronym für den Grundkörper mit einem Strich ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nOCF₃ | CₙH₂ₙ₊₁ | OCF₃ | H | H |
| nOCF₃.F | CₙH₂ₙ₊₁ | OCF₃ | F | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

**Tabelle A**

| | |
|---|---|
| 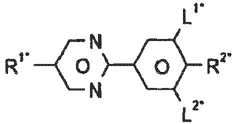 | 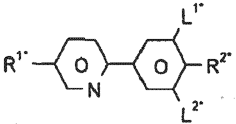 |
| **PYP** | **PYRP** |
| 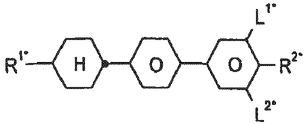 | 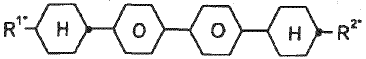 |
| **BCH** | **CBC** |
| 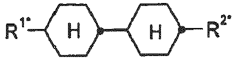 | 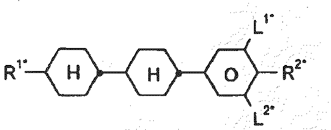 |
| **CCH** | **CCP** |
| 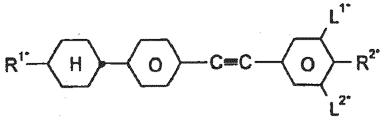 | 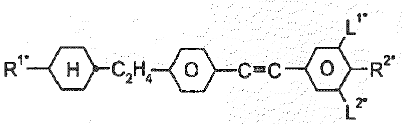 |
| **CPTP** | **CEPTP** |
| 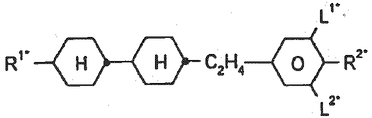 | 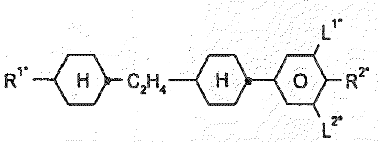 |
| **ECCP** | **CECP** |
| 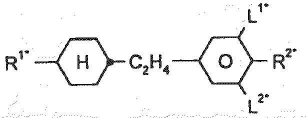 | 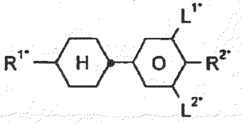 |
| **EPCH** | **PCH** |
| 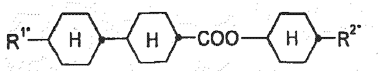 | |
| **CH** | |
| 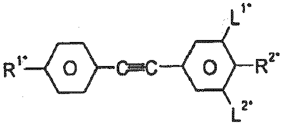 | 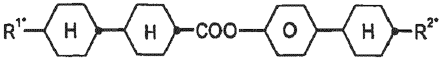 |
| **PTP** | **CCPC** |
| 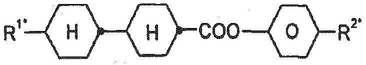 | 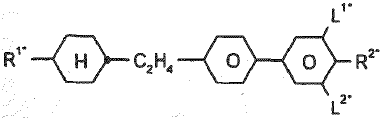 |
| **CP** | **BECH** |
| 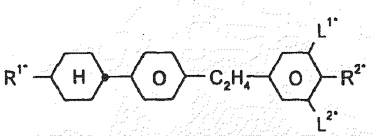 | 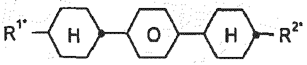 |
| **EBCH** | **CPC** |
| 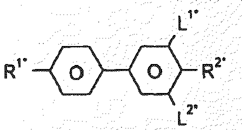 | 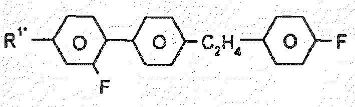 |
| **B** | **FET-nF** |
| 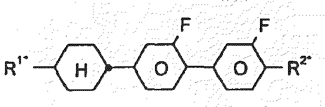 | 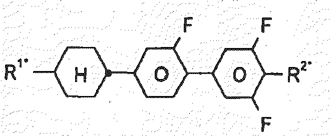 |
| **CGG** | **CGU** |
| 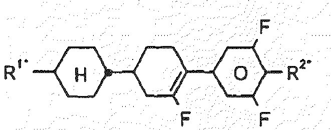 | |
| **CFU** | |

**Tabelle B**

| n, m, z bedeuten unabhängig voneinander vorzugsweise 1, 2, 3, 4, 5 oder 6. | |
|---|---|
| 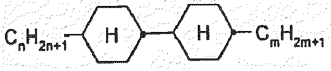 | 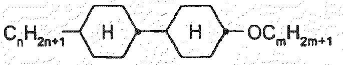 |
| CCH-nm | CCH-nOm |
| 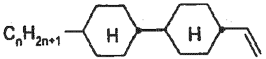 | 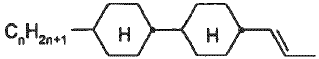 |
| CC-n-V | CC-n-V1 |
| 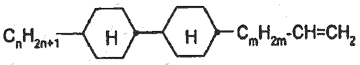 | 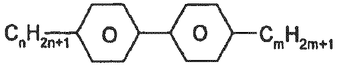 |
| CC-n-mV | PP-n-m |
| 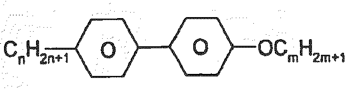 | 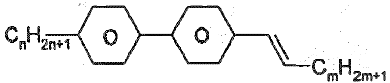 |
| PP-n-Om | PP-n-Vm |
| 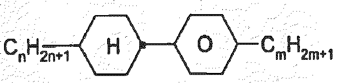 | 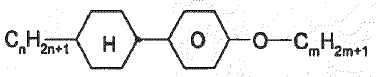 |
| PCH-nm | PCH-nOm |
| 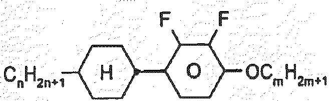 | 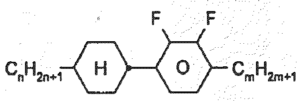 |
| CY-n-Om | CY-n-m |
| 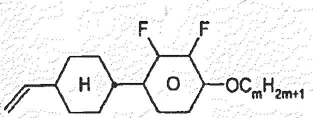 | 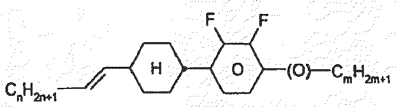 |
| CY-V-Om | CY-nV-(O)m |
| 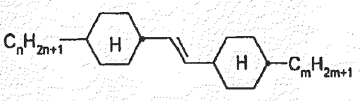 | 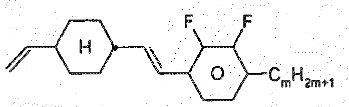 |
| CVC-n-m | CVY-V-m |
| 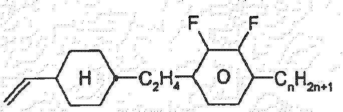 | 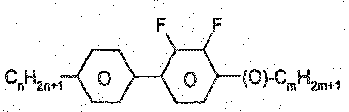 |
| CEY-V-m | PY-n-(O)m |
| 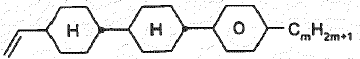 | 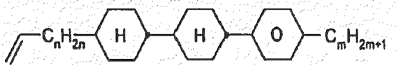 |
| CCP-V-m | CCP-Vn-m |
| 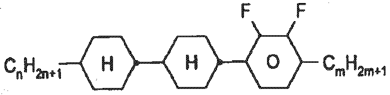 | 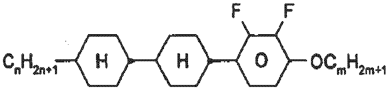 |
| CCY-n-m | CCY-n-Om |
| 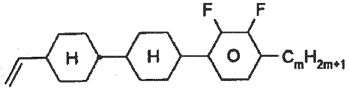 | 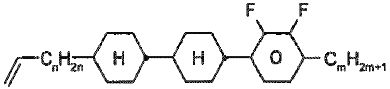 |
| CCY-V-m | CCY-Vn-m |
| 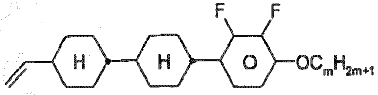 | 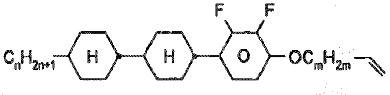 |
| CCY-V-Om | CCY-n-OmV |
| 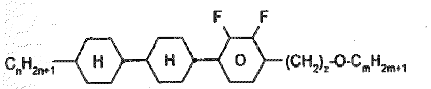 | 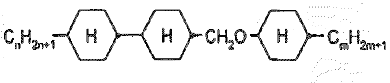 |
| CCY-n-zOm | CCOC-n-m |
| 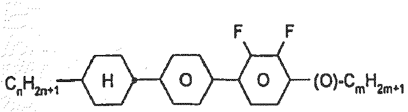 | 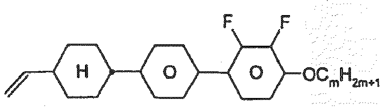 |
| CPY-n-(O)m | CPY-V-Om |
| 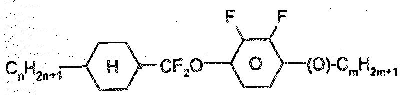 | 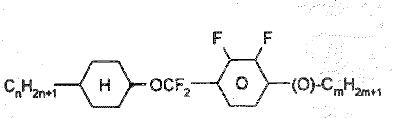 |
| CQY-n-(O)m | CQIY-n-(O)m |
| 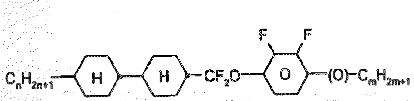 | 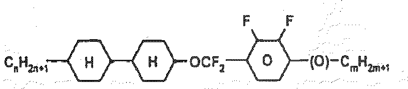 |
| CCQY-n-(O)m | CCQIY-n-(O)m |
| 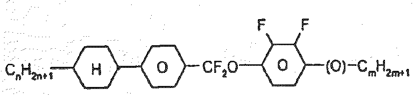 | 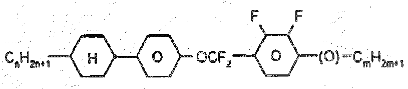 |
| CPQY-n-(O)m | CPQIY-n-Om |
| 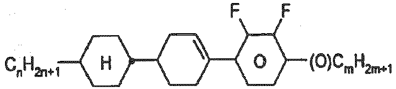 | 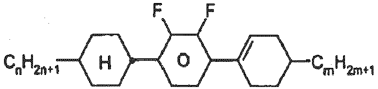 |
| CLY-n-(O)m | CYLI-n-m |
| 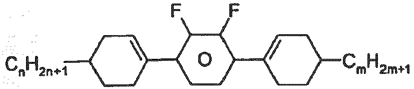 | 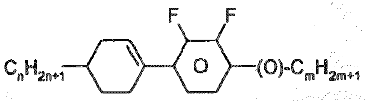 |
| LYLI-n-m | LY-n-(O)m |
| 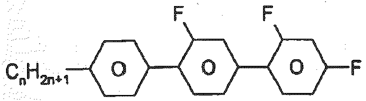 | 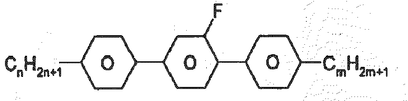 |
| PGIGI-n-F | PGP-n-m |
| 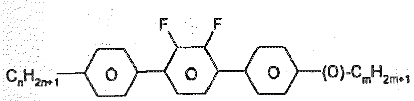 | 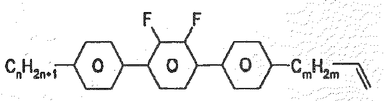 |
| PYP-n-(O)m | PYP-n-mV |
| 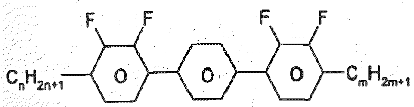 | 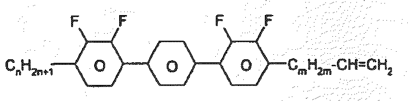 |
| YPY-n-m | YPY-n-mV |
| 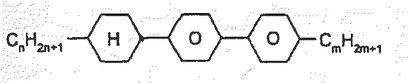 | 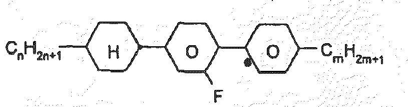 |
| BCH-nm | BCH-nmF |
| 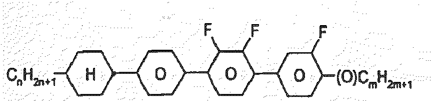 | 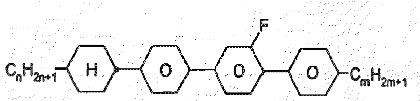 |
| CPYP-n-(O)m | CPGP-n-m |
| 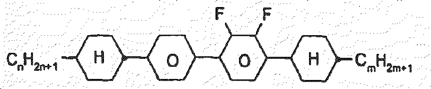 | 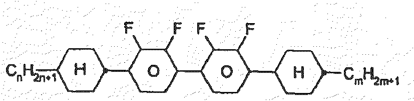 |
| CPYC-n-m | CYYC-n-m |
| 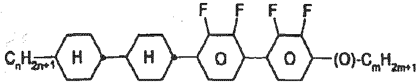 | 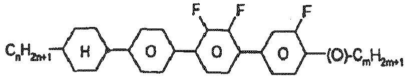 |
| CCYY-n-m | CPYG-n-(O)m |
| 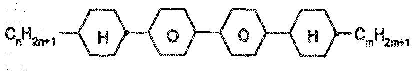 | 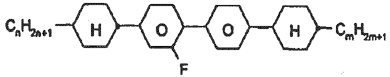 |
| CBC-nm | CBC-nmF |
| 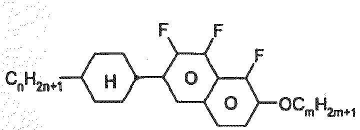 | 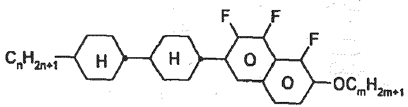 |
| CNap-n-Om | CCNap-n-Om |
| 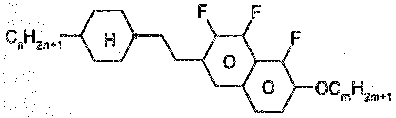 | 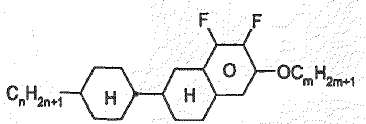 |
| CENap-n-Om | CTNap-n-Om |
| 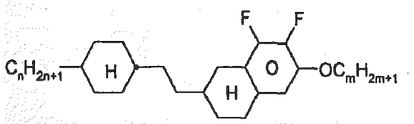 | 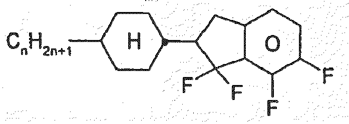 |
| CETNap-n-Om | CK-n-F |
| 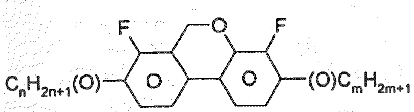 | 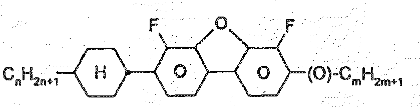 |
| DFDBC-n(O)-(O)m | C-DFDBF-n-(O)m |
| 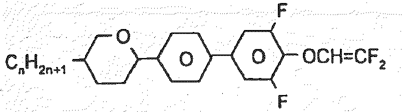 | 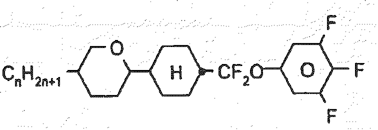 |
| **APU-n-OXF** | **ACQU-n-F** |
| 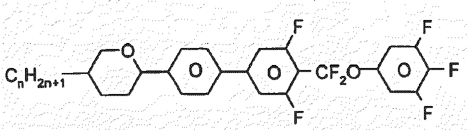 | |
| **APUQU-n-F** | |
| 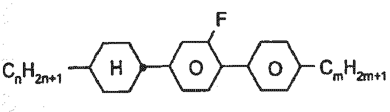 | 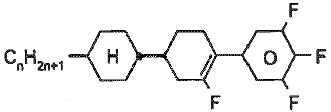 |
| **BCH-n.Fm** | **CFU-n-F** |
| 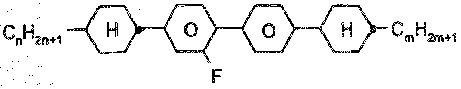 | |
| **CBC-nmF** | |
| 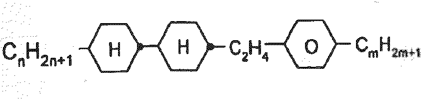 | 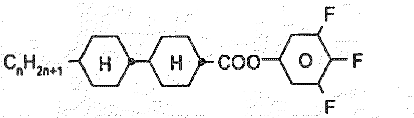 |
| **ECCP-nm** | **CCZU-n-F** |
| 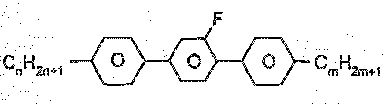 | 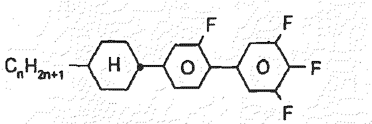 |
| **PGP-n-m** | **CGU-n-F** |
| 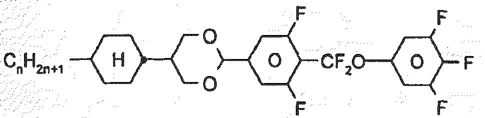 | |
| **CDUQU-n-F** | |
| 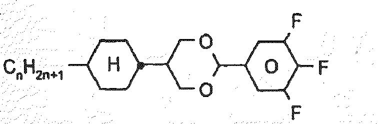 | 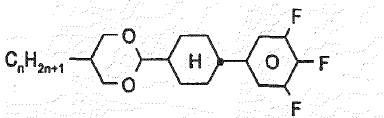 |
| C**DU-n-F** | **DCU-n-F** |
| 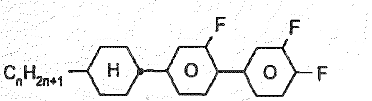 | 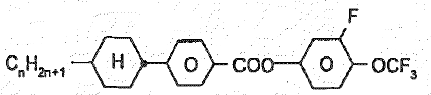 |
| **CGG-n-F** | **CPZG-n-OT** |
| 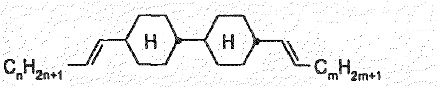 | |
| **CC-nV-Vm** | |
| 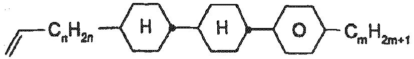 | 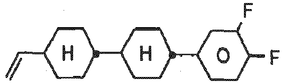 |
| **CCP-Vn-m** | **CCG-V-F** |
| 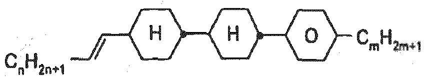 | |
| **CCP-nV-m** | |
| 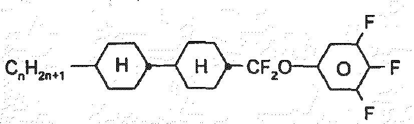 | 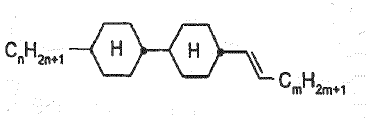 |
| **CCQU-n-F** | **CC-n-Vm** |
| 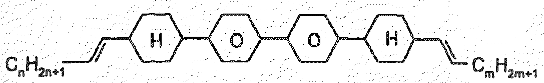 | |
| **CPPC-nV-Vm** | |
| 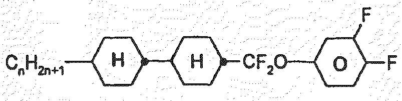 | 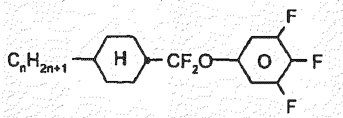 |
| **CCQG-n-F** | **CQU-n-F** |
| 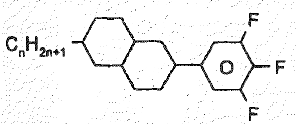 | 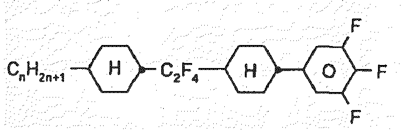 |
| **Dec-U-n-F** | **CWCU-n-F** |
| 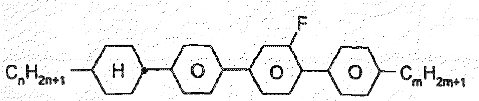 | |
| **CPGP-n-m** | |
| 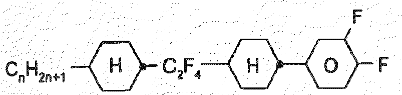 | |
| **CWCG-n-F** | |
| 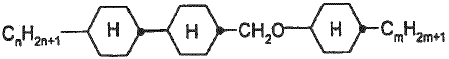 | |
| **CCOC-n-m** | |
| 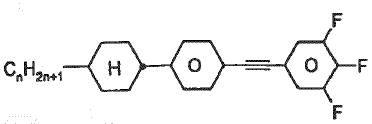 | 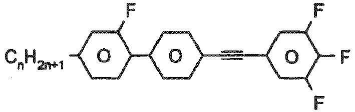 |
| **CPTU-n-F** | **GPTU-n-F** |
| 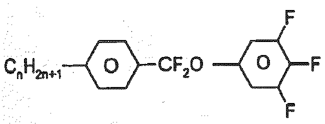 | 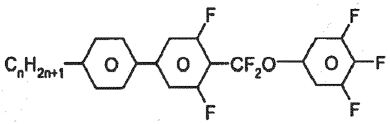 |
| **PQU-n-F** | **PUQU-n-F** |
| 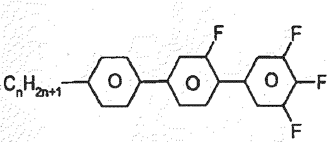 | 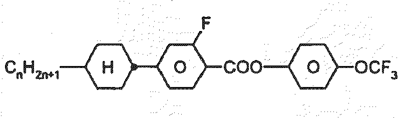 |
| **PGU-n-F** | **CGZP-n-OT** |
| 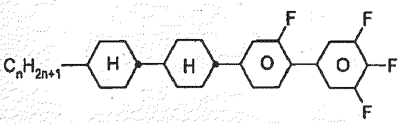 | 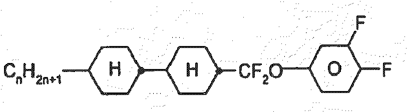 |
| **CCGU-n-F** | **CCQG-n-F** |
| 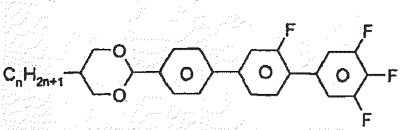 | 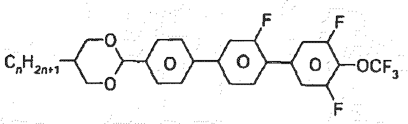 |
| **DPGU-n-F** | **DPGU-n-OT** |
| 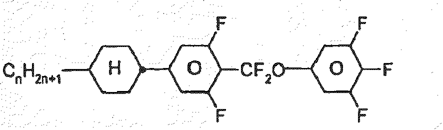 | |
| **CUQU-n-F** | |
| 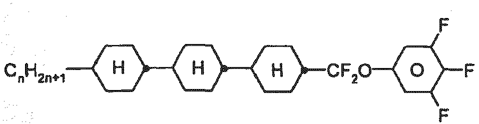 | |
| **CCCQU-n-F** | |
| 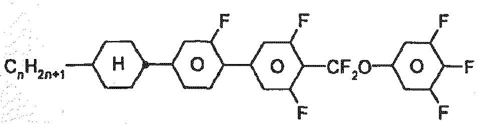 | |
| **CGUQU-n-F** | |
| 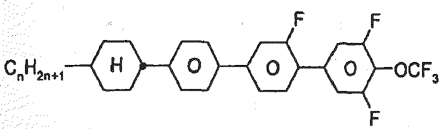 | |
| **CPGU-n-OT** | |
| 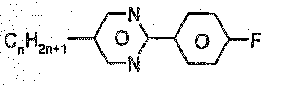 | 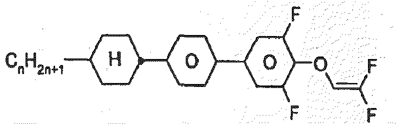 |
| **PYP-n-F** | **CPU-n-OXF** |
| 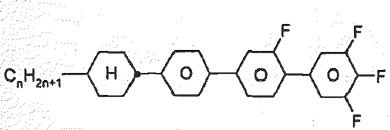 | 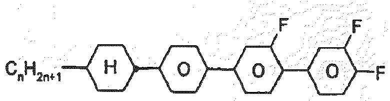 |
| **CPGU-n-F** | **CPGG-n-F** |
| 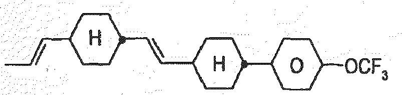 | 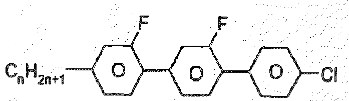 |
| **CVCP-1V-OT** | **GGP-n-Cl** |
| 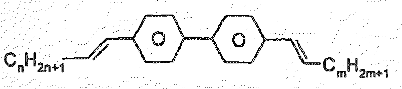 | 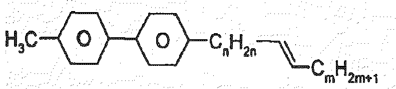 |
| **PP-nV-Vm** | **PP-1-nVm** |
| 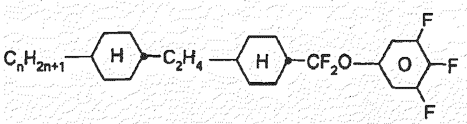 | |
| **CWCQU-n-F** | |
| 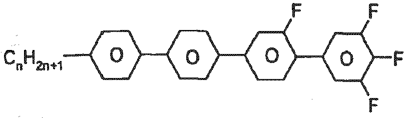 | |
| **PPGU-n-F** | |
| 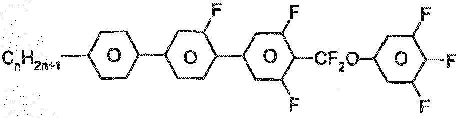 | |
| **PGUQU-n-F** | |
| 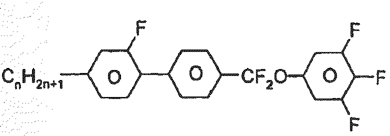 | 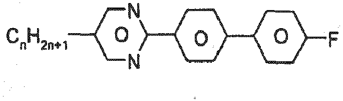 |
| **GPQU-n-F** | **MPP-n-F** |
| 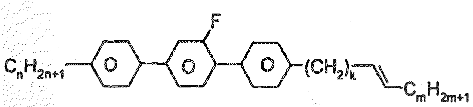 | |
| **PGP-n-kVm** | |
| 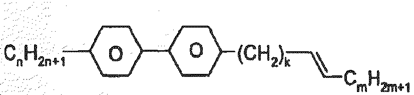 | |
| **PP-n-kVm** | |
| 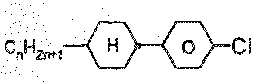 | 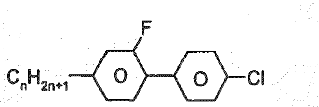 |
| **PCH-nCl** | **GP-n-Cl** |
| 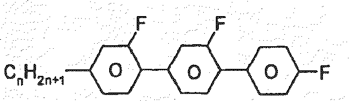 | 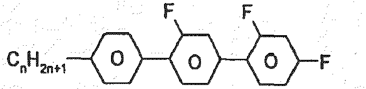 |
| **GGP-n-F** | **PGIGI-n-F** |
| 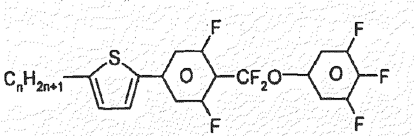 | |
| **SUQU-n-F** | |
| 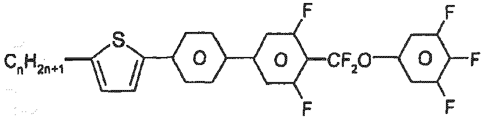 | |
| **SPUQU-n-F** | |

In einer bevorzugten Ausführungsform der vorliegenden Erfindung enthalten die erfindungsgemäßen LC-Medien eine oder mehrere Verbindungen ausgewählt aus der Gruppe bestehend aus Verbindungen der Tabelle A und B.

**Tabelle C**

| In der Tabelle C werden mögliche chirale Dotierstoffe angegeben, die den erfindungsgemäßen FK-Medien zugesetzt werden können. | |
|---|---|
| 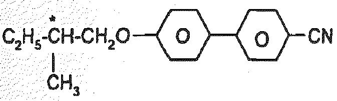 | 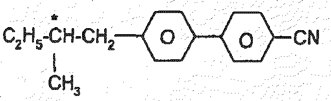 |
| **C 15** | **CB 15** |
| 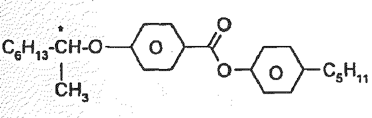 | 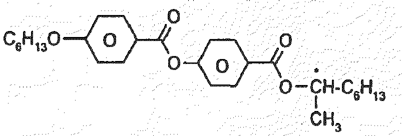 |
| **CM 21** | **R/S-811** |
| 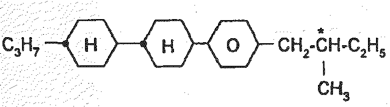 | 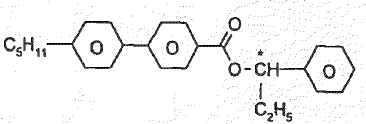 |
| **CM 44** | **CM 45** |
| 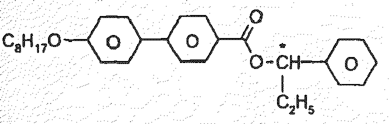 | 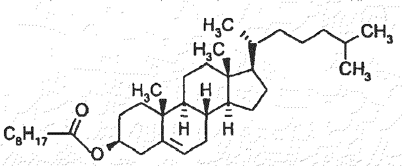 |
| **CM 47** | **CN** |
| 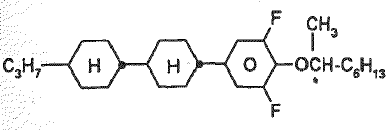 | 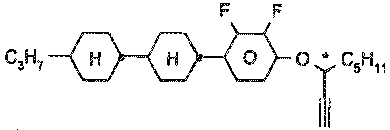 |
| **R/S-2011** | **R/S-3011** |
| 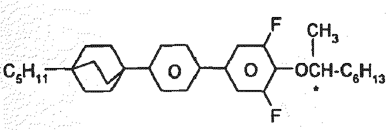 | 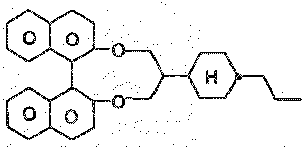 |
| **R/S-4011** | **R/S-5011** |
| 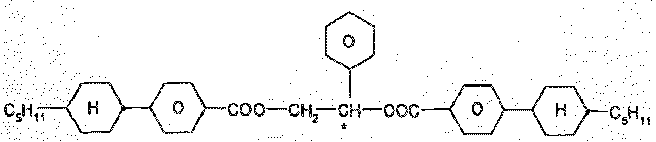 | |
| **R/S-1011** | |

Optional enthalten die FK-Medien 0 bis 10 Gew.%, insbesondere 0,01 bis 5 Gew.%, besonders bevorzugt 0,1 bis 3 Gew.% an Dotierstoffen, vorzugsweise ausgewählt aus der Gruppe bestehend aus Verbindungen der Tabelle C.

**Tabelle D**

| In der Tabelle D werden mögliche Stabilisatoren angegeben, die den erfindungsgemäßen FK-Medien zugesetzt werden können. (n bedeutet hier eine ganze Zahl von 1 bis 12, vorzugsweise 1, 2, 3, 4, 5, 6, 7 oder 8, endständige Methylgruppen sind nicht gezeigt). | |
|---|---|
| 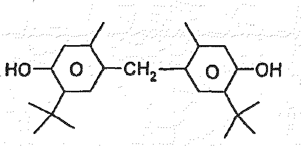 | 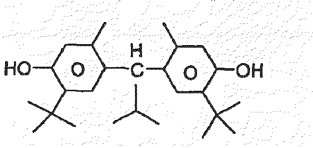 |
| 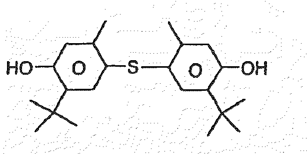 | 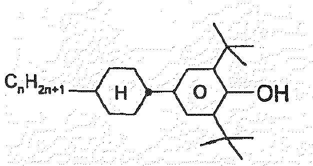 |
| 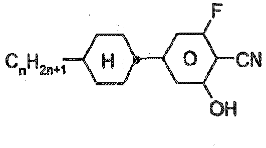 | 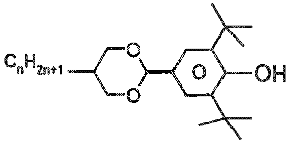 |
| 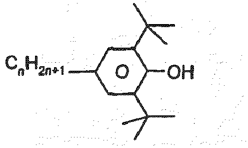 | 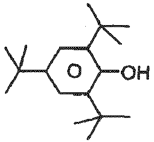 |
| 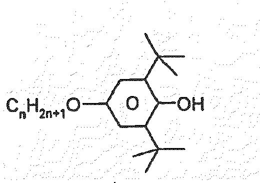 | 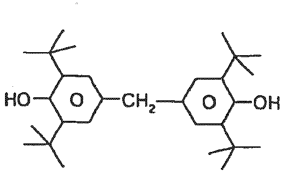 |
| 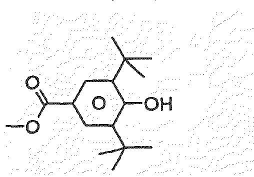 | 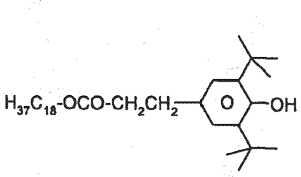 |
| 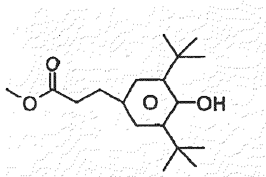 | 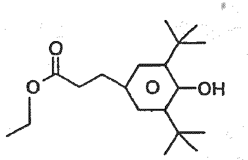 |
| 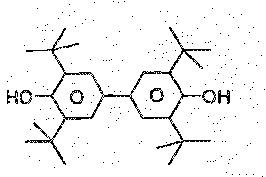 | 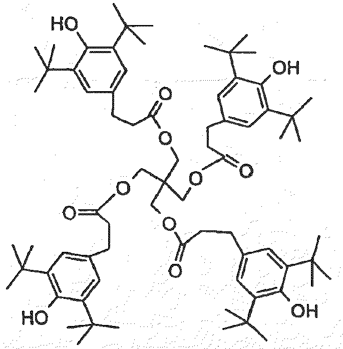 |
| 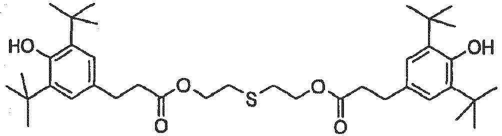 | |
| 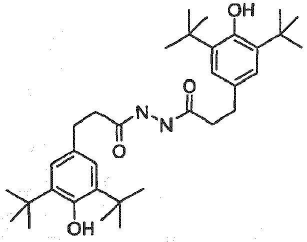 | 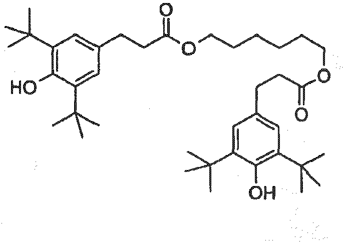 |
| 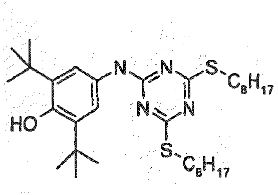 | 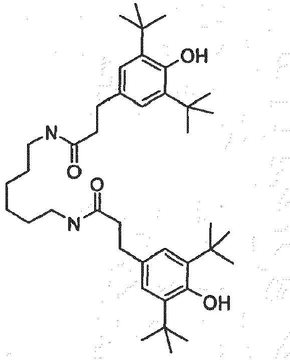 |
| 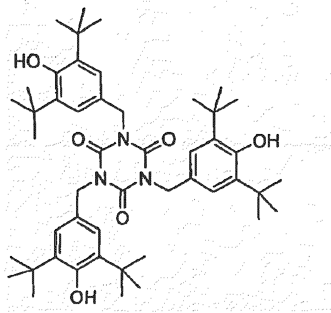 | 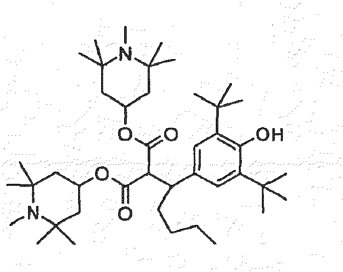 |
| 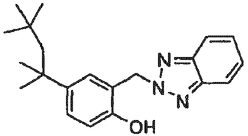 | 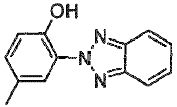 |
| 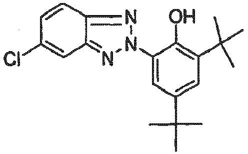 | 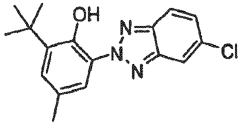 |
| 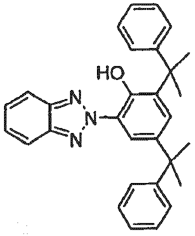 | 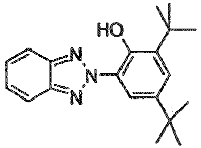 |
| 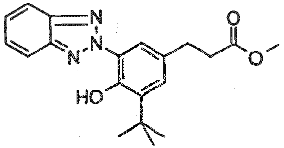 | 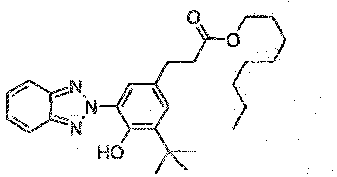 |
| 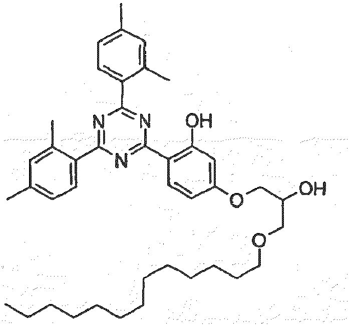 | 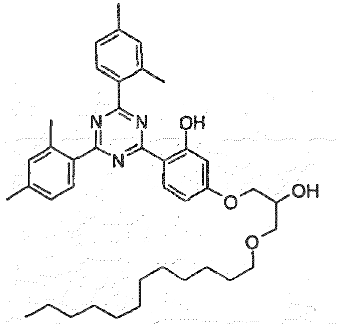 |
| 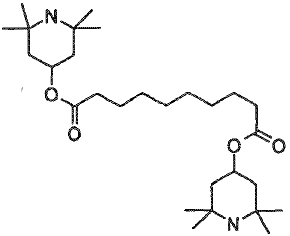 | 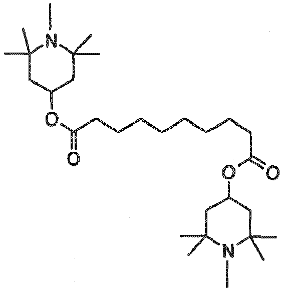 |

Vorzugsweise enthalten die FK-Medien 0 bis 10 Gew.%, insbesondere 1 ppm bis 5 Gew.%, besonders bevorzugt 1ppm bis 1 Gew.% an Stabilisatoren. Vorzugsweise enthalten die FK-Medien einen oder mehrere Stabilisatoren ausgewählt aus der Gruppe bestehend aus Verbindungen der Tabelle D.

**Tabelle E**

| In der Tabelle E sind Beispielverbindungen zusammengestellt, die in den FK-Medien gemäß der vorliegenden Erfindung vorzugsweise als reaktive Verbindungen verwendet werden können. | |
|---|---|
| 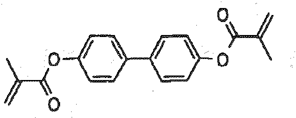 | 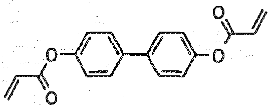 |
| **RM-1** | **RM-2** |
| 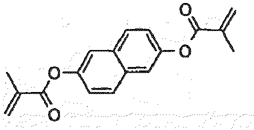 | 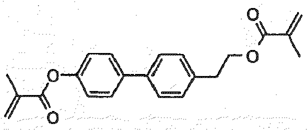 |
| **RM-3** | **RM-4** |
| 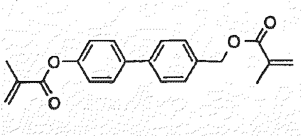 | 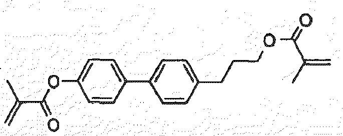 |
| **RM-5** | **RM-6** |
| 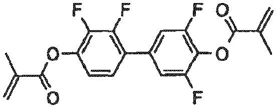 | 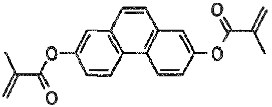 |
| **RM-7** | **RM-8** |
| 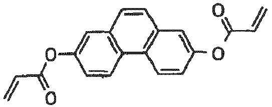 | 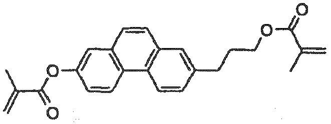 |
| **RM-9** | **RM-10** |
| 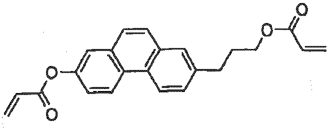 | 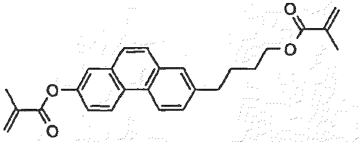 |
| **RM-11** | **RM-12** |
| 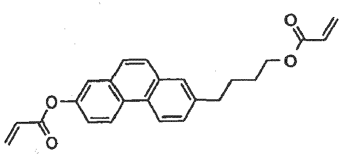 | |
| **RM-13** | |
| 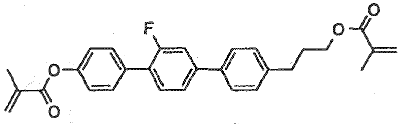 | |
| **RM-14** | |
| 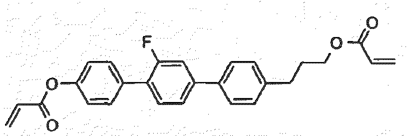 | |
| **RM-15** | |
| 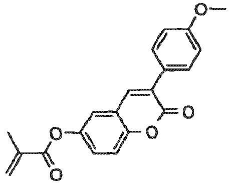 | 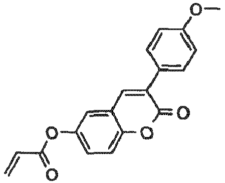 |
| **RM-16** | **RM-17** |
| 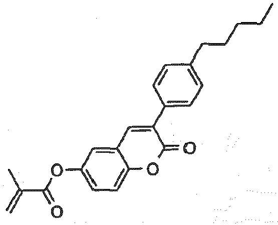 | 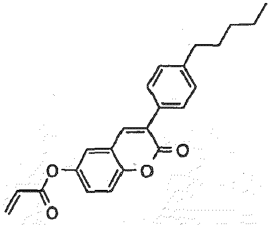 |
| **RM-18** | **RM-19** |
| 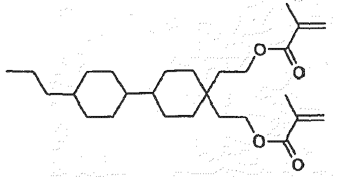 | 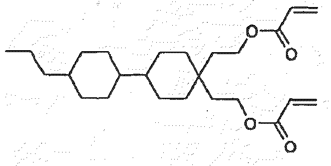 |
| **RM-20** | **RM-21** |
| 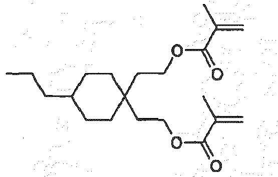 | |
| **RM-22** | |
| 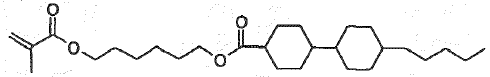 | |
| **RM-23** | |
| 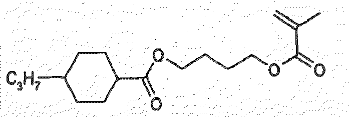 | |
| **RM-24** | |
| 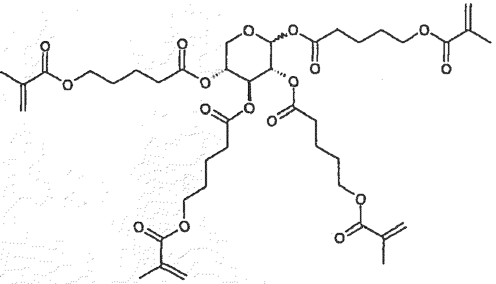 | |
| **RM-25** | |
| 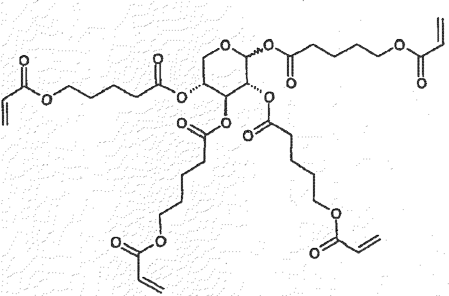 | |
| **RM-26** | |
| 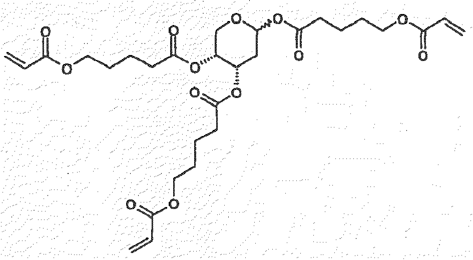 | |
| **RM-27** | |
| 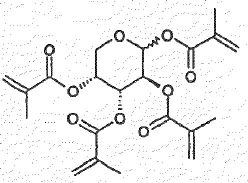 | |
| **RM-28** | |

In einer bevorzugten Ausführungsform der vorliegenden Erfindung enthalten die mesogenen Medien eine oder mehrere Verbindungen ausgewählt aus der Gruppe der Verbindungen der Tabelle E.

In der vorliegenden Anmeldung bedeutet der Begriff "Verbindungen", auch geschrieben als "Verbindung(en)", sofern nicht explizit anders angegeben, sowohl eine als auch mehrere Verbindungen. Umgekehrt schließt der Begriff "Verbindung" generell auch mehrere Verbindungen ein, sofern dies laut Definition möglich und nicht anders angegeben ist. Gleiches gilt für die Begriffe FK-Medien und FK-Medium. Der Begriff "Komponente" umfasst jeweils eine oder mehrere Stoffe, Verbindungen und/oder Teilchen.

Außerdem werden folgende Abkürzungen und Symbole verwendet:
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Permittivität senkrecht zum Direktor bei 20°C und 1 kHz,
- ε_{||}: dielektrische Permittivität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN].

Soweit nicht explizit anders vermerkt, sind in der vorliegenden Anmeldung alle Konzentrationen in Gewichtsprozent angegeben und beziehen sich auf die entsprechende Gesamtmischung, enthaltend alle festen oder flüssigkristallinen Komponenten, ohne Lösungsmittel.

Alle physikalischen Eigenschaften werden und wurden nach "Merck Liquid Crystals, Physical Properties of Liquid Crystals", Status Nov. 1997, Merck

KGaA, Deutschland bestimmt und gelten für eine Temperatur von 20°C und Δn wird bei 589 nm und Δε bei 1 kHz bestimmt, sofern nicht jeweils explizit anders angegeben.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei optional gleichzeitig eine Spannung an die Anzeige angelegt wird (üblicherweise 10 bis 30 V Wechselstrom, 1 kHz). In den Beispielen wird, falls nicht anders angegeben, eine Quecksilberdampflampe mit 100 mW/cm² verwendet, die Intensität wird mit einem Standard-UV-Meter (Fabrikat Ushio UNI meter) gemessen, der mit einem Bandpassfilter bei 320 nm ausgerüstet ist.

Die folgenden Beispiele erläutern die vorliegende Erfindung, ohne sie in irgendeiner Weise beschränken zu sollen. Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

Weitere Kombinationen der Ausführungsformen und Varianten der Erfindung gemäß der Beschreibung ergeben sich auch aus den Ansprüchen.

### Beispiele

Die eingesetzten Verbindungen, soweit nicht kommerziell erhältlich, werden nach Standard-Laborvorschriften synthetisiert. Die FK-Medien stammen von der Merck KGaA, Deutschland.

### Synthesebeispiele

### Beispiel 1: N-[2-(2'-Fluor-4-propyl-[1,1';4',1"]terphenyl-4"-yloxy)-ethyl]-ethan-1,2-diamin

### 1.1 4-Brom-3-fluor-4'-methoxy-biphenyl

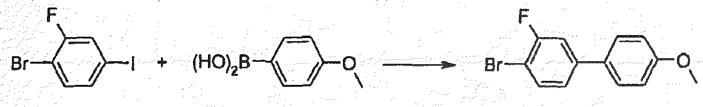

49,0 g (0,163 mol) 1-Brom-2-fluor-4-iodbenzol und 24,7 g (0,163 mol) 4-Methoxybenzolboronsäure werden in einer Mischung aus 325 ml Toluol, 165 ml Wasser und 165 ml Ethanol gelöst und nach Zugabe von 1,9 g (1,64 mmol) Tetrakis(triphenylphosphin)palladium und 34,9 g (0,33 mol) Natriumcarbonat über Nacht unter Rückfluss erhitzt. Die org. Phase wird abgetrennt, über Natriumsulfat getrocknet und eingeengt. Der Rückstand wird mit Heptan/Toluol (1:1) über Kieselgel filtriert und das Rohprodukt aus Ethanol umkristallisiert. Man erhält 4-Brom-3-fluor-4'-methoxy-biphenyl als farblosen Feststoff.

### 1.2 2'-Fluor-4"-methoxy-4-propyl-[1,1';4',1"]terphenyl

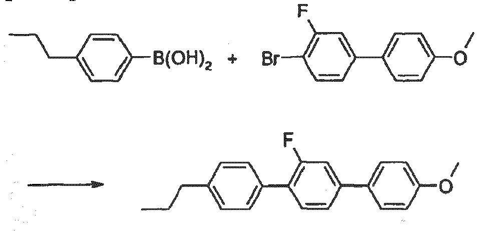

28,0 g (99,0 mmol) 4-Brom-3-fluor-4'-methoxy-biphenyl, 28,8 g (99,2 mmol) Natriummetaborat-Octahydrat und 1,4 g (1,96 mmol) Bis(triphenylphosphin)palladium(II)chlorid werden in 75 ml Wasser und 100 ml THF vorgelegt, mit 0,1 ml Hydrazinhydrat versetzt und nach Zugabe einer Lösung von 16,3 g (99,0 mmol) 4-Propylbenzolboronsäure über Nacht unter Rückfluß erhitzt. Die org. Phase wird abgetrennt, i. Vak. eingeengt, mit Heptan/Toluol über Kieselgel filtriert und das Rohprodukt aus Ethanol/Toluol (14:1) umkristallisiert. Man erhält 2'-Fluor-4"-methoxy-4-propyl-[1,1';4',1"]terphenyl als farblose Kristalle.

### 1.3 2'-Fluor-4-propyl-[1,1';4',1"]terphenyl-4"-ol

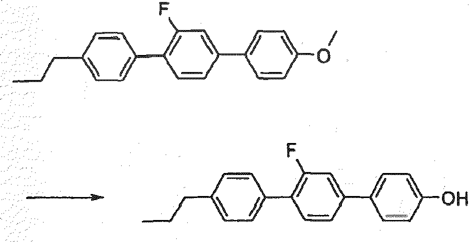

27,2 g (85 mmol) werden in 400 ml Dlchlormethan bei -10°C vorgelegt und tropfenweise mit 100 ml (100 mmol) einer 1 M Lösung von Bortribromid in Hexan versetzt. Die Kühlung wird entfernt und der Ansatz 3 h bei Raumtemp. rühren gelassen. Anschließend wird der Ansatz unter Kühlung mit 200 ml Wasser hydrolysiert, das ausgefallene Produkt durch Zugabe von 800 ml warmem Dichlormethan gelöst und die wässrige Phase abgetrennt. Die org. Phase wird mit Wasser und ges. Natriumhydrogencarbonatlsg. gewaschen und über Natriumsulfat getrocknet. Man erhält 2'-Fluor-4-propyl-[1,1';4',1"]terphenyl-4"-ol als farblosen Feststoff, der ohne weitere Aufreinigung in der nächsten Stufe eingesetzt wird.

### 1.4 4"-(2-Benzyloxy-ethoxy)-2'-fluor-4-propyl-[1,1';4',1"]terphenyl

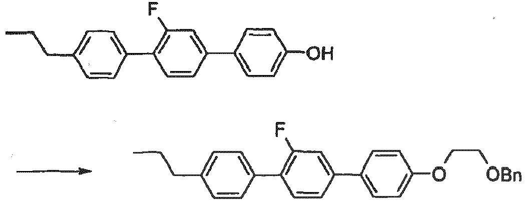

Das Rohprodukt aus Stufe 1.4 wird in 300 ml Ethylmethylketon gelöst, 23,9 g (173 mmol) Kaliumcarbonat sowie 20,0 g (90 mmol) 2-Benzyloxyethylbromid hinzugefügt und über Nacht unter Rückfluss erhitzt. Der Ansatz wird filtriert, das Filtrat eingeengt und der Rückstand mit Toluol über Kieselgel filtriert. Kristallisation des Rohproduktes aus Ethanol/Toluol (6:1) liefert 4"-(2-Benzyloxy-ethoxy)-2'-fluor-4-propyl-[1,1';4',1"]terphenyl als farblosen Feststoff.

### 1.5 2-(2'-Fluor-4-propyl-[1,1';4',1"]terphenyl-4"-yloxy)-ethanol

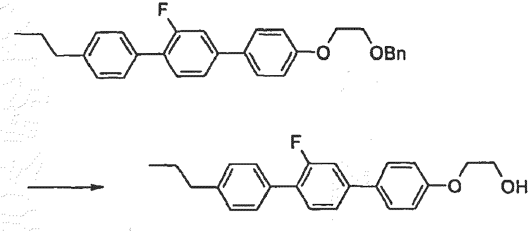

24,5 g (56 mmol) 4"-(2-Benzyloxy-ethoxy)-2'-fluor-4-propyl-[1,1';4',1"]terphenyl werden in THF an Palladiumkohle bis zum Stillstand hydriert. Der Katalysator wird abfiltriert, das Filtrat eingeengt und das Rohprodukt aus Ethanol/Toluol (8:1) umkristallisiert. Man erhält 2-(2'-Fluor-4-propyl-[1,1';4',1"]terphenyl-4"-yloxy)-ethanol als farblose Nadeln.

### 1.6 Toluol-4-sulfonsäure-2-(2'-fluor-4-propyl-[1,1';4',1"]terpheny1-4"-yloxy)-ethylester

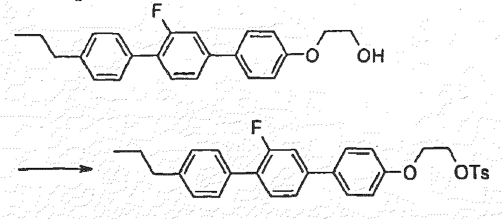

7,0 g (20 mmol) 2-(2'-Fluor-4-propyl-[1,1';4',1"]terphenyl-4"-yloxy)-ethanol werden in 200 ml Dichlormethan gelöst, 3,3 ml (40 mmol) Pyridin und 120 mg DMAP hinzugefügt und auf 10 °C gekühlt. Nach Zugabe von 4,6 g (24 mmol) Tosylchlorid wir der Ansatz über Nacht bei Raumtemperatur gerührt und anschließend mit 500 ml Wasser versetzt. Die wässrige Phase wird abgetrennt und mit Dichlormethan extrahiert. Die vereinigten org. Phasen werden mit verd. Salzsäure und mit ges. Natriumhydrogencarbonatlösung gewaschen und über Natriumsulfat getrocknet. Das Lösungsmittel wird i. Vak. entfernt und der Rückstand durch Chromatographie mit Toluol an Kieselgel gereinigt. Man erhält Toluol-4-sulfonsäure-2-(2'-fluor-4-propyl-[1,1';4',1"]terphenyl-4"-yloxy)-ethylester als farblosen Feststoff.

### 1.7 N-[2-(2'-Fluor-4-propyl-[1,1';4',1"]terphenyl-4"-yloxy)-ethyl]-ethan-1,2-diamin

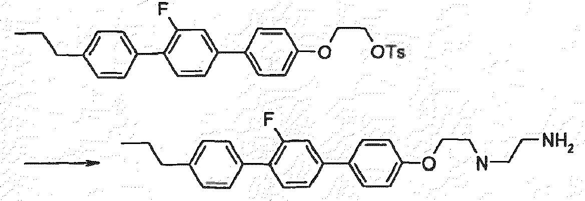

1,00 g (1,98 mmol) Toluol-4-sulfonsäure-2-(2'-fluor-4-propyl-[1,1';4',1"]terphenyl-4"-yloxy)-ethylester werden in 20 ml Ethylendiamin über Nacht auf 60 °C erwärmt. Anschließend wird das Amin i. Vak entfernt und der Rückstand mit Dichlormethan/Methanol/25proz. Ammoniak (80:20:2) über Kieselgel filtriert. Man erhält N-[2-(2'-Fluor-4-propyl-[1,1';4',1"]terphenyl-4"-yloxy)-ethyl]-ethan-1,2-diamin als farblosen Feststoff.
¹H-NMR (300 MHz, CDCl₃) δ = 0.98 ppm (t, J = 7,4 Hz, 3 H, CH₃), 1,56 (s, br., 2 H, NH₂), 1,69 (sext., J = 7,4 Hz, 2 H, CH₂C*H*₂CH₃), 2,64 (t, J = 7,4 Hz, 2 H, -C*H*₂Et), 2,76 (t, J = 5,8 Hz, 2 H, -C*H*₂NH₂), 2,85 (t, J = 5,8 Hz, 2 H, C*H*₂-CH₂NH₂), 3,05 (t, J = 5,2 Hz, 2 H, OCH₂C*H*₂-), 4,13 (t, J = 5,8 Hz, 2 H, -OCH₂-), 6,99 (AB-d, J = 8,7 Hz, 2 H, Ar-H), 7,26 (d, J = 8,5 Hz, 2 H, Ar-H), 7,33 (dd, J = 1,7 Hz, J = 12,1 Hz, 1 H, Ar-H), 7,38 (dd, J = 1,7 Hz, J = 8,0 Hz, 1 H, Ar-H), 7,50 (m_{c}, 2 H, Ar-H), 7,54 (AB-d, J = 8,7 Hz, 2 H, Ar-H).

### Beispiel 2: N-[2-(2'-Fluor-4-propyl-[1,1';4',1"]terphenyl-4"-yloxy)-ethyl]-propan-1,3-diamin

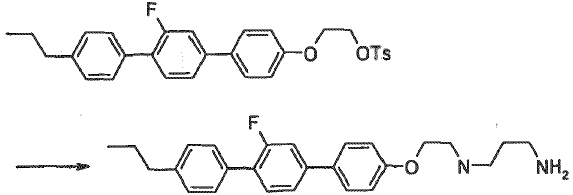

In Analogie zu Beispiel 1 erhält man aus Toluol-4-sulfonsäure-2-(2'-fluor-4-propyl-[1,1';4',1"]terphenyl-4"-yloxy)-ethylester und 1,3-Diaminopropan das N-[2-(2'-Fluor-4-propyl-[1,1';4',1"]terphenyl-4"-yloxy)-ethyl]-propan-1,3-diamin.
¹H-NMR (300 MHz, CDCl₃) δ = 0.98 ppm (t, J = 7,3 Hz, 3 H, CH₃), 1,55 - 1,90 (m, 6 H, darin: 1,67 (quint., J = 7,1 Hz, -NH-CH₂-C*H*₂-CH₂NH₂); s, br., NH2; CH₃C*H*₂CH₂-), 2,63 (dd, J = 7,3 Hz, J = 8,0 Hz, 2 H, Ar-C*H*₂Et), 2,75 (t, J = 7,1 Hz, 2 H, - C*H*₂NH₂), 2,78 (t, J = 6,9 Hz, 2 H, -NH-C*H*₂-(CH₂)₂NH₂), 3,01 (t, J = 5,3 Hz, 2 H, -OCH₂C*H*₂-NH), 3,41 (s, 1 H, -NH-), 4,11 (t, J = 5,3 Hz, 2 H, -O-CH₂-), 6,98 (AB-d, J = 8,8 Hz, 2 H, Ar-H), 7,26 (d, J = 8,2 Hz, 2 H, Ar-H), 7,32 (dd, J = 1,8 Hz, J = 12,2 Hz, 1 H, Ar-H), 7,37 (dd, J = 1,8 Hz, J = 8,0 Hz, 1 H, Ar-H), 7,43 - 7,56 (m, 5 H, Ar-H).

### Beispiel 3:[2-(2'-Fluor-4-propyl-[1,1';4',1"]terphenyl-4"-yloxy)-ethyl]-(2-methoxy-ethyl)-amin

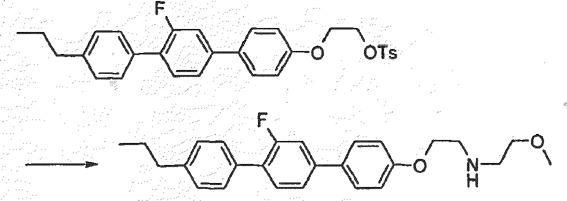

In Analogie zu Beispiel 1 erhält man aus Toluol-4-sulfonsäure-2-(2'-fluor-4-propyl-[1,1';4',1"]terphenyl-4"-yloxy)-ethylester und 2-Methoxy-1-ethylamin das [2-(2'-Fluor-4-propyl-[1,1';4',1"]terphenyl-4"-yloxy)-ethyl]-(2-methoxy-ethyl)-amin als farblosen Feststoff.
¹H-NMR (400 MHz, CDCl₃) δ = 0.98 ppm (t, J = 7,3 Hz, 3 H, CH₃), 1,69 (sext., J = 7,5 Hz, 2 H, CH₃C*H*₂CH₂-), 1,74 (s, br., NH), 2,64 (dd, J = 6,7 Hz, J = 8,6 Hz, 2 H, -C*H*₂-Et), 2,89 (t, J = 5,3 Hz, 2 H, - CH₂-NH-), 3,06 (t, 5,3 Hz, 2 H, -CH₂-NH-), 3,38 (s, 3 H, OCH₃), 3,54 (dd, J = 5,3, J = 5,3 Hz, 2 H, -O-CH₂-), 4,13 (t, 5,3 Hz, 2 H, -O-CH₂-), 7,00 (AB-d, J = 8,7 Hz, 2 H, Ar-H), 7,26 (d, J = 8,3 Hz, 2 H, Ar-H), 7,32 (dd, J = 1,7 Hz, J = 12,2 Hz, 1 H, Ar-H), 7,39 (dd, J = 1,8 Hz, J = 8,0 Hz, 1 H, Ar-H), 7,44 - 7,57 (m, 5 H, Ar-H).

### Beispiel 4: 2'-Fluor-4"-[2-(2-methoxy-ethoxy)-ethoxy]-4-propyl-[1,1';4',1"]terphenyl

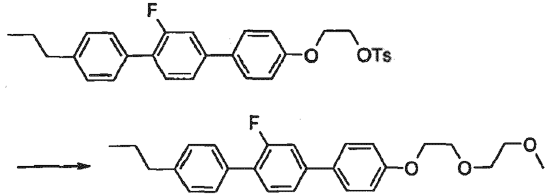

735 mg (18,4 mmol) einer 60proz. Dispersion von Natriumhydrid in Mineralöl werden unter Stickstoff mit Pentan gewaschen und i. Vak. getrocknet. Anschließend werden 30 ml THF und 1,21 g (15,9 mmol) Ethylenglykolmonomethylether hinzugegeben und bis zum Ende der Gasentwicklung bei Raumtemp. rühren gelassen. Nach Zugabe einer Lösung von 5,00 g (12,2 mmol) Toluol-4-sulfonsäure-2-(2'-fluor-4-propyl-[1,1';4',1"]terphenyl-4"-yloxy)-ethylester in 10 ml THF wird der Ansatz 3 d bei Raumtemperatur rühren gelassen und dann überschüssiges Natriumhydrid durch Zugabe von Methanol zersetzt. Die Lösung wird auf Wasser gegeben, mit 2 M Salzäure angesäuert und dreimal mit MTB-Ether extrahiert. Die vereinigten org. Phasen werden über Natriumsulfat getrocknet und i. Vak. Eingeengt. Das Rohprodukt wird mit Dichlormethan über Kieselgel filtriert und aus Toluol umkristallisiert. Man erhält 2'-Fluor-4"-[2-(2-methoxy-ethoxy)-ethoxy]-4-propyl-[1,1';4',1"]terphenyl als farblose Kristalle.

### Beispiel 5: N-{2-[(3S,5S,8R,9S,10S,13R,14S,17R)-17-((R)-1,5-Dimethyl-hexyl)-10,13-dimethyl-hexadecahydro-cyclopenta[a]phenanthren-3-yloxy]-ethyl}-ethan-1,2-diamin

### 5.1 tert-Butyl-{2-[(3S,5S,8R,9S,10S,13R,14S,17R)-17-((R)-1,5-dimethyl-hexyl)-10,13-dimethyl-hexadecahydro-cyclopenta[a]phenanthren-3-yloxy]-ethoxy}-dimethylsilan

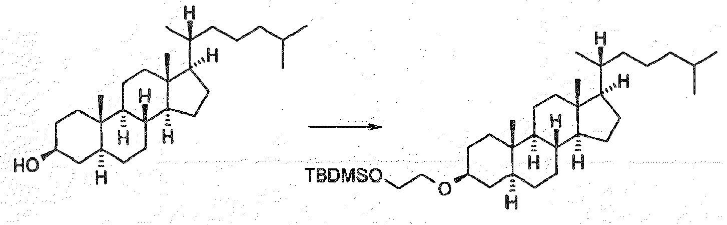

1,15 g (28,8 mmol) einer 60proz. Suspension von Natriumhydrid in Paraffinöl werden in 70 ml THF vorgelegt und eine Lösung von 9,0 g (23,2 mmol) (3S,5S,8R,9S,10S,13R,14S,17R)-17-((R)-1,5-Dimethyl-hexyl)-10,13-dimethyl-hexadecahydro-cyclopenta[a]phenanthren-3-ol in 60 ml THF zutropfen gelassen. Der Ansatz wird 2 auf 70 °C erhitzt, mit einer Lösung von 10 ml (49 mmol) (2-Bromethoxy)-tert.-butyl-dimethylsilan in 50 ml THF versetzt und über Nacht bei 70 °C gerührt. Anschließend wird mit 300 ml Wasser hydrolysiert und dreimal mit MTB-Ether extrahiert. Die vereinigten org. Phasen werden mit Wasser gewaschen und über Natriumsulfat getrocknet. Das Lösungsmittel wird i. Vak. entfernt und der Rückstand mit Toluol an Kieselgel chromatographiert. Man erhält tert-Butyl-{2-[(3S,5S,8R,9S,10S,13R,14S,17R)-17-((R)-1,5-dimethyl-hexyl)-10,13-dimethyl-hexadecahydro-cyclopenta[a]phenanthren-3-yloxy]-ethoxy}-dimethylsilan als farblose Kristalle.

### 5.2 2-[(3S,5S,8R,9S,10S,13R,14S,17R)-17-((R)-1,5-Dimethyl-hexyl)-10,13-dimethyl-hexadecahydro-cyclopenta[a]phenanthren-3-yloxy]-ethanol

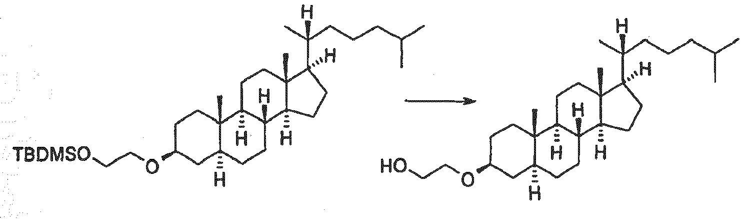

1,90 g (3,47 mmol) tert-Butyl-{2-[(3S,5S,8R,9S,10S,13R,14S,17R)-17-((R)-1,5-dimethyl-hexyl)-10,13-dimethyl-hexadecahydro-cyclopenta[a]-phenanthren-3-yloxy]-ethoxy}-dimethylsilan werden in 20 ml THF gelöst und unter Eiskühlung tropfenweise mit 3,8 ml (38 mmol) einer 1 M Lösung von Tetrabutylammoniumfluorid in THF versetzt. Die Kühlung wird entfernt, der Ansatz 15 min bei Raumtemp. rühren gelassen und auf Wasser gegeben. Die wässrige Phase wird abgetrennt und mit MTB-Ether extrahiert. Die vereinigten org. Phasen werden mit ges. Natriumchloridlsg. gewaschen, über Natriumsulfat getrocknet und das Lösungsmittel wird i. Vak. entfernt. Man erhält 2-[(3S,5S,8R,9S,10S,13R,14S,17R)-17-((R)-1,5-Dimethyl-hexyl)-10,13-dimethyl-hexadecahydro-cyclopenta[a]phenanthren-3-yloxy]-ethanol als zähes gelbes Öl, das ohne weitere Aufreinigung in der nächsten Stufe eingesetzt wird.

### 5.3 Toluol-4-sulfonsäure-2-[(3S,5S,8R,9S,10S,13R,14S,17R)-17-((R)-1,5-dimethyl-hexyl)-10,13-dimethyl-hexadecahydro-cyclopenta[a]-phenanthren-3-yloxy]-ethylester

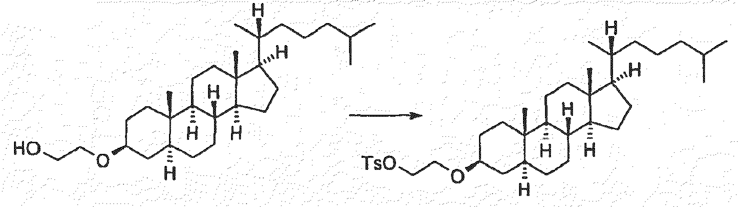

In Analogie zu Beispiel 1 erhält man aus 2-[(3S,5S,8R,9S,10S,13R,14S,17R)-17-((R)-1,5-Dimethyl-hexyl)-10,13-dimethyl-hexadecahydro-cyclopenta[a]phenanthren-3-yloxy]-ethanol den Toluol-4-sulfonsäure-2-[(3S,5S,8R,9S,10S,13R,14S,17R)-17-((R)-1,5-dimethyl-hexyl)-10,13-dimethyl-hexadecahydro-cyclopenta[a]phenanthren-3-yloxy]-ethylester als zähes Öl.

### 5.4 N-{2-[(3S,5S,8R,9S,10S,13R,14S,17R)-17-((R)-1,5-Dimethyl-hexyl)-10,13-dimethyl-hexadecahydro-cyclopenta[a]phenanthren-3-yloxy]-ethyl}-ethan-1,2-diamin

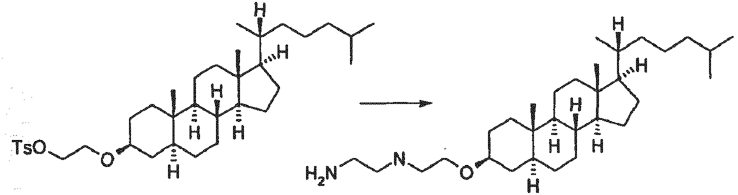

In Analogie zu Beispiel 1 erhielt man aus Toluol-4-sulfonsäure-2-[(3S,5S,8R,9S,10S,13R,14S,17R)-17-((R)-1,5-dimethyl-hexyl)-10,13-dimethyl-hexadecahydro-cyclopenta[a]phenanthren-3-yloxy]-ethylester das N-{2-[(3S,5S,8R,9S,10S,13R,14S,17R)-17-((R)-1,5-Dimethyl-hexyl)-10,13-dimethyl-hexadecahydro-cyclopenta[a]phenanthren-3-yloxy]-ethyl}-ethan-1,2-diamin als farbloses zähes Öl.
¹H-NMR (500 MHz, CDCl₃) δ = 0,65 (s, 3 H, -CH₃), 0,75 - 0,89 (m, 46 H, darin: 0,79 (s, 3 H, -CH₃),0,86 (d, J = 2,1 Hz, 3 H, -CH₃), 0,87 (d, J = 2,1 Hz, 3 H, -CH₃), 0,90 (d, J = 6,5 Hz, 3 H, -CH₃), Alkyl-H, -NH-, -NH₂), 1,96 (ddd, J = 12,6 Hz, J = 3,2 Hz, J = 3,2 Hz, 1 H), 2,69 (t, J = 6,0 Hz, 2 H, -C*H*₂NH₂), 2,77 (dd, J = 5,4 Hz, J = 5,4 Hz, 2 H, -OCH₂C*H*₂NH-), 2,81 (t, J = 6,0 Hz, 2 H, -NHC*H*₂CH₂NH₂), 3,22 (dddd, J = 4.7 Hz, J = 4.7 Hz, J = 4.7 Hz, J = 4.7 Hz, 1 H, >CH-O-), 3.58 (m_{c}, 2 H, -CH₂O-).

Analog oder nach Literaturvorschrift werden folgende Verbindungen zur Verwendung in FK-Medien hergestellt (teilweise auch kommerziell verfügbar):

| Beispiel Nr. | Strukturformel | |
|---|---|---|
| 6. | 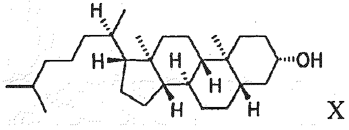 | |
| 7. | 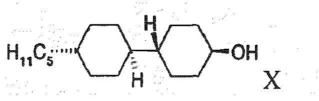 | |
| 8. | 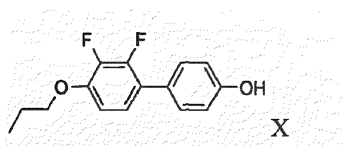 | |
| 9. | 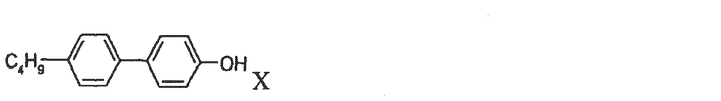 | |
| 10. | 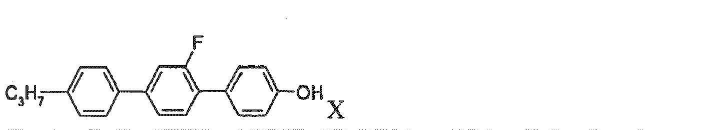 | |
| 11. | 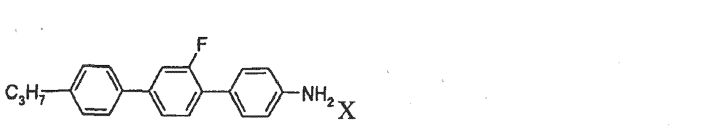 | |
| 12. | 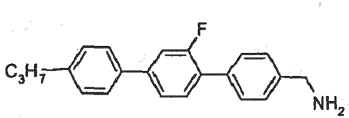 | |
| 13. | 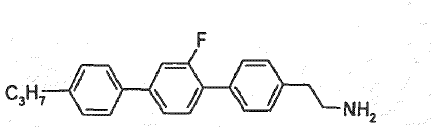 | |
| 14. | 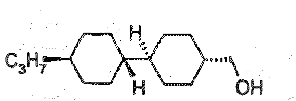 | |
| 15. | 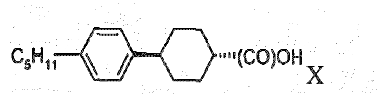 | |
| 16. | 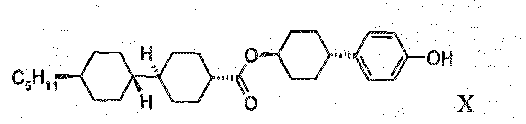 | |
| 17. | 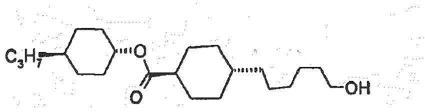 | |
| 18. | 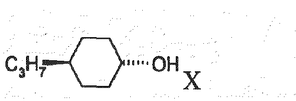 | |
| 19. | 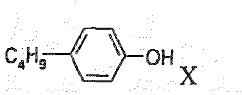 | |
| 20. | 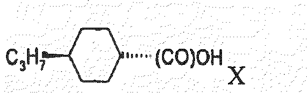 | |
| 21. | 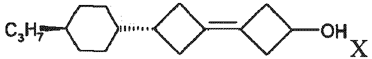 | |
| 22. | 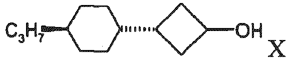 | |
| X = nicht erfindungsgemäß | | |

### Mischungsbeispiele

Zur Herstellung von erfindungsgemäßen FK-Medien werden die folgenden flüssigkristallinen Mischungen bestehend aus niedermolekularen Komponenten in den angegebenen prozentualen Gewichtsanteilen verwendet.

**Tabelle 1: Nematisches FK-Medium M1 (Δε < 0)**

| | | | |
|---|---|---|---|
| CY-3-O4 | 14 % | Kp. | + 80 °C |
| CCY-3-O2 | 9 % | Δn | 0,090 |
| CCY-3-O3 | 9 % | Δε | -3,3 |
| CPY-2-O2 | 10 % | ε_{∥} | 3,4 |
| CPY-3-O2 | 10 % | K₃/K₁ | 0,97 |
| CCY-3-1 | 8 % | | |
| CCH-34 | 9 % | | |
| CCH-35 | 6 % | | |
| PCH-53 | 10 % | | |
| CCH-301 | 6 % | | |
| CCH-303 | 9 % | | |

**Tabelle 2: Nematisches FK-Medium M2 (Δε > 0) (Vergleich)**

| | | | |
|---|---|---|---|
| CC-4-V | 10 % | Kp. | + 77°C |
| CC-5-V | 13.5 % | Δn | 0.113 |
| PGU-3-F | 6.5 % | Δε | 19.2 |
| ACQU-2-F | 10 % | ε_{∥} | 23.8 |
| ACQU-3-F | 12 % | K₃/K₁ | 0.97 |
| PUQU-3-F | 11 % | | |
| CCP-V-1 | 12 % | | |
| APUQU-2-F | 6 % | | |
| APUQU-3-F | 7 % | | |
| PGUQU-3-F | 8% | | |
| CPGU-3-OT | 4% | | |

### Mischungsbeispiel 1

Zu einem nematischen FK-Medium **M1** des VA-Typs (Δε < 0) gemäß Tabelle 1 wird die Verbindung aus **Synthesebeispiel** 5 (0,2 Gew.%) zugesetzt und homogenisiert.

Verwendung in Testzellen ohne Vororientierungsschicht ('alignment layer'): Die entstandene Mischung wird in eine Testzelle gefüllt (ohne Polyimid-Orientierungsschicht, Schichtdicke d ≈ 4,0 µm, beidseitige ITO-Beschichtung, ohne Passivierungsschicht). Das FK-Medium weist eine spontane homöotrope (vertikale) Orientierung zu den Substratoberflächen auf. Diese Orientierung bleibt bis 70 °C stabil. Im temperaturstabilen Bereich lässt sich die VA-Zelle zwischen gekreuzten Polarisatoren durch Anlegen einer Spannung zwischen 0 und 30 V reversibel schalten.

### Mischungsbeispiel 2 (zum Vergleich)

Zu einem nematischen FK-Medium **M2** des VA-IPS-Typs (Δε > 0) gemäß Tabelle 2 wird die Verbindung aus **Synthesebeispiel 5** (0,5 Gew.%) zugesetzt und homogenisiert.

Verwendung in Testzellen ohne Vororientierungsschicht ('alignment layer'): Die entstandene Mischung wird in eine Testzelle gefüllt (ohne Polyimid-Orientierungsschicht, Schichtdicke d ≈ 10 µm, auf einer Substratoberfläche angeordnete ITO-Interdigitalelektroden, Glas auf der gegenüberliegenden Substratoberfläche, ohne Passivierungsschicht). Das FK-Medium weist eine spontane homöotrope (vertikale) Orientierung zu den Substratoberflächen auf. Diese Orientierung bleibt bis 70 °C stabil. Im temperaturstabilen Bereich lässt sich die VA-IPS-Zelle zwischen gekreuzten Polarisatoren durch Anlegen einer Spannung zwischen 0 und 20 V reversibel schalten.

### Mischungsbeispiele 3-17

Die Verbindungen der **Synthesebeispiele 1, 2, 3** und der **Beispiele 6-17** werden analog Mischungsbeispiel 1 zu einem nematischen FK-Medium **M1** (Δε < 0) gemäß Tabelle 1 zugesetzt und homogenisiert. Die Gewichtsanteile der Verbindungen im Medium sind in der Tabelle 3 angegeben. Das erhaltene FK-Medium wird jeweils in eine Testzelle ohne Vororientierungsschicht gefüllt und weist eine spontane homöotrope (vertikale) Orientierung zu den Substratoberflächen auf. Im temperaturstabilen Bereich lässt sich die VA-Zelle zwischen gekreuzten Polarisatoren durch Anlegen einer Spannung zwischen 0 und 30 V reversibel schalten.

**Tabelle 3: Gewichtsanteile für Dotierung in M1 und Orientierung der erhaltenen FK-Mischung bei 25°C und 70°C. Testzelle des VA-Typs**

| Mischungsbeispiel | Verbindung Beispiel Nr. | Gewichtsanteil | Orientierung bei 25 °C / schaltbar | Orientierung bei 70 °C / schaltbar |
|---|---|---|---|---|
| 3 | 1 | 0.25 % | Homöotrop / Ja | Homöotrop / Ja |
| 4 | 2 | 0.15 % | Homöotrop / Ja | Homöotrop / Ja |
| 5 | 3 | 5.0 % | Homöotrop / Ja | Homöotrop / Ja |
| 6 | 6X | 1.0 % | Homöotrop / Ja | Homöotrop / Ja |
| 7 | 7X | 1.5 % | Homöotrop / Ja | Homöotrop / Ja |
| 8 | 8X | 2.5 % | Homöotrop / Ja | Homöotrop / Ja |
| 9 | 9X | 2.5 % | Homöotrop / Ja | Homöotrop / Ja |
| 10 | 10X | 2.5 % | Homöotrop / Ja | Homöotrop / Ja |
| 11 | 11X | 2.5 % | Homöotrop / Ja | Homöotrop / Ja |
| 12 | 12 | 0.5 % | Homöotrop / Ja | Homöotrop / Ja |
| 13 | 13 | 0.5 % | Homöotrop / Ja | Homöotrop / Ja |
| 14 | 14 | 2.0 % | Homöotrop / Ja | Homöotrop / Ja |
| 15 | 15X | 0.7 % | Homöotrop / Ja | Homöotrop / Ja |
| 16 | 16X | 1.0 % | Homöotrop / Ja | Homöotrop / Ja |
| 17 | 17 | 2.0 % | Homöotrop / Ja | Homöotrop / Ja |
| 18 | 18X | 2.0 % | Homöotrop / Ja | |
| 19 | 19X | 2.0 % | Homöotrop / Ja | |
| 20 | 20X | 1.0 % | Homöotrop / Ja | |
| 21 | 21X | 0.7 % | Homöotrop / Ja | |
| 22 | 22X | 0.5 % | Homöotrop / Ja | |
| X = nicht erfindungsgemäß | | | | |

### Mischungsbeispiele 22-26 (zum Vergleich)

Die Verbindungen des **Synthesesebeispiels 1** und der **Beispiele 6, 7, 10, 11** werden analog Mischungsbeispiel 2 zu einem nematischen FK-Medium **M2** (Δε > 0) gemäß Tabelle 2 zugesetzt und homogenisiert. Die Gewichtsanteile der Verbindungen im Medium sind in der Tabelle 4 angegeben. Das erhaltene FK-Medium wird jeweils in eine Testzelle ohne Vororientierungsschicht gefüllt und weist eine spontane homöotrope (vertikale) Orientierung zu den Substratoberflächen auf. Im temperaturstabilen Bereich lässt sich die VA-IPS-Zelle zwischen gekreuzten Polarisatoren durch Anlegen einer Spannung zwischen 0 und 20 V reversibel schalten.

**Tabelle 4: Gewichtsanteile für Dotierung in M2 und Orientierung der erhaltenen FK-Mischung bei 25°C und 60°C. Testzelle des VA-IPS-Typs**

| MischungsBeispiel Nr. | Verbindung Beispiel Nr. | Gewichtsanteil | Orientierung bei 25 °C / schaltbar | Orientierung bei 60 °C / schaltbar |
|---|---|---|---|---|
| 22 | 1 | 0.5 % | Homöotrop / Ja | Homöotrop / Ja |
| 23 | 6 | 3.0 % | Homöotrop / Ja | Planar |
| 24 | 7 | 1.0 % | Homöotrop / Ja | Homöotrop / Ja |
| 25 | 10 | 1.7 % | Homöotrop / Ja | Planar |
| 26 | 11 | 2.5 % | Homöotrop / Ja | Planar |
| 27 | 18 | 2.0 % | Homöotrop / Ja | |
| 28 | 19 | 2.0 % | Homöotrop / Ja | |
| 29 | 20 | 1.0 % | Homöotrop / Ja | |
| 30 | 21 | 0.7 % | Homöotrop / Ja | |
| 31 | 22 | 0.5 % | Homöotrop / Ja | |

### Mischungsbeispiel 32 (Polymerstabilisierung von Mischungsbeispiel 6) nicht erfindungsgemäß

Zu einem nematischen FK-Medium **M1** (Δε < 0) gemäß Tabelle 1 werden eine polymerisierbare Verbindung (**RM-1**, 0,5 Gew.%) und eine selbstorientierende Verbindung (**6**, 1,0 Gew.%) zugesetzt und homogenisiert.

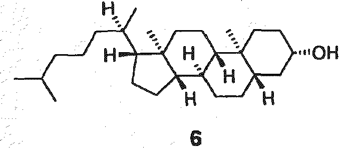

Verwendung in Testzellen ohne Vororientierungsschicht ('alignment layer'): Die entstandene Mischung wird in eine Testzelle gefüllt (ohne Polyimid-Orientierungsschicht, Schichtdicke d ≈ 4,0 µm, beidseitige ITO-Beschichtung, ohne Passivierungsschicht). Das FK-Medium weist eine spontane homöotrope (vertikale) Orientierung zu den Substratoberflächen auf. Unter Anlegen einer Spannung größer als die optische Schwellenspannung wird die Zelle 15 min mit UV-Licht der Intensität 100 mW/cm² bei 40°C bestrahlt. Dadurch erfolgt Polymerisation der monomeren, polymerisierbaren Verbindung. Die homöotrope Orientierung wird damit zusätzlich stabilisiert und ein 'pre-tilt' wird eingestellt. Die erhaltene PSA-VA-Zelle lässt sich bis 70 °C unter Anlegen einer Spannung zwischen 0 und 30 V reversibel schalten. Die Schaltzeiten sind, im Vergleich zu der nicht polymerisierten Zelle, verkürzt.

### Mischungsbeispiel 33 (Polymerstabilisierung von Mischungsbeispiel 7) nicht erfindungsgemäß

Zu einem nematischen FK-Medium **M1** (Δε < 0) gemäß Tabelle 1 werden eine polymerisierbare Verbindung **(RM-1**, 0,5 Gew.%) und eine selbstorientierende Verbindung (7, 1,7 Gew.%) zugesetzt und homogenisiert. 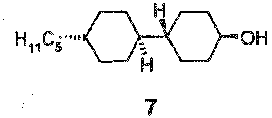 Verwendung in Testzellen ohne Vororientierungsschicht ('alignment layer'): Die entstandene Mischung wird in eine Testzelle gefüllt (ohne Polyimid-Orientierungsschicht, Schichtdicke d ≈ 4,0 µm, beidseitige ITO-Beschichtung, ohne Passivierungsschicht). Das FK-Medium weist eine spontane homöotrope (vertikale) Orientierung zu den Substratoberflächen auf. Unter Anlegen einer Spannung größer als die optische Schwellenspannung wird die Zelle 15 min mit UV-Licht der Intensität 100 mW/cm² bei 40°C bestrahlt. Dadurch erfolgt Polymerisation der monomeren Verbindung. Die homöotrope Orientierung wird damit zusätzlich stabilisiert und ein 'pre-tilt' wird eingestellt. Die erhalten PSA-VA-Zelle lässt sich bis 70 °C durch Anlegen einer Spannung zwischen 0 und 30 V reversibel schalten. Die Schaltzeiten sind, im Vergleich zu der nicht polymerisierten Zelle, verkürzt.

## Patentansprüche

1. FK-Medium mit negativer dielektrischer Anisotropie enthaltend eine niedermolekulare flüssigkristalline Komponente, zusätzlich eine polymerisierbare oder eine polymerisierte Komponente, wobei die polymerisierte Komponente erhältlich ist durch Polymerisation einer polymerisierbaren Komponente und eine oder mehrere organische Verbindungen, wobei die organische Verbindung **dadurch gekennzeichnet ist, dass** sie mindestens eine polare Ankergruppe aufweist und mindestens eine Ringgruppe aufweist, wobei die polare Ankergruppe mindestens eine OH-Struktur oder ein N-Atom in einer primären oder sekundären Amingruppe umfasst,
und wobei die Ankergruppe nicht aus einer OH-Gruppe besteht, die wie die OH-Gruppe in 2,6-Di-tert-butylphenol abgeschirmt ist,
und wobei die organische Verbindung eine Verbindung der Formel I:
R¹-A¹-(Z²-A²)ₘ₁-R² (I)
umfasst, worin
A¹ und A² jeweils unabhängig voneinander eine aromatische, heteroaromatische, alicyclische oder heterocyclische Gruppe, welche auch anellierte Ringe enthalten kann, und welche auch durch eine Gruppe L ein- oder mehrfach substituiert sein kann,
L jeweils unabhängig voneinander -OH, -(CH₂)ₙ₁-OH, F, Cl, Br, I, -CN, -NO₂ , -NCO, -NCS, -OCN, -SCN, -C(=O)N(R⁰)₂, -C(=O)R⁰, -N(R⁰)₂, -(CH₂)ₙ₁-N(R⁰)₂, optional substituiertes Silyl, optional substituiertes Aryl oder Cycloalkyl mit 6 bis 20 C-Atomen, oder geradkettiges oder verzweigtes Alkyl, Alkoxy, Alkylcarbonyl, Alkoxycarbonyl, Alkylcarbonyloxy oder Alkoxycarbonyloxy mit 1 bis 25 C-Atomen, worin auch ein oder mehrere H-Atome durch F oder Cl ersetzt sein können,
Z² jeweils unabhängig voneinander -O-, -S-, -CO-, -CO-O-, -OCO-, -O-CO-O-, -OCH₂-, -CH₂O-, -SCH₂-, -CH₂S-, -CF₂O-, -OCF₂-, -CF₂S-, -SCF₂-, -(CH₂)ₙ₁-, -CF₂CH₂-, -CH₂CF₂-, -(CF₂)ₙ₁-, -CH=CH-, -CF=CF-, -C≡C-, -CH=CH-COO-, -OCO-CH=CH-, CR⁰R⁰⁰ oder eine Einfachbindung,
R⁰ und R⁰⁰ jeweils unabhängig voneinander H oder Alkyl mit 1 bis 12 C-Atomen,
R¹H, Halogen, geradkettiges, verzweigtes oder cyclisches Alkyl mit 1 bis 25 C-Atomen, worin auch eine oder mehrere nicht benachbarte CH₂-Gruppen durch, -NR⁰-, -O-, -S-, -CO-, -CO-O-, -O-CO-, -O-CO-O- so ersetzt sein können, dass N-, O- und/oder S-Atome nicht direkt miteinander verknüpft sind, und worin auch ein oder mehrere tertiäre Kohlenstoffatome (CH-Gruppen) durch N ersetzt sein können, und worin auch ein oder mehrere H-Atome durch F oder Cl, ersetzt sein können,
m1 0, 1, 2, 3, 4 oder 5, und
n1 1, 2, 3 oder 4,
bedeutet,
und wobei die Gruppe R² eine Gruppe der Teilformel (A1)
-Sp-[X²-Z³-]ₖX¹ (A1)
umfasst, worin
Sp eine Abstandsgruppe,
X¹ eine Gruppe -NH₂, -NHR¹¹ -NR¹¹₂, -OH, -(CO)OH oder eine Gruppe der Formeln 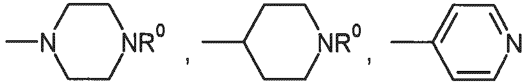 oder 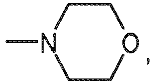
R⁰ H oder Alkyl mit 1 bis 12 C-Atomen,
X² jeweils unabhängig -NH-, -NR¹¹-, -O- oder eine Einfachbindung,
Z³ jeweils unabhängig jeweils unabhängig voneinander eine Alkylengruppe mit 1-15 C-Atomen, carbocyclische Ringe mit 5 oder 6 C-Atomen, oder Kombinationen aus einem oder mehreren Ringen und Alkylengruppen, worin jeweils Wasserstoff durch -OH, -OR¹¹, -(CO)OH, -NH₂, -NHR¹¹, -NR¹¹₂, oder Halogen (bevorzugt F, Cl) ersetzt sein kann,
R¹¹ jeweils unabhängig einen halogenierten oder unsubstituierten Alkylrest mit 1 bis 15 C-Atomen, wobei in diesem Rest auch eine oder mehrere CH₂-Gruppen jeweils unabhängig voneinander durch -C≡C-, -CH=CH-, -(CO)O-, -O(CO)-, -(CO)-oder -O- so ersetzt sein können, dass O-Atome nicht direkt miteinander verknüpft sind, und wobei zwei Reste R¹¹ miteinander zu einem Ring verknüpft sein können, und
k 0, 1, 2 oder 3
bedeuten.

2. FK-Medium nach Anspruch 1, **dadurch gekennzeichnet, dass** die organische Verbindung eine relative Molmasse von mehr als 100 g/mol hat.

3. FK-Medium nach Anspruch 1 oder 2, **dadurch gekennzeichnet, dass** es zusätzlich eine oder mehrere Verbindungen ausgewählt aus der Gruppe der Verbindungen der Formeln A, B und C enthält, 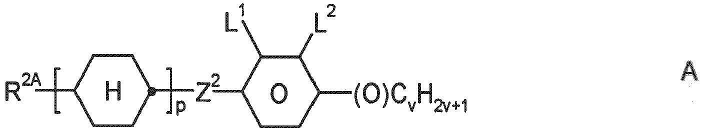 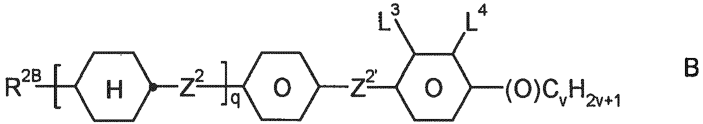 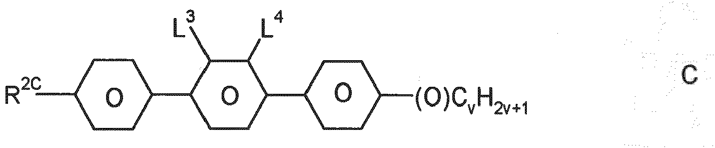 worin
R^{2A}, R^{2B} und R^{2C} jeweils unabhängig voneinander H, einen unsubstituierten, einen einfach durch CN oder CF₃ oder mindestens einfach durch Halogen substituierten Alkylrest mit bis zu 15 C-Atomen, wobei in diesen Resten auch eine oder mehrere CH₂-Gruppen durch -O-, -S-, 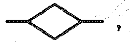 -C≡C-, -CF₂O-, -OCF₂-, -OC-O- oder -O-CO- so ersetzt sein können, dass O-Atome nicht direkt miteinander verknüpft sind,
L¹⁻⁴ jeweils unabhängig voneinander F, Cl, CF₃ oder CHF₂,
Z² und Z^{2'} jeweils unabhängig voneinander Einfachbindung, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
p 1 oder 2,
q 0 oder 1, und
v 1 bis 6
bedeuten.

4. FK-Medium nach einem oder mehreren der Ansprüche 1 bis 3 **dadurch gekennzeichnet, dass** für die Verbindung der Formel I
Z² eine Einfachbindung
bedeutet.

5. FK-Medium nach einem oder mehreren der Ansprüche 1 bis 4, **dadurch gekennzeichnet, dass** es die Verbindungen der Formel I in einer Konzentration von weniger als 10 Gew.-% enthält.

6. FK-Medium nach einem oder mehreren der Ansprüche 1 bis 5, **dadurch gekennzeichnet, dass** es eine oder mehrere polymerisierbare Verbindungen der Formel M oder eine polymerisierte Komponente, die eine oder mehrere Verbindungen der Formel M in polymerisierter Form enthält, umfasst:
P^{a}-(Sp^{a})ₛ₁-A²-(Z¹-A¹)ₙ-(Sp^{b})ₛ₂-P^{b} M
worin die einzelnen Reste folgende Bedeutung besitzen:
P^{a}, P^{b} jeweils unabhängig voneinander eine polymerisierbare Gruppe,
Sp^{a}, Sp^{b} bei jedem Auftreten gleich oder verschieden eine Abstandsgruppe,
s1, s2 jeweils unabhängig voneinander 0 oder 1,
A¹, A² jeweils unabhängig voneinander einen Rest ausgewählt aus folgenden Gruppen
a) der Gruppe bestehend aus trans-1,4-Cyclohexylen, 1,4-Cyclohexenylen und 4,4'-Bicyclohexylen, worin auch eine oder mehrere nicht benachbarte CH₂-Gruppen durch -O-und/oder -S- ersetzt sein können und worin auch ein oder mehrere H-Atome durch F ersetzt sein können,
b) der Gruppe bestehend aus 1,4-Phenylen und 1,3-Phenylen, worin auch eine oder zwei CH-Gruppen durch N ersetzt sein können und worin auch ein oder mehrere H-Atome durch L ersetzt sein können,
c) der Gruppe bestehend aus Tetrahydropyran-2,5-diyl, 1,3-Dioxan-2,5-diyl, Tetrahydrofuran-2,5-diyl, Cyclobut-1,3-diyl, Piperidin-1,4-diyl, Thiophen-2,5-diyl und Selenophen-2,5-diyl, welche auch ein oder mehrfach durch L substituiert sein können,
d) der Gruppe bestehend aus gesättigten, teilweise ungesättigten oder vollständig ungesättigten, und optional substituierten, polycyclischen Resten mit 5 bis 20 cyclischen C-Atomen, von denen auch eines oder mehrere durch Heteroatome ersetzt sein können, vorzugsweise ausgewählt aus der Gruppe bestehend aus Bicyclo[1.1.1]pentan-1,3-diyl, Bicyclo[2.2.2]octan-1,4-diyl, Spiro[3.3]heptan-2,6-diyl, 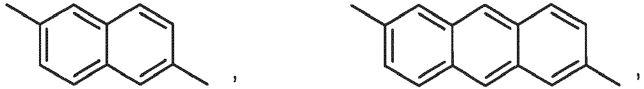 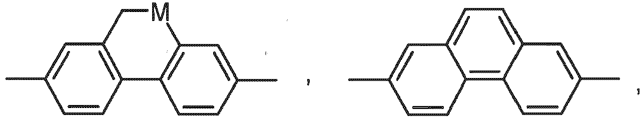 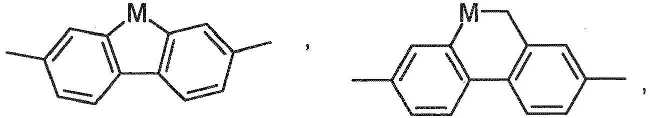 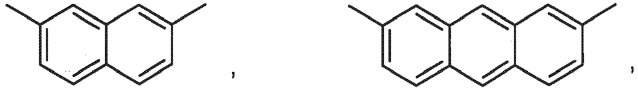 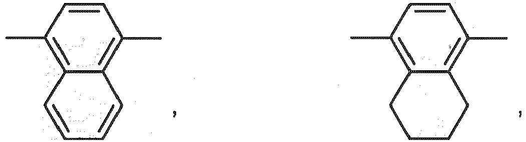 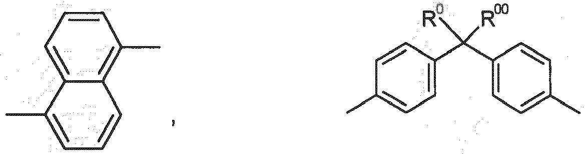 wobei in diesen Resten auch ein oder mehrere H- Atome durch L ersetzt sein können, und/oder eine oder mehrere Doppelbindungen durch Einfachbindungen ersetzt sein können, und/oder ein oder mehrere CH-Gruppen durch N ersetzt sein können,
n 0, 1, 2 oder 3,
Z¹ jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist, -O-, -CO-, -C(R^{c}R^{d})-, -CH₂CF₂-, -CF₂CF₂-, oder eine Einfachbindung,
R^{c}, R^{d} jeweils unabhängig voneinander H oder Alkyl mit 1 bis 6 C-Atomen,
L bei jedem Auftreten gleich oder verschieden F, Cl, CN, SCN, SF₅ oder geradkettiges oder verzwiegtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
R⁰, R⁰⁰ jeweils unabhängig voneinander H, F oder geradkettiges oder verzweigtes Alkyl mit 1 bis 12 C-Atomen, worin auch ein oder mehrere H-Atome durch F ersetzt sein können,
M -O-, -S-, -CH₂-, -CHY¹- oder -CY¹Y²-, und
Y¹ und Y² jeweils unabhängig voneinander eine der oben für R⁰ angegebenen Bedeutungen, Cl oder CN.

7. FK-Medium nach einem oder mehreren der Ansprüche 1 bis 6, **dadurch gekennzeichnet, dass** die Verbindung der Formel I eine oder mehrere polymerisierbare Gruppen aufweist.

8. FK-Anzeige enthaltend eine FK-Zelle mit zwei Substraten und mindestens zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums nach einem oder mehreren der Ansprüche 1 bis 7,
wobei die organische Verbindung oder die Verbindung der Formel I geeignet ist, eine homöotrope Ausrichtung des FK-Mediums gegenüber den Substratoberflächen herbeizuführen.

9. FK-Anzeige nach Anspruch 8, **dadurch gekennzeichnet, dass** die Substrate keine Orientierungsschichten zur homöotropen Ausrichtung aufweisen.

10. FK-Anzeige nach Anspruch 8, **dadurch gekennzeichnet, dass** die Substrate ein- oder beidseitig Orientierungsschichten aufweisen, die nicht gerieben sind.

11. FK-Anzeige nach einem oder mehreren der Ansprüche 8 bis 10, **dadurch gekennzeichnet, dass** es sich um eine VA-Anzeige mit einem FK-Medium mit negativer dielektrischer Anisotropie und auf gegenüberliegenden Substraten angeordneten Elektroden handelt.

12. Verfahren zu Herstellung eines FK-Mediums, **dadurch gekennzeichnet, dass** man eine oder mehrere organische Verbindungen mit mindestens einer polaren Ankergruppe und mindestens einer Ringgruppe nach Anspruch 1 mit einer niedermolekularen flüssigkristalline Komponente mischt und optional eine oder mehrere polymerisierbare Verbindungen und/oder Additive zugibt.

13. Verwendung von organischen Verbindungen mit mindestens einer polaren Ankergruppe und mindestens einer Ringgruppe nach einem oder mehreren der vorangehenden Ansprüche als Additiv für FK-Medien zur Herbeiführung einer homöotropen Orientierung gegenüber einer das FK-Medium begrenzenden Oberfläche.

14. Verfahren zu Herstellung einer FK-Anzeige enthaltend eine FK-Zelle mit zwei Substraten und mindestens zwei Elektroden, wobei mindestens ein Substrat lichtdurchlässig ist und mindestens ein Substrat eine oder zwei Elektroden aufweist, umfassend die Verfahrensschritte:
- Befüllen der Zelle mit einem FK-Medium enthaltend eine niedermolekulare flüssigkristalline Komponente, eine polymerisierbare Komponente und eine organische Verbindung mit mindestens einer polaren Ankergruppe nach einem oder mehreren der Ansprüche 1 bis 7, die geeignet ist, eine homöotrope (vertikale) Ausrichtung des FK-Mediums gegenüber den Substratoberflächen herbeizuführen, und optional
- Polymerisieren der polymerisierbaren Komponente, optional unter Anlegen einer Spannung an die Zelle oder unter der Wirkung eines elektrischen Feldes.

## Claims

1. LC medium having negative dielectric anisotropy, containing a low-molecular-weight liquid-crystalline component, additionally a polymerizable or a polymerized component,
wherein the polymerized component is obtainable by polymerization of a polymerizable component, and one or more organic compounds, wherein the organic compound is **characterized in that** it has at least one polar anchor group and at least one ring group,
wherein the polar anchor group comprises at least one OH structure or an N atom in a primary or secondary amine group,
and wherein the anchor group does not consist of an OH group which is shielded like the OH group in 2,6-di-tert-butylphenol,
and wherein the organic compound comprises a compound of formula I:
R¹-A¹-(Z²-A²)ₘ₁-R² (I)
wherein
A¹ and A² each independently of one another denote an aromatic, heteroaromatic, alicyclic, or heterocyclic group which can also contain fused rings and which may also be mono- or poly-substituted by a group L,
L each independently denotes -OH, -(CH₂)ₙ₁-OH, F, Cl, Br, I, -CN, -NO₂, -NCO, -NCS, -OCN, -SCN, -C(=O)N(RO)₂, -C(=O)RO, -N(RO)₂, -(CH₂)ₙ₁-N(RO)₂, optionally substituted silyl, optionally substituted aryl or cycloalkyl with 6 to 20 C atoms, or straight-chain or branched alkyl, alkoxy, alkylcarbonyl, alkoxycarbonyl, alkylcarbonyloxy, or alkoxycarbonyloxy with 1 to 25 C atoms, wherein one or more H atoms may also be replaced by F or Cl,
Z² each independently denotes -O-, -S-, -CO-, -CO-O-, -OCO-, -O-CO-O-, -OCH₂-, -CH₂O-, -SCH₂-, -CH₂S-, -CF₂O-, -OCF₂-, -CF₂S-, -SCF₂-. -(CH₂)ₙ₁-, -CF₂CH₂-, -CH₂CF₂-, -(CF₂)ₙ₁-, -CH=CH-, -CF=CF-, -C≡C-, -CH=CH-COO-, -OCO-CH=CH-, CR⁰R⁰⁰ or a single bond,
R⁰ and R⁰⁰ each independently denote H or alkyl with 1 to 12 C atoms,
R¹ denotes H, halogen, straight-chain, branched or cyclic alkyl with 1 to 25 C atoms, wherein one or more non-adjacent CH₂ groups may also be replaced by -NRO-, -O-, -S-, -CO-, -CO-O-, -O-CO-, -O-CO-O- in such a way that N-, O- and/or S atoms are not directly coupled to each other and wherein one or more tertiary carbon atoms (CH groups) may also be replaced by N and wherein one or more H atoms may also be replaced by F or Cl,
m1 denotes 0, 1, 2, 3, 4, or 5; and
n1 denotes 1, 2, 3, or 4;
and wherein the group R² comprises a group of sub-formula (A1)
-Sp-[X²-Z³-]ₖX¹ (A1)
wherein
Sp denotes a spacer group;
X¹ denotes a group -NH₂, -NHR¹¹-NR¹¹₂, -OH, -(CO)OH or a group of formulae 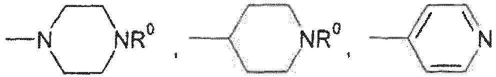 or 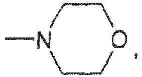
R⁰ denotes H or alkyl with 1 to 12 C atoms;
X² in each case independently denotes -NH-, -NR¹¹-, -O- or a single bond;
Z³ in each case independently denotes an alkylene group having 1-15 C atoms, carbocyclic rings having 5 or 6 C atoms, or combinations of one or more rings and alkylene groups, in each of which hydrogen may be replaced by -OH, -OR¹¹, -(CO)OH, -NH₂, -NHR¹¹, -NR¹¹₂, or halogen (preferably F, CI);
R¹¹ in each case independently denotes a halogenated or unsubstituted alkyl radical having 1 to 15 C atoms, wherein in said radical one or more CH₂ groups each independently can also be replaced by -C=C-, -CH=CH-, -(CO)O-, -O(CO)-, -(CO)-, or -O- in such a way that O atoms are not linked directly to each other, and wherein two radicals R ¹¹ may be linked to each other to form a ring; and
k denotes 0, 1, 2, or 3.

2. LC medium according to claim 1, **characterized in that** the organic compound has a relative molar mass of more than 100 g/mol.

3. LC medium according to claim 1 or 2, **characterized in that** it additionally contains one or more compounds selected from the group of compounds of formulae A, B, and C 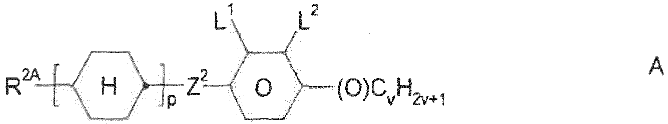 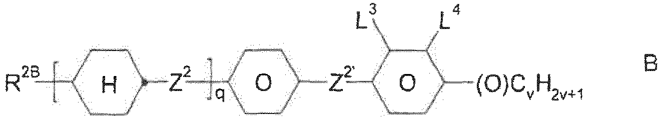 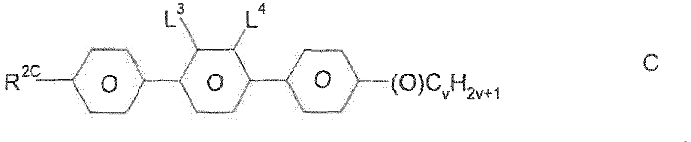 wherein
R^{2A}, R^{2B}, and R^{2C} each independently denote H, an alkyl radical having up to 15 C atoms which is unsubstituted or monosubstituted by CN or CF₃ or at least monosubstituted by halogen, wherein in these radicals one or more CH₂ groups may also be replaced by -O-, -S-, 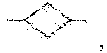 -C=C-, -CF₂O-, -OCF₂-, -OC-O-, or -O-CO- in such a way that O atoms are not linked directly to each other;
L¹⁻⁴ each independently denote F, Cl, CF₃, or CHF₂,
Z² and Z^{2'} each independently denote a single bond, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
p denotes 1 or 2;
q denotes 0 or 1; and
v denotes 1 to 6.

4. LC medium according to one or more of claims 1 to 3, **characterized in that** for the compound of formula I
Z² denotes a single bond.

5. LC medium according to one or more of claims 1 to 4, **characterized in that** it contains the compounds of formula I in a concentration of less than 10wt.%.

6. LC medium according to one or more of claims 1 to 5, **characterized in that** it comprises one or more polymerizable compounds of formula M or a polymerized component containing one or more compounds of formula M in a polymerized form:
P^{a}-(Sp^{a})ₛ₁-A²-(Z¹-A¹)ₙ-(Sp^{b})ₛ₂-P^{b} M
in which the individual radicals have the following meanings:
P^{a}, P^{b} each independently denote a polymerizable group;
Sp^{a}, Sp^{b} at each occurrence identical or differently denote a spacer group;
s¹, s² each independently denote 0 or 1;
A¹, A² each independently denote a radical selected from the following groups
a) the group consisting of trans-1,4-cyclohexylene, 1,4-cyclohexenylene, and 4,4'-bicyclohexylene, wherein one or more non-adjacent CH₂ groups may also be replaced by -O- and/or -S- and wherein one or more H atoms may also be replaced by F;
b) the group consisting of 1,4-phenylene and 1,3-phenylene, wherein one or two CH groups may also be replaced by N and wherein one or more H atoms may also be replaced by L,
c) the group consisting of tetrahydropyrane-2,5-diyl, 1,3-dioxane-2,5-diyl, tetrahydrofurane-2,5-diyl, cyclobut-1,3-diyl, piperidine-1,4-diyl, thiophene-2,5-diyl, and selenophene-2,5-diyl which may also be mono or poly-substituted by L;
d) the group consisting of saturated, partially unsaturated or completely unsaturated and optionally substituted, polycyclic radicals with 5 to 20 cyclic C atoms one or more of which may also be replaced by heteroatoms, preferably selected from the group consisting of bicyclo[1.1.1]pentane-1,3-diyl, bicyclo[2.2.2]octane-1,4-diyl, spiro[3.3]heptane-2,6-diyl, 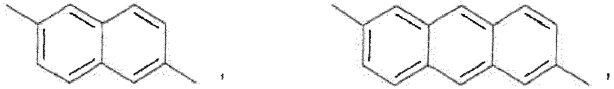 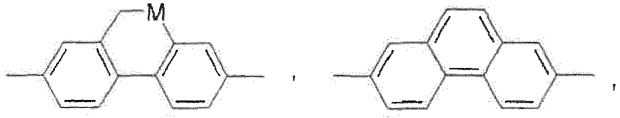 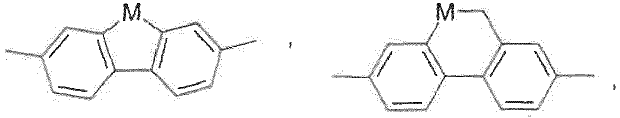 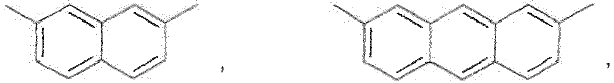 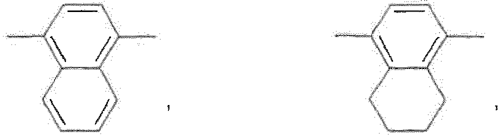 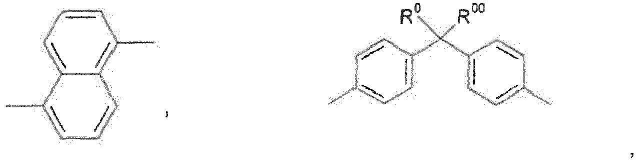
n wherein in said radicals one or more H atoms may also be replaced by L and/or one or more double bonds may be replaced by single bonds, and/or one or more CH groups may be replaced by N,
denotes 0, 1, 2, or 3,
Z¹ each independently denotes -CO-O-, -O-CO-, -CH₂O-, -OCH₂-, -CF₂O-, -OCF₂-, or -(CH₂)ₙ-, wherein n is 2, 3, or 4, -O-, -CO-, -C(R^{c}R^{d})-, -CH₂CF₂-, -CF₂CF₂-, or a single bond,
R^{c}, R^{d} each independently denote H or alkyl with 1 to 6 C atoms;
L at each occurrence identically or differently denotes F, Cl, CN, SCN, SF₅ or straight-chain or branched, in each case optionally fluorinated alkyl, alkoxy, alkyl carbonyl; alkoxy carbonyl, alkyl carbonyloxy, or alkoxy carbonyloxy with 1 to 12 C atoms;
R⁰, R⁰⁰ each independently denote H, F or straight-chain or branched alkyl with 1 to 12 C atoms, wherein one or more H atoms may also be replaced by F;
M -O-, -S-, -CH₂-, -CHY¹- or -CY¹Y²-; and
Y¹ and Y² each independently have the meanings given for R⁰ above, Cl or CN.

7. LC medium according to one or more of claims 1 to 6, **characterized in that** the compound of formula I has one or more polymerizable groups.

8. LC display containing an LC cell having two substrates and at least two electrodes, wherein at least one substrate is transparent to light and at least one substrate has one or two electrodes as well as a layer of an LC medium according to one or more of claims 1 to 7 located between the substrates, wherein the organic compound or the compound of formula I is suitable for effecting a homeotropic alignment of the LC medium with respect to the substrate surfaces.

9. LC display according to claim 8, **characterized in that** the substrates have no alignment layers for homeotropic alignment.

10. LC display according to claim 8, **characterized in that** the substrates on one or both sides have alignment layers which are not rubbed.

11. LC display according to one or more of claims 8 to 10, **characterized in that** it is a VA display with an LC medium having a negative dielectric anisotropy and electrodes arranged on opposite substrates.

12. A process for the production of an LC medium, **characterized in that** one or more organic compounds with at least one polar anchor group and at least one ring group according to claim 1 are mixed with a low-molecular-weight liquid-crystalline component and optionally one or more polymerizable compounds and/or additives are added.

13. Use of organic compounds with at least one polar anchor group and at least one ring group according to one or more of the preceding claims as an additive for LC media for effecting a homeotropic alignment with respect to a surface delimiting the LC medium.

14. A process for the production of an LC display containing an LC cell having two substrates and at least two electrodes, wherein at least one substrate is transparent to light and at least one substrate has one or two electrodes, comprising the process steps:
- filling the cell with an LC medium containing a low-molecular-weight liquid-crystalline component, a polymerizable component, and an organic compound having at least one polar anchor group according to one or more of claims 1 to 7 which is suitable for effecting a homeotropic (vertical) alignment of the LC medium with respect to the substrate surfaces; and optionally
- polymerizing the polymerizable component, optionally with application of a voltage to the cell or under the action of an electrical field.

## Revendications

1. °Milieu FK à anisotropie diélectrique négative contenant un composant de cristaux liquides à faible poids moléculaire, ainsi qu'un composant polymérisable ou un composant polymérisé, dans lequel le composant polymérisé peut être obtenu par polymérisation d'un composant polymérisable et d'un ou plusieurs composés organiques, dans lequel le composé organique est **caractérisé en ce que** qu'il présente au moins un groupe d'ancrage polaire et présente au moins un groupe cyclique, dans lequel le groupe d'ancrage polaire comprenant au moins une structure OH ou un atome de N dans un groupe amine primaire ou secondaire,
et dans lequel le groupe d'ancrage ne consiste pas en un groupe OH qui est protégé comme le groupe OH dans du 2,6-di-tert-butylphénol,
et dans lequel le composé organique comprend un composé de formule I :
R¹-A¹-(Z²-A²)ₘ₁-R² (I)
dans lequel
A¹ et A² signifient chacun indépendamment un groupe aromatique, hétéroaromatique, alicyclique ou hétérocyclique, qui peut également contenir des cycles condensés et qui peut également être mono- ou polysubstitué par un groupe L,
chaque L signifie indépendamment -OH, -(CH₂)ₙ₁-OH, F, Cl, Br, I, - CN, -NO₂, -NCO, -NCS, -OCN, -SCN, -C(=O)N(R⁰)₂, -C(=O)R⁰, -N(R⁰)₂, -(CH₂)ₙ₁-N(R⁰)₂, un silyle facultativement substitué, un aryle ou cycloalkyle facultativement substitué présentant de 6 à 20 atomes de C, ou un alkyle, alcoxy, alkylcarbonyle, alcoxycarbonyle, alkylcarbonyloxy ou alcoxycarbonyloxy linéaire ou ramifié présentant de 1 à 25 atomes de C, dans lequel un ou plusieurs atomes de H peuvent également être remplacés par du F ou Cl,
chaque Z² signifie indépendamment -O-, -S-, -CO-, -CO-O-, -OCO-, - O-CO-O-, -OCH₂-, -CH₂O-, -SCH₂-, -CH₂S-, -CF₂O-, -OCF₂-, -CF₂S-, -SCF₂-, - (CH₂)ₙ₁-, -CF₂CH₂-, -CH₂CF₂-, -(CF₂)ₙ₁-, -CH=CH-, -CF=CF-, -C≡C-, -CH=CH-COO-, -OCO-CH=CH-, CR⁰R⁰⁰ ou une liaison simple,
R⁰ et R⁰⁰ signifient chacun indépendamment H ou un alkyle présentant de 1 à 12 atomes de C,
R¹ signifie H, un halogène, alkyle linéaire, ramifié ou cyclique présentant de 1 à 25 atomes de C, dans lequel un ou plusieurs groupes CH₂ non adjacents peuvent également être remplacés par, -NR⁰-, -O-, -CO-, -CO -O-, -O-CO-, -O-CO-O-, de sorte que les atomes de N, O et/ou S ne soient pas liés directement les uns aux autres, et dans lequel également un ou plusieurs atomes de carbone tertiaires (groupes CH) peuvent être remplacés par du N, et dans lequel un ou plusieurs atomes de H peuvent également être remplacés par du F ou Cl,
m1 signifie 0,1, 2, 3, 4 ou 5, et
n1 signifie 1, 2, 3 ou 4,
et dans lequel le groupe R² comprend un groupe de la formule partielle (A1)
-Sp-[X²-Z³-]ₖX¹ (A1),
dans lequel
Sp signifie un groupe d'espacement,
X¹ signifie un groupe -NH₂, -NHR¹¹ -N¹¹₂, -OH, -(CO)OH ou un groupe des formules 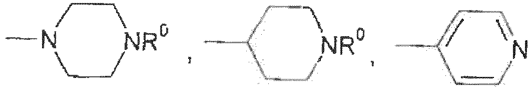 ou 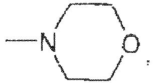
R⁰ signifie H ou un alkyle présentant de 1 à 12 atomes de C,
chaque X² signifie indépendamment -NH-, -NR¹¹-, -O- ou une liaison simple,
chaque Z³ signifie, indépendamment, un groupe alkylène présentant de 1 à 15 atomes de C, des cycles carbocycliques présentant 5 ou 6 atomes de C, ou des combinaisons d'un ou plusieurs cycles et groupes alkylène, dans lequel chaque hydrogène peut être remplacé par -OH, -OR¹¹, -(CO)OH, -NH₂, -NHR¹¹, - NR¹¹₂ ou un halogène (de préférence F, Cl),
chaque R¹¹ signifie indépendamment un radical alkyle halogéné ou non substitué présentant de 1 à 15 atomes de C, dans lequel dans ce radical, un ou plusieurs groupes CH₂ peuvent chacun indépendamment être remplacés par - C=C-, -CH=CH-, -(CO)O-, -O(CO)-, -(CO)- ou -O-, de sorte que les atomes de O ne sont pas liés directement entre eux, et dans lequel deux radicaux R¹¹ peuvent être liés pour former un cycle, et
k signifie 0, 1, 2 ou 3.

2. Milieu FK selon la revendication 1, **caractérisé en ce que** le composé organique présente une masse molaire relative supérieure à 100 g/mol.

3. Milieu FK selon la revendication 1 ou 2, **caractérisé en ce qu'**il contient en outre un ou plusieurs composés choisis dans le groupe des composés de formules A, B et C, 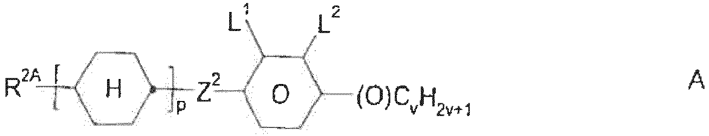 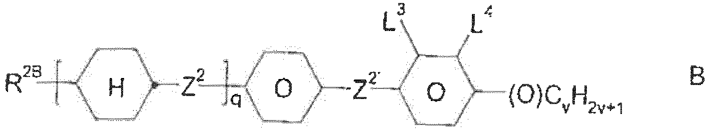 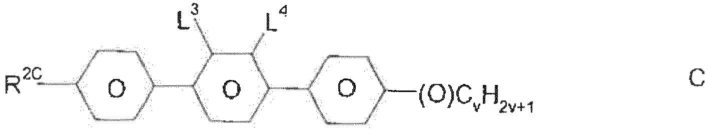 dans lequel
R^{2A}, R^{2B} et R^{2C} représentent chacun indépendamment H, un radical alkyle non substitué, monosubstitué simplement par CN ou CF₃, ou au moins monosubstitué simplement par un halogène, présentant jusqu'à 15 atomes de C, dans lequel dans ces radicaux, un ou plusieurs groupes CH₂- peuvent également être remplacés par -O-, -S-, 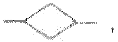 -C=C-, -CF₂O-, -OCF₂-, -OC-O- ou -O-CO-, de sorte que les atomes de O ne sont pas liés directement les uns aux autres,
chaque L¹⁻⁴ signifie indépendamment F, Cl, CF₃ ou CHF₂,
Z² et Z² signifient chacun indépendamment une liaison simple, - CH₂CH₂-, -CH=CH-, -CF₂O-, -OCF₂-, -CH₂O-, -OCH₂-, -COO-, -OCO-, -C₂F₄-, - CF=CF-, -CH=CHCH₂O-,
p signifie 1 ou 2,
q signifie 0 ou 1, et
v signifie 1 à 6.

4. Milieu FK selon une ou plusieurs des revendications 1 à 3, **caractérisé en ce que**
Z² signifie une liaison simple.

5. Milieu FK selon une ou plusieurs des revendications 1 à 4, **caractérisé en ce qu'**il contient les composés de formule I à une concentration inférieure à 10 % en poids.

6. Milieu FK selon une ou plusieurs des revendications 1 à 5, **caractérisé en ce qu'**il comprend un ou plusieurs composés polymérisables de formule M ou un composant polymérisé qui contient un ou plusieurs composés de formule M sous une forme polymérisée :
-°P^{a}-(Sp^{a})ₛ₁-A²-(Z¹-A¹)ₙ-(Sp^{b})ₛ₂-P^{b} M
dans lequel les radicaux individuels présentent la signification suivante :
- °P^{a}, P^{b} signifient chacun indépendamment un groupe polymérisable,
- °Sp^{a}, Sp^{b} signifient à chaque occurrence, de manière identique ou différente, un groupe d'espacement,
- °s¹, s² signifient chacun indépendamment 0 ou 1,
- °A¹, A² signifient chacun indépendamment un radical choisi parmi les groupes suivants
a) le groupe constitué de trans-1,4-cyclohexylène, de 1,4-cyclohexénylène et de 4,4'-bicyclohexylène, dans lequel un ou plusieurs groupes CH₂ non adjacents peuvent également être remplacés par -O- et/ou -S - et dans lequel un ou plusieurs atomes de H peuvent également être remplacés par du F,
b) le groupe constitué de 1,4-phénylène et 1,3-phénylène, dans lequel un ou deux groupes CH peuvent également être remplacés par du N et dans lequel un ou plusieurs atomes de H peuvent également être remplacés par du L,
c) le groupe constitué de tétrahydropyranne-2,5-diyle, 1,3-dioxane-2,5-diyle, tétrahydrofuranne-2,5-diyle, cyclobut-1,3-diyle, pipéridine-1,4-diyle, thiophène-2,5-diyle et sélénophène-2,5-diyle, qui peuvent également être substitués une ou plusieurs fois par du L,
d) le groupe constitué de radicaux polycycliques saturés, partiellement insaturés ou totalement insaturés, et facultativement substitués, présentant de de 5 à 20 atomes de C cycliques, dont un ou plusieurs peuvent également être remplacés par des hétéroatomes, de préférence choisis dans le groupe constitué de bicyclo[1.1.1]pentane-1,3-diyle, bicyclo[2.2.2]octane-1,4-diyle, spiro[3.3]heptane-2,6-diyle, 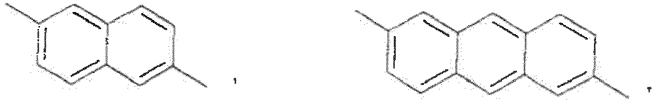 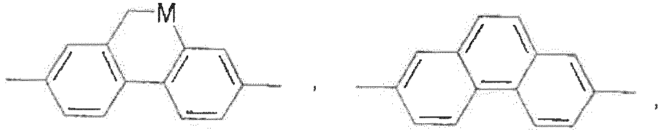 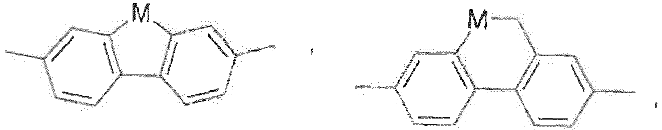 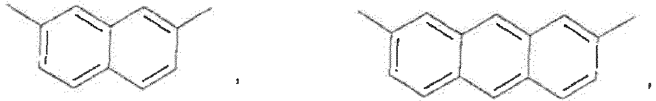 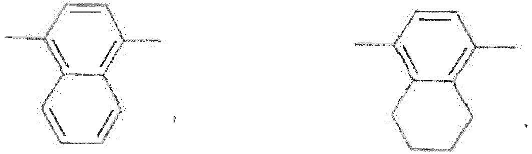 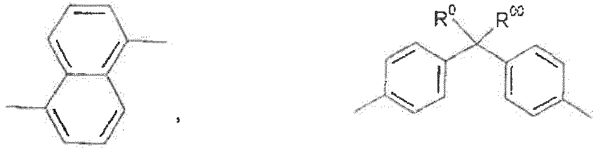
dans lequel dans ces radicaux, un ou plusieurs atomes de H peuvent également être remplacés par du L, et/ou une ou plusieurs liaisons doubles peuvent être remplacées par des liaisons simples, et/ou un ou plusieurs groupes CH peuvent être remplacés par du N,
n signifie 0, 1, 2 ou 3,
chaque Z¹ signifie indépendamment -CO-O-, -O-CO-, -CH₂O-, - OCH₂-, -CF₂O-, -OCF₂-, ou -(CH₂)ₙ-, dans lequel n signifie 2, 3 ou 4, -O-, -CO-, - C(R^{c}R^{d})-, -CH₂CF₂-, -CF₂CF₂-, ou une liaison simple,
Rc, Rd signifient chacun indépendamment H ou alkyle présentant de 1 à 6 atomes de C,
L à chaque occurrence, de manière identique ou différente, signifie F, Cl, CN, SCN, SF₅ ou un alkyle, alcoxy, alkylcarbonyle, alcoxycarbonyle, alkylcarbonyloxy ou alcoxycarbonyloxy linéaire ou ramifié, chacun facultativement fluoré, présentant de 1 à 12 atomes de C,
R⁰, R⁰⁰ signifient chacun indépendamment H, F ou un alkyle linéaire ou ramifié présentant de 1 à 12 atomes de C, dans lequel un ou plusieurs atomes de H peuvent également être remplacés par du F,
M -O-, -S-, -CH₂-, -CHY¹- ou -CY¹Y²-, et
Y¹ et Y² signifient chacun indépendamment l'une des significations données ci-dessus pour R⁰, Cl ou CN.

7. Milieu FK selon une ou plusieurs des revendications 1 à 6, **caractérisé en ce que** le composé de formule I présente un ou plusieurs groupes polymérisables.

8. Écran FK comprenant une cellule FK avec deux substrats et au moins deux électrodes, dans lequel au moins un substrat est translucide et au moins un substrat présente une ou deux électrodes, ainsi qu'une couche d'un milieu FK située entre les substrats selon une ou plusieurs des revendications 1 à 7,
dans lequel le composé organique ou le composé de formule I est apte à provoquer une orientation homéotrope du milieu FK par rapport aux surfaces du substrat.

9. Écran FK selon la revendication 8, **caractérisé en ce que** les substrats ne présentent pas de couches d'orientation pour une orientation homéotrope.

10. Écran FK selon la revendication 8, **caractérisé en ce que** les substrats présentent des couches d'orientation d'un ou des deux côtés, qui ne sont pas frottées.

11. Écran FK selon une ou plusieurs des revendications 8 à 10, **caractérisé en ce qu'**il s'agit d'un écran VA comprenant un milieu FK avec anisotropie diélectrique négative et des électrodes agencées sur des substrats mutuellement opposés.

12. Procédé de préparation d'un milieu FK, **caractérisé en ce qu'**un ou plusieurs composés organiques présentant au moins un groupe d'ancrage polaire et au moins un groupe cyclique selon la revendication 1 sont mélangés avec un composant de cristaux liquides à faible poids moléculaire et facultativement un ou plusieurs composés polymérisables et/ou additifs sont ajoutés.

13. Utilisation de composés organiques présentant au moins un groupe d'ancrage polaire et au moins un groupe cyclique selon une ou plusieurs des revendications précédentes comme additif pour des milieux FK pour obtenir une orientation homéotrope vers une surface délimitant le Milieu FK.

14. Procédé de production d'un écran FK contenant une cellule FK présentant deux substrats et au moins deux électrodes, dans lequel au moins un substrat est translucide et au moins un substrat présente une ou deux électrodes, comprenant les étapes de procédé suivantes consistant à :
- remplir la cellule avec un milieu FK contenant un composant de cristaux liquides à faible poids moléculaire, un composant polymérisable et un composé organique présentant au moins un groupe d'ancrage polaire selon une ou plusieurs des revendications 1 à 7, qui est apte à provoquer une orientation homéotrope (verticale) du milieu FK par rapport aux surfaces du substrat, et facultativement
- polymériser le composant polymérisable, facultativement par application d'une tension à la cellule ou sous l'action d'un champ électrique.
